# EUROPEAN PATENT SPECIFICATION

(11) **EP 1 206 937 B1**
(45) Date of publication and mention of the grant of the patent: **23.01.2008**
(21) Application number: 00946319.1
(22) Date of filing: 14.07.2000
(51) Int. Cl.: A61K 31/4164, A61P 17/00

(54) **NITROIMIDAZOLE EXTERNAL PREPARATIONS FOR THE TREATMENT OF ATOPIC DERMATITIS**
NITROIMIDAZOLPRÄPARATIONEN ZUR ÄUSSEREN ANWENDUNG ZUR BEHANDLUNG VON ATOPISCHER DERMATITIS
PREPARATIONS DE NITROIMIDAZOLE A USAGE EXTERNE POUR TRAITER LA DERMATITE ATOPIQUE

(30) Priority: 16.07.1999 JP 23449699; 21.07.1999 JP 20650899; 24.09.1999 JP 27107799; 28.09.1999 JP 31284099; 14.01.2000 JP 2000042012; 04.02.2000 JP 2000067746
(43) Date of publication of application: 22.05.2002
(73) Proprietor: SHOEI CO., LTD., Fukuoka-shi, Fukuoka 812-0011 (JP)
(72) Inventor: NISHIMUTA, Nishizumi, Fukuoka-shi Fukuoka 812-0011 (JP); NISHIMUTA, Kazuhiro, Fukuoka-shi Fukuoka 812-0011 (JP)
(74) Representative: Bawden, Peter Charles
(86) International application number: PCT/JP2000/004728
(87) International publication number: WO 2001/005400

(56) References cited:
- EP-A- 0 685 230
- WO-A-93/20817
- WO-A-96/01117
- WO-A-99/04829
- WO-A1-89/06537
- WO-A2-98/27960
- CA-A- 2 161 737
- FARAH C A ET AL: "TINIDAZOLE-TIOCONAZOLE COMBINATION IN THE TREATMENT OF VAGINAL INFECTION DUE TO TRICHOMONAS GARDNERELLA-VAGINALIS OR CANDIDA-ALBICANS" BIOLOGICAL ABSTRACTS. - MICROFILMS, BIOSIS. PHILADELPHIA, PA, US, vol. 84, 1987, page COMPLETE01, XP002091996 ISSN: 0192-6985
- DATABASE WPI Section Ch, Week 199806 Derwent Publications Ltd., London, GB; Class B05, AN 1998-052870 XP002305924 & CN 1 137 890 A (DONG M) 18 December 1996 (1996-12-18)
- DATABASE WPI Section Ch, Week 199347 Derwent Publications Ltd., London, GB; Class B05, AN 1993-369372 XP002305925 & CN 1 068 038 A (YANG C) 20 January 1993 (1993-01-20)
- DATABASE WPI Section Ch, Week 199807 Derwent Publications Ltd., London, GB; Class B05, AN 1998-075255 XP002305926 & RU 2 080 864 C1 (NAMOKONOV E V) 10 June 1997 (1997-06-10)
- NANDA VANDANA S: "Common dermatoses" AMERICAN JOURNAL OF OBSTETRICS AND GYNECOLOGY, vol. 173, no. 2, 1995, pages 488-495, XP002305923 ISSN: 0002-9378
- PATENT ABSTRACTS OF JAPAN vol. 1997, no. 10, 31 October 1997 (1997-10-31) & JP 9 157161 A (LION CORP), 17 June 1997 (1997-06-17)

## Description

### Field Of The Invention

The present invention relates to an external preparation which is used for a therapeutics, prophylactic treatment or amelioration of atopic dermatitis which comprises a nitroimidazole derivative as an active ingredient.

### Background Of The Invention

### (Atopic Dermatitis)

An atopic dermatitis is known to be a type I allergic reaction responsive to IgE. Various external preparations for therapeutic treatment of atopic skin disease have been developed so far, which comprises a compound with an activity for inhibiting PCA reactions which is responsive to IgE, as an active ingredient. However, in a practical application, there has not been known any that is adequately effective, while steroids such as adrenocortical hormones still continue to constitute the mainstream of external preparations for a therapeutic treatment of atopic skin diseases.

Although the steroids currently used for atopic dermatitis, which are applied for skin diseases and other skin diseases, have excellent therapeutic effects, when used over a long period of time, systemic side effects are produced including the functional suppression of hypothalamus, pituitary and adrenal cortex. In addition, despite being external preparations, they frequently exhibit local side effects in the form of skin symptoms such as exacerbation of skin infections and acne characteristic of adrenocortical hormones. Scars, liver spots and freckles in the course of administration as well as problems related to rebound following discontinuation of administration have also been pointed out.

Due to the above problems, immunosuppressants, antihistamines and antiallergics, etc. have been developed as therapeutic agents for atopic dermatitis. However, immunosuppressants involve problems such as exacerbation of bacterial skin infections, and antihistamines involve problems having adverse side effects such as drug rash.

With respect to atopic dermatitis whose cause has not yet be identified, patients having symptoms and their family members thereof suffer from itching and pain on a daily basis and also are perplexed by sleeplessness and various other symptoms. The Patients are not only relying on treatment at hospitals or clinics, but also on private treatment, and etc. Since definitive treatment methods have not yet be established at health care institutes, such as universities, hospitals and etc., there is an urgent need for the development of more effective external preparation for a therapeutic or prophylactic treatment of atopic dermatitis that is free of side effects to take the place of steroid-type anti-inflammatory external preparations.

### (Psoriasis)

Psoriasis is one of the most difficult skin diseases to cure and mechanism thereof is unknown. The symptoms recur repeatedly and no fundamental curative method has established so far.

Examples of a therapeutic treatment of psoriasis include application of ointments such as salicylic acid ointment, urea ointment, ointments used for the purpose of moisture retention and vitamin A ointment, heat treatment and soft X-ray, and ointments containing tranilast, cyclosporin or methotrexate, depending on locations and symptoms of the psoriasis. However, these treatments have hardly any therapeutic efficacy, and external steroid preparations are used mainly for treatment since they are relatively more effective. Today, although the therapeutic efficacy on psoriasis of external steroid preparations is not as great as that for other skin diseases, since there is no other therapeutic treatment available, treatment has been conducted through long-time use of external steroid preparations. However, as widely known, the adverse side effects associated with external steroid preparations are recognized as a problem.

In the treatment of psoriasis, there are problems related to the therapeutic efficacy and adverse side effects of external steroid preparations. Thus, rather than the external steroid preparations, attention has recently been focused on external vitamin D3 preparations. For example, an external active vitamin D3 preparation currently available on the market includes tacalcitol, and this preparation has no adverse side effects as with external steroid preparations, and its therapeutic effects are known to be relatively better than external steroid preparations.

However, even when treated with the above external steroid preparations or external vitamin D3 preparations, the treatment period is normally from several weeks to several months, and there are patients who undergo treatment for a long period of time extending several years to several tens of years. In addition, almost of the patients suffer from relapses which again leads to another period of prolonged treatment.

Thus, if an external preparation were available that could more effectively treat or prevent psoriasis, these types of problems would be able to be solved, therefore, such an external preparation has been desired.

### (Hircus, Body Odor and Osmidrosis)

Hircus is the same type of body odor as foot odor, and is thought to occur as a result of components of apocrine perspiration, which is secreted from apocrine glands located in the hair follicles of the skin, being degraded by various normal flora resulting in the production of a foul odor. As medical treatment of hircus, external preparations such as aluminum chloride solution or formalin alcohol solution are prescribed and used. The treatment is to inhibit odor to a certain degree through antiperspirant action, but they are unable to completely eliminate odor, recurring the odor when perspiration occurs again. In addition, these drugs are also known to cause a high incidence of side effects such as skin rash, itching and rubor following their use.

However, there are currently no medical pharmaceuticals available for the treatment of hircus, foot odor or other forms of body odor in the field of dermatology. Known antiperspirants such as the above aluminum chloride solution and formalin alcohol solution are unable to obtain satisfactory therapeutic results.

Consequently, a surgical operation is employed in which the skin of the armpits is resected to remove the apocrine glands, but this is an extremely bothersome procedure, results in a surgical scar over a wide area following surgery, results in atrophy of skin and muscle, and leaves the skin in a keloid state. Furthermore, there is a considerable economic burden, and relapse is relatively common. In addition, there is frequent occurrence of sequela resulting in neural impairment due to surgical error. The bother, surgical scars and adverse side effects of this surgical treatment along with the mental suffering caused by the sequela are immeasurable for the person undergoing the procedure.

Thus, there has been required for a therapeutic or prophylactic agent for external use for hircus, body odor and osmidrosis that is economical and does not suffer the patient.

### (Pigmentation, Blotches and Scars)

For scars due to the sequelae of drug rash, burns, herpes, keloids and smallpox, pigmentation and blotches etc. caused by ultraviolet ray irradiation or cosmetics, there still are no effective external medications despite their having a serious effect on daily life, thereby creating the need for the development of such a medication.

### (Contact Dermatitis, etc.)

There is also a need for an external preparation that is free from side effects and effectively used for a therapeutic or prophylactic treatment of contact dermatitis, plant dermatitis or insect bites, a therapeutic or prophylactic treatment of dermal pruritis or drug rash, a therapeutic or prophylactic treatment of chilblain, a therapeutic or prophylactic treatment of erythroderma, a therapeutic or prophylactic treatment of tinea, a therapeutic or prophylactic treatment of suppurative skin diseases, a therapeutic or prophylactic treatment of pressure sores, a therapeutic or prophylactic treatment of wounds, as well as a therapeutic or prophylactic treatment of palmoplantar pustulosis, lichen planus, lichen nitidus, pityriasis rubra pilaris, pityriasis rosea, erythema (including polymorphic exudative erythema, erythema nodosum and Darier's erythema annulare centrifugum), chronic discoid lupus erythematosus, drug rash and toxic rash, alopecia areata, burns (including scars and keloids), pemphigus, Duhring dermatitis herpetiformus (including pemphigoid), seborrheic dermatitis, dermal stomatitis, Candidiasis (including interdigital erosion, intertrigo, dermal Candidiasis, infantile parasitic erythema, perionychia and vaginal Candidiasis) and tinea versicolor.

It should be noted that the following are known with respect to metronidazole and tinidazole among the nitroimidazole derivatives of the present invention.

A compound metronidazole (2-(2-methyl-5-nitroimidazol-1-yl) ethanol) is a nitroimidazole derivative that was synthesized by Jacob of Rhone-Poulenc Rorer S.A. (France) in 1957. Metronidazole was found to have potent anti-Trichomonas activity by Cosar & Julou. Durel first reported in 1959 that Trichomonas protozoa disappeared following the use of this drug against human trichomoniasis. In addition, it is known that this drug has a strong antimicrobial activity against Entamoeba histolytica. Moreover, it has also been reported to have bactericidal activity against other anaerobes by both oral administration and topical administration. Its mechanism of action is thought to be the result of the nitro group of metronidazole being reduced by the microorganism, and this reduction in turn leads to a dysfunction such as cleavage in the double stranded DNA of the microorganism thereby inhibiting mitosis and proliferations.

Tinidazole was synthesized in 1966 by the Pfizer Inc. in the United States as a compound having even more potent effect than metronidazole which is an orally used chemotherapeutic agent, and having low toxicity. This compound primarily has anti-Trichomonas activity. Thus, not only does it have superior effects against infections caused by Trichomonas vaginalis as well as against Trichomonas vaginalis infecting the vulva, cervical tube, urinary tract and rectum, but also exhibits antimicrobial activity against anaerobic microorganisms as well and is used clinically for such purposes. Its mechanism of action is thought to involve reduction of the nitro group of tinidazole by the microorganism resulting in this reduction product causing functional impairment such as cleavage of double strand DNA, thereby inhibiting mitosis and proliferations of the microorganism.

In addition, with respect to metronidazole, the following information is known regarding the effect of its administration on immunity. Namely, it is clearly indicated in Int. Arch. Allergy Appl. Immun., 54, 422 (1977) that, in mice orally administered metronidazole, although the formation of granuloma by intravenous injection of the eggs of Schistosoma mansoni was inhibited, nonspecific granuloma formation was not inhibited. According to Int. J. Radiation Oncology Biol Phys., 9, 701 (1983), intraperitoneal administration of metronidazole is known to inhibit swelling of the ears induced by dinitrofluorobenzene in mice sensitized with dinitrofluorobenzene. In addition, according to the Indian J. Exp. Biol., 25, 177 (1987), it is clearly indicated that intraperitoneal administration of metronidazole significantly inhibits increases in anti-TBA antibody titer to TBA vaccine in rabbits, and according to Indian J. Exp. Biol., 29, 867 (1991), it is clearly indicated that intraperitoneal administration of metronidazole inhibits a delayed immune reaction to intravenous injection of ovine erythrocytes while also demonstrating inhibitory action on leukocyte migration. Moreover, known effects of metronidazole on inflammation include metronidazole external preparations being effective against inflammatory skin diseases such as rosacea (International Unexamined Patent Publication No. WO88/06888, International Unexamined Patent Publication No. WO89/06537, International Unexamined Patent Publication No. WO94/08350, International Unexamined Patent Publication No. W096101117 and International Unexamined Patent Publication No. WO98/27960). In addition, according to Mykosen, 27, 475 (1984), the reason why metronidazole exhibits therapeutic effects at a concentration at which it does not exhibit antimicrobial activity against P. ovale and so forth is because of its anti-inflammatory activity, and according to Br. J. Dermatol., 114, 231 (1986), metronidazole has inhibitory activity on the production of active oxygen species, and the reason why metronidazole is effective against rosacea is partially due to its anti-inflammatory activity. International Surgery, 60, 75 (1975) indicates that oral administration of metronidazole is effective against pododermal ulcers.

On the other hand, with respect to tinidazole, it is described in Indian J. Exp. Biol., 29, 867 (1991) with respect to immunity that intraperitoneal administration of tinidazole tends to inhibit delayed immune reaction to intravenous injection of ovine erythrocytes, and that it clearly exhibits inhibitory action of leukocyte migration. Moreover, with respect to inflammation, tinidazole external preparations are known to be used for the therapeutic treatment of skin inflammation (International Unexamined Patent Publication No. WO93/20817, International Unexamined Patent Publication No. WO98/27960).

With respect to the use of metronidazole for the therapeutic treatment of psoriasis, it is disclosed in US Patent Publication No. US 4,491,588 that oral preparations of metronidazole are effective for the treatment of psoriasis, and in International Unexamined Patent Publication No. WO96/01117, psoriasis is indicated as being one of the inflammatory diseases that can be cured with external preparations of metronidazole.

However, in these references mentioned above pertaining to immunity, with the exception of the Int. J. Radiation Oncology Biol. Phys., 9, 701 (1983), all of the immune reactions observed are immune reactions other than on the skin surface. In addition, the observed immunosuppressive effects are remarkably lower in comparison with those of immunosuppressants used clinically, and it is therefore considered that external preparations of metronidazole or tinidazole cannot be expected to be effective as therapeutic agents for atopic dermatitis. Further, there is no correlation between the effectiveness in treatment of atopic dermatitis and the effectiveness in the model of contact dermatitis used in the Int. J. Radiation Oncology Biol. Phys., 9, 701 (1983) in which the only immune reaction on the skin surface is observed. Moreover, it has not been known that typical therapeutic agents for inflammatory disease are used for therapeutic treatment of atopic dermatitis. In addition, the use of a nitroimidazole derivative for treatment of atopic dermatitis is also previously unknown.

Further, US Patent No. 4,491,588 discloses the treatment of psoriasis by oral administration of metronidazole, and although ketoconazole, which is similarly disclosed as being effective in the treatment of psoriasis, has been granted a right as an oral preparation (US Patent No. 4,491,588) and as an external preparation (US Patent No. 4,569,935), only an oral preparation has been granted a right with respect to metronidazole. The present invention is directed to findings that an external preparation of metronidazole is superior to the oral preparation in terms of effect and toxicity. Moreover, since the therapeutic use for psoriasis indicated in International Unexamined Patent Publication No. WO96/01117 is one example of a typical inflammatory disease, and the disclosed contents merely indicate that an external preparation of metronidazole is able to inhibit the formation of edema caused by local stimulation by arachidonic acid, as was described by the applicant himself to the effect that, "conventional non-steroid anti-inflammatory drugs, such as cyclooxygenase or lipoxygenase reaction inhibitors (including indometacin, naproxen and phenylbutazone) and preparations able to inhibit conduit plasma backflow (such as vasoconstrictors) are excellent reaction inhibitors in this model", this is an experimental system in which conventional non-steroid anti-inflammatory drugs (NSAIDs) also exhibit excellent effect. In this publication, it is deduced that metronidazole can be used for the treatment of "eczema, psoriasis, rosacea, lupus vulgaris, ulcers and seborrheic dermatitis", etc. only by virtue of confirming its action. However, this patent application cannot be included in a prior art reference of the present application since the etiology of psoriasis is unknown, nearly all NSAIDs do not exhibit therapeutic effects against psoriasis and the therapeutic effect against psoriasis has actually not been confirmed.

### Disclosure of the Invention

The present inventors have made extensive and intensive studies on a therapeutic or prophylactic agent for atopic dermatitis, and have found that an external preparation containing a nitroimidazole derivative as an active ingredient is extremely effective as a therapeutic or prophylactic agent for atopic dermatitis, and have found that the invention is highly safe and is free from adverse side effects, and thus, the present invention has been completed. In addition, the present inventors also have found that it is also particularly effective for a therapeutic and prophylactic treatment of facial atopy and pediatric atopy, for which the treatment has been difficult.

The present inventors have found that antiamyctic effects appear rapidly when crotamiton is contained in an external preparation containing a nitroimidazole derivative.

Other features of the present invention include a use of the nitroimidazole derivative for producing of an external preparation for skin disease for a prophylactic or therapeutic treatment of atopic dermatitis,

In addition, the present inventors also have found that an external preparation which comprises at least one compound of the nitroimidazole derivatives and at least one agent selected from the group consisting of an antimycotic agent, antibacterial agent, sulfa, immunosuppressant, anti-inflammatory agent, antibiotic, antiviral agent, metabolic antagonist, antihistamine, tissue repair promoter, vitamin, antiallergic, local anesthetic, hair agent and steroid being administered simultaneously or separately with an interval allows a concentration of these drugs other than nitroimidazole to be reduced, thereby eliminating adverse side effects while also being fast-acting. Moreover, it was also found that similar effects are demonstrated even at concentrations at which these agents other than nitroimidazole do not exhibit pharmacological effects.

The external preparation for a therapeutic or prophylactic treatment or amelioration of atopic dermatitis according to the present invention comprises a nitroimidazole derivative represented by the following formula (I), a pharmaceutically acceptable salt thereof, an ester thereof or other derivatives thereof as an active ingredient: 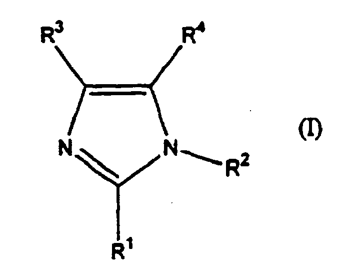 wherein R¹, R³ and R⁴ may be the same or different from each other and each independently represent a hydrogen atom, a nitro group, a lower alkyl group, a lower alkyl group substituted by 1 or more substituents which may be the same or different selected from a Substituent group α and Substituent group β, a lower alkenyl group or a lower alkenyl group substituted by 1 or more substituents which may be the same or different selected from the Substituent group α and the Substituent group β; and R² represents a hydrogen atom, a lower alkyl group, a lower alkyl group substituted by 1 or more substituents which may be the same or different selected from the Substituent group α and the Substituent group β, a lower alkenyl group or a lower alkenyl group substituted by 1 or more substituents which may be the same or different selected from the Substituent group α and the Substituent group β, provided that any one of R¹, R³ and R⁴ is a nitro group,
Substituent group α
comprises a lower alkyloxy group, a lower alkyloxy group substituted by 1 or more substituents which may be the same or different selected from the Substituent group β, a lower alkylcarbonyloxy group, a lower alkylcarbonyloxy group substituted by 1 or 2 or more substituents which may be the same or different selected from the Substituent group β, a lower alkylsulfonyl group, a lower alkylsulfonyl group substituted by 1 or more substituents which may be the same or different selected from the Substituent group β, a cycloalkyl group, a cycloalkyl group substituted by 1 or more substituents which may be the same or different selected from the Substituent group β, a heteroaryl group, a heteroaryl group substituted by 1 or more substituents which may be the same or different selected from the Substituent group β, an aryl group and an aryl group substituted by 1 or 2 or more substituents which may be the same or different selected from the Substituent group β.
Substituent group β
comprises a hydroxy group, a mercapto group, a halogen atom, an amino group, a lower alkylamino group, a lower alkyloxy group, a lower alkenyl group, a cyano group, a carboxy group, a carbamoyloxy group, a carboxyamide group, a thiocarboxyamide group a morpholino group and etc.

Of the above external preparations, preferred are
(1) an external preparation wherein R⁴ is a nitro group,
(2) an external preparation of (1) above wherein R¹ and R² are the same or different and represent a lower alkyl group, a lower alkyl group substituted by 1 or 2 or more substituents selected from the Substituent group α and the Substituent group β, a lower alkenyl group, or a lower alkenyl group substituted by 1 or 2 or more substituents which may be the same or different selected from the Substituent group α and the Substituent group β, and R³ is a hydrogen atom,
(3) an external preparation of (2) wherein <Substituent group α> is a lower alkyloxy group and the Substituent group β is a hydroxy group, an amino group, a halogen atom, a cycloalkyl group, a heteroaryl group or an aryl group,
(4) an external preparation of (3) wherein the Substituent group β is a hydroxy group, an amino group, a halogen atom or a heteroaryl group,
(5) an external preparation of (3) wherein R¹ is a lower alkyl group,
(6) an external preparation of (3) wherein R² is a lower alkyl group substituted by a hydroxy group,
(7) an external preparation of (2) wherein the Substituent group α is a lower alkylsulfonyl group or a lower alkylsulfonyl group substituted by substituents which may be the same or different selected from the Substituent group β and the Substituent group β is a hydroxy group, a halogen atom, an amino group, a lower alkylamino group, a lower alkyloxy group, a lower alkenyl group, a cyano group, a carboxy group, a cycloalkyl group or an aryl group and etc,
(8) an external preparation of (7) wherein R¹ is a lower alkyl group or a lower alkyl group substituted by substituent which may be the same or different selected from Substituent group β, and
(9) an external preparation of (7) wherein R² is a lower alkylsulfonyl group or a lower alkyl group substituted by the lower alkylsulfonyl group substituted by substituent which may be the same or different selected from the Substituent group β.

The above (1) and (2), (3) to (5) or (7) and (8) represent more preferable compound as the number becomes larger. In the general formula (I), external preparations obtained by optionally selecting R¹ to R⁴ from (1) to (9) and optionally combining those are also preferable and the more preferable external preparations are (5)-(6) and (8)-(9). Still further preferable external preparations are selected from the following groups:
Compound group
2-(2-methyl-5-nitroimidazol-1-yl)ethanol (general name: metronidazole) and, 1-(2-ethylsulfonylethyl)-2-methyl-5-nitroimidazole (general name: tinidazole).

In the above, examples of the "lower alkyl group" of R¹ to R⁴ and the "lower alkyl group" of the "lower alkyl group substituted by 1 or more substituents which may be the same or different selected from the Substituent group α and the Substituent group β" may include a straight or branched alkyl group having 1 to 6 carbon atoms such as methyl, ethyl, n-propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl and 2-ethylbutyl, preferably the straight or branched alkyl group having 1 to 3 carbon atoms, more preferably the methyl group in R¹ and the ethyl group in R².

In the above formula, examples of the "lower alkenyl group" in R¹ to R⁴ and Substituent group α and Substituent group β and the "lower alkenyl group" of the "lower alkenyl group substituted by 1 or 2 or more substituents which may be the same or different selected from the Substituent group α and the Substituent group β" may include a straight or branched alkenyl group having 2 to 6 carbon atoms such as ethenyl, 1-propenyl, 2-propenyl, 1-methyl-2-propenyl, 1-methyl-1-propenyl, 2-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl 5-hexenyl and etc., preferably the straight or branched alkenyl group having 3 to 5 carbon atoms.

In the above formula (I), examples of the "halogen atom" in Substituent group β may include a fluorine atom, a chlorine atom, a bromine atom and an iodine atom, preferably a fluorine atom and a chlorine atom.

In the above formula (I), the "lower alkyloxy group" in the Substituent group α and the Substituent group β and the "lower alkyloxy group" of the "lower alkyloxy group substituted by 1 or more substituents which may be the same or different selected from the Substituent group β" represent a group in which the above-mentioned "lower alkyl group" is bonded to an oxygen atom and examples of such group may include a straight or branched alkyloxy group having 1 to 6 carbon atoms such as methoxy, ethoxy, n-propoxy, isopropoxy, n-butoxy, isobutoxy, s-butoxy, tert-butoxy, n-pentoxy, isopentoxy, 2-methylbutoxy, neopentoxy, n-hexyloxy, 4-methylpentoxy, 3-methylpentoxy, 2-methylpentoxy, 3,3-dimethylbutoxy, 2,2-dimethylbutoxy, 1,1-dimethylbutoxy, 1,2-dimethylbutoxy, 1,3-dimethylbutoxy, 2,3-dimethylbutoxy and etc., preferably a straight or branched alkyloxy group having 1 to 3 carbon atoms, more preferably a methoxy group.

In the above formula (I), the "lower alkylcarbonyloxy group" in Substituent group α and the "lower alkylcarbonyloxy group" of the "lower alkylcarbonyloxy group substituted by 1 or more substituents which may be the same or different selected from the Substituent group β" represent a group in which the above "lower alkyl group" is bonded to a carbonyloxy group and examples of such group may include a straight or branched alkylcarbonyloxy group having 2 to 7 carbon atoms such as acetyloxy, propionyloxy, butyryloxy, isobutyryloxy, pentanoyloxy, pivaloyloxy, valeryloxy, isovaleryloxy and hexanoyloxy, preferably a straight or branched alkylcarbonyloxy group having 2 to 4 carbon atoms, more preferably a formyloxy group or an acetyloxy group.

In the above formula (I), the "lower alkylsulfonyl group" in Substituent group α and the "lower alkylsulfonyl group" of the "lower alkylsulfonyl group substituted by 1 or 2 or more substituents which may be the same or different selected from <Substituent group β>" represent a group in which the above "lower alkyl group" is bonded to a sulfonyl group and examples of such group may include a straight or branched alkylsulfonyl group having 1 to 6 carbon atoms such as methanesulfonyl, ethanesulfonyl, n-propanesulfonyl, isopropanesulfonyl, n-butanesulfonyl, isobutanesulfonyl, s-butanesulfonyl, tert-butanesulfonyl, n-pentanesulfonyl, isopentanesulfonyl, 2-methylbutanesulfonyl, neopentanesulfonyl, n-hexanesulfonyl, 4-methylpentanesulfonyl, 3-methylpentanesulfonyl, 2-methylpentanesulfonyl, 3,3-dimethylbutanesulfonyl, 2,2-dimethylbutansulfonyl, 1,1-dimethylbutanesulfonyl, 1,2-dimethylbutanesulfonyl, 1,3-dimethylbutanesulfonyl, 2,3-dimethylbutanesulfonyl and etc., preferably a straight or branched alkylsulfonyl group having 1 to 3 carbon atoms, more preferably an ethanesulfonyl group.

In the above formula (I), the "lower alkylamino group" in Substituent group β represents a group in which the above "lower alkyl group" is substituted by an amino group and examples of such group may include a straight or branched alkylamino group having 1 to 6 carbon atoms such as methylamino, ethylamino, n-propylamino, isopropylamino, n-butylamino, isobutylamino, s-butylamino, tert-butylamino, n-pentylamino, isopentylamino, 2-methylbutylamino, neopentylamino, 1-ethylpropylamino, n-hexylamino, isohexylamino, 4-methylpentylamino, 3-methylpentylamino, 2-methylpentylamino, 1-methylpentylamino, 3,3-dimethylbutylamino, 2,2-dimethylbutylamino, 1,1-dimethylbutylamino, 1,2-dimethylbutylamino, 1,3-dimethylbutylamino, 2,3-dimethylbutylamino, 2-ethylbutylamino and etc., preferably a straight or branched alkylamino group having 1 to 6 carbon atoms, more preferably a methylamino group or an ethylamino group.

In the above formula (I), the "cycloalkyl group" in Substituent group α and the "cycloalkyl group" of the "cycloalkyl group substituted by 1 or more substituents which may be the same or different selected from the Substituent group β" may include a 3 to 10-membered saturated cyclic hydrocarbon group which may be condensed such as cyclopropyl, cyclobutyl, cyclopentyl, cyclohexyl, cycloheptyl, norbornyl and adamantyl, preferably a 5 to 7-membered saturated cyclic hydrocarbon group.

In the above formula (I), examples of the "heteroaryl group" in Substituent group α and the "heteroaryl group" of the "heteroaryl group substituted by 1 or more substituents which may be the same or different selected from the Substituent group β" may include a 5 to 7-membered aromatic heterocyclic group such as furyl, thienyl, pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyranyl, pyridyl, pyridazinyl, pyrimidinyl, pyrazinyl and etc., preferably a pyridyl group.

In the above formula (I), examples of the "aryl group" in Substituent group α and the "aryl group" of the "aryl group substituted by 1 or more substituents which may be the same or different selected from the Substituent group β" may include an aromatic hydrocarbon group having 5 to 14 carbon atoms such as phenyl, indenyl, naphthyl, phenanethrenyl, anthrathenyl and etc., preferably an aromatic hydrocarbon group having 6 to 10 carbon atoms, more preferably a phenyl group.

Since the compound (I) of the present invention can be converted to a salt, the "pharmacologically acceptable salt thereof" represents such salt and may preferably include metal salts such as alkali metal salts, e.g., a sodium salt, a potassium salt and a lithium salt and etc., metal salt such as alkaline earth metal salts, e.g., a calcium salt and a magnesium salt, an aluminum salt, an iron salt, a zinc salt, a copper salt, a nickel salt and a cobalt salt; amine salts such as inorganic salts, e.g., an ammonium salt and organic salts, e.g., a t-octylamine salt, a dibenzylamine salt, a morpholine salt, a glucosamine salt, a phenylglycinealkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocain salt, a procain salt, a diethanolamine salt, an N-benzyl-phenethylamine salt, a piperazine salt, a tetramethylammonium salt, a tris(hydroxymethyl)aminomethane salt and etc.; inorganic acid salts such as halogenated hydroacid salts, e.g., a hydrofluoric acid salt, a hydrochloric acid salt, a hydrobromic acid salt and a hydroiodic acid salt, a nitric acid salt, a perchloric acid salt, a sulfric acid salt, a phosphoric acid salt and etc.; organic acid salts such as lower alkanesulfonic acid salt, e.g., a methanesulfonic acid salt, a trifluoromethanesulfonic acid salt and an ethanesulfonic acid salt, arylsulfonic acid salts, e.g., a benzenesulfonic acid salt and a p-toluenesulfonic acid salt, an acetic acid salt, a malic acid salt, a fumaric acid salt, a succinic acid salt, a citric acid salt, a tartaric acid salt, an oxalic acid salt and maleic acid salt; and amino acid salts such as a glycine salt, a lysine salt, an arginine salt, an ornithine salt, a glutamic acid salt and an aspartic acid salt. In the case where the salt becomes the metal salts or the amine salts, it is limited to the case where the compound (I) has an acidic group.

Moreover, in case the compound (I) of the present invention absorbs moisture content by allowing it to stand in the atmosphere so that adsorbed water is deposited thereon to form a hydrate, such salt is also included in the present invention.

Further, in case the compound (I) of the present invention absorbs a certain kind of solvent to form a solvate, such salt is also included in the present invention.

Since the compound (I) of the present invention can be converted to an ester, the "ester thereof" means such ester and represents "ester of a hydroxy group" and "ester of a carboxy group", wherein the respective ester residues are "a general protective group" or "a protective group cleavable by a biological method such as hydrolysis in a living body".

The "general protective group" represents a protective group cleavable by a chemical method such as hydrogenation decomposition, hydrolysis, electrolysis and optical decomposition and the "general protective group" relating to the "ester of the hydroxy group" may include "an aliphatic acyl group" such as an alkylcarbonyl group, e.g., formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl heneicosanoyl and etc., a carboxylated alkylcarbonyl group, e.g., succinoyl, glutanoyl and adipoyl, a halogeno- lower alkylcarbonyl group, e.g., chloroacetyl, dichloroacetyl, trichloroacetyl and trifluoroacetyl, a saturated cyclic hydrocarbon-carbonyl group, e.g., cyclopropylcarbonyl, cyclobutylcarbonyl, cyclopentylcarbonyl, cyclohexylcarbonyl, cycloheptylcarbonyl and cyclooctylcarbonyl, a lower alkoxy lower alkylcarbonyl group, e.g., methoxyacetyl, and an unsaturated alkylcarbonyl group, e.g., (E)-2-methyl-2-butenoyl;
"an aromatic acyl group" such as an arylcarbonyl group, e.g., benzoyl, α-naphthoyl, β-naphthoyl, pyridoyl, thienoyl and furoyl, a halogeno arylcarbonyl group, e.g., 2-bromobenzoyl and 4-chlorobenzoyl, a lower alkylated arylcarbonyl group, e.g., 2,4,6-trimethylbenzoyl and 4-toluoyl, a lower alkoxylated arylcarbonyl group, e.g., 4-anisoyl, a carboxylated arylcarbonyl group, e.g., 2-carboxybenzoyl, 3-carboxybenzoyl and 4-carboxybenzoyl, a nitrated arylcarbonyl group, e.g., 4-nitrobenzoyl and 2-nitrobenzoyl, a lower alkoxy carbonylated arylcarbonyl group, e.g., 2-(methoxycarbonyl)benzoyl an arylated arylcarbonyl group and etc, e.g., 4-phenylbenzoyl; "an aralkylcarbonyl group" such as a lower alkylcarbonyl group substituted by 1 to 3 aryl groups, e.g., phenylacetyl, α-naphthylpropionyl, β-napthytbutyryl, diphenylisobutyryl, triphenylacetyl, α-napthyldiphenylisobutyryl and 9-anthrylpentanoyl and a lower alkylcarbonyl group substituted by 1 to 3 aryl groups of which aryl ring is substituted by a lower alkyl group, a lower alkoxy group, a nitro group, a halogen atom ,a cyano group and etc., e.g., 4-methylphenylacetyl, 2,4,6-trimethylphenylformyl, 3,4,5-trimethylphenylbutyryl, 4-methoxyphenylisobutyryl, 4-methoxyphenyldiphenylpivaloyl, 2-nitrophenylacetyl, 4-nitrophenylpropionyl, 4-chlorophenylbutyryl, 4-bromophenylacetyl and 4-cyanophenylpentanoyl; "a tetrahydropyranyl group or a tetrahydrothiopyranyl group" such as tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl; "a tetrahydrofuranyl group or a tetrahydrothiofuranyl group" such as tetrahydrofuran-2-yl and tetrahydrothiofuran-2-yl; "a silyl group" such as a tri-lower alkylsilyl group, e.g., trimethylsilyl, triethylsilyl, isopropyldimethylsilyl, t-butyldimethylsilyl, methyldiisopropylsilyl, methyldi-t-butylsilyl and triisopropylsilyl and a tri-lower alkylsilyl group substituted by 1 or 2 aryl groups, e.g., diphenyl-methylsilyl, diphenylbutylsilyl, diphenylisopropylsilyl and phenyldiisopropylsilyl; "an alkoxymethyl group" such as a lower alkoxymethyl group, e.g., methoxymethyl, 1,1-dimethyl-1-methoxymethyl, ethoxymethyl, propoxymethyl, isopropoxymethyl, butoxymethyl and tert-butoxymethyl, a lower alkoxylated lower alkoxymethyl group, e.g., 2-methoxyethoxymethyl and a halogeno-lower alkoxymethyl group, e.g., 2,2,2-trichloroethoxymethyl and bis(2-ch loroethoxy)methyl;
"a substituted ethyl group" such as a lower alkoxylated ethyl group, e.g., 1-ethoxyethyl and 1-(isopropoxy)ethyl and a halogenated ethyl group, e.g., 2,2,2-trichloroethyl; "an aralkyl group" such as a lower alkyl group substituted by 1 to 3 aryl groups, e.g., benzyl, α-naphthylmethyl, β-naphthylmethyl, diphenylmethyl, triphenylmethyl, α-naphthyldiphenylmethyl and 9-anthrylmethyl and a lower alkyl group substituted by 1 to 3 aryl groups of which aryl ring is substituted by a lower alkyl group, a lower alkoxy group, a nitro group, a halogen atom or a cyano group, e.g., 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl and 4-cyanobenzyl; "an alkoxycarbonyl group" such as a lower alkoxycarbonyl group, e.g., methoxycarbonyl, ethoxycarbonyl, tert-butoxycarbonyl and isobutoxycarbonyl and a lower alkoxycarbonyl group substituted by a halogen atom or a tri-lower alkylsilyl group, e.g., 2,2,2-trichloroethoxycarbonyl, 2-trimethylsilylethoxycarbonyl and etc.; "an alkenyloxycarbonyl group" such as vinyloxycarbonyl and allyloxycarbonyl; and "an aralkyloxycarbonyl group" of which aryl ring may be substituted by 1 or 2 lower alkoxy or nitro groups such as benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl and etc.

Whereas, the "general protective group" relating to the "ester of the carboxy group" may preferably include "a lower alkyl group" such as methyl, ethyl, n-propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl and etc.; "an alkenyl group" such as ethenyl, 1-propenyl, 2-propenyl, 1-methyl-2-propenyl, 1-methyl-1-propenyl, 2-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl, 5-hexenyl and etc.; "an alkynyl group" such as ethynyl, 2-propynyl, 1-methyl-2-propynyl, 2-methyl-2-propynyl, 2-ethyl-2-propynyl, 2-butynyl, 1-methyl-2-butynyl, 2-methyl-2-butynyl, 1-ethyl-2-butynyl, 3-butynyl, 1-methyl-3-butynyl, 2-methyl-3-butynyl, 1-ethyl-3-butynyl, 2-pentynyl, 1-methyl-2-pentynyl, 2-methyl-2-pentynyl, 3-pentynyl, 1-methyl-3-pentynyl, 2-methyl-3-pentynyl, 4-pentynyl, 1-methyl-4-pentynyl, 2-methyl-4-pentynyl, 2-hexynyl, 3-hexynyl, 4-hexynyl, 5-hexynyl and etc.; "a halogeno- lower alkyl group" such as trifluoromethyl, trichloromethyl, difluoromethyl, dichloromethyl, dibromomethyl, fluoromethyl, 2,2,2-trifluoroethyl, 2,2,2-trichloroethyl, 2-bromoethyl, 2-chloroethyl, 2-fluoroethyl, 2-iodoethyl, 3-chloropropyl, 4-fluorobutyl, 6-iodohexyl and 2,2-dibromoethyl; "a hydroxy lower alkyl group" such as 2-hydroxyethyl, 2,3-dihydroxypropyl, 3-hydroxypropyl, 3,4-dihydroxybutyl 4-hydroxybutyl and etc.; "an aliphatic acyl"-"lower alkyl group" such as acetylmethyl; "an aralkyl group" such as "a lower alkyl group" substituted by 1 to 3 aryl groups, e.g., benzyl, phenethyl, 3-phenylpropyl, α-naphthylmethyl, β-naphthylmethyl, diphenylmethyl, triphenylmethyl, 6-phenylhexyl, α-naphthyldiphenylmethyl and 9-anthrylmethyl and etc., and a lower alkyl group substituted by 1 to 3 aryl group of which aryl ring is substituted by a lower alkyl group, a lower alkoxy group, a nitro group, a halogen atom, a cyano group or an alkoxycarbonyl group, e.g., 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl, 4-cyanobenzyl, 4-cyanobenzyldiphenylmethyl, bis(2-nitrophenyl)methyl, piperonyl, 4-methoxycarbonylbenzyl and etc.; and "a silyl group" such as trimethylsilyl, triethylsilyl, isopropyldimethylsilyl, tertbutyldimethylsilyl, methyldiisopropylsilyl, methyldi-tert-butylsilyl, triisopropylsilyl, methyldiphenylsilyl, isopropyldiphenylsilyl, butyldiphenylsilyl, phenyldiisopropylsilyl, and etc.

"The protective group cleavable by a biological method such as hydrolysis in a living body" represents a protective group which is cleaved by a biological method such as hydrolysis in a human body and produces free acids or the salts thereof. Whether a derivative is cleavable or not can be determined by administering it to an experimental animal such as a rat or mouse by an intravenous injection, analyzing a body fluid of the animal to detect the original compound or the pharmacologically acceptable salt thereof.

"The protective group cleavable by a biological method such as hydrolysis in a living body" relating to "the ester of the hydroxy group" may include a 1-("aliphatic acyl"oxy) "lower alkyl group" such as formyloxymethyl, acetoxymethyl, dimethylaminoacetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl, 1-pivaloyloxyhexyl and etc.; a 1-("aliphatic acyl"thio) "lower alkyl group" such as formylthiomethyl, acetylthiomethyl, dimethylaminoacetylthiomethyl, propionylthiomethyl, butyrylthiomethyl, pivaloylthiomethyl, valerylthiomethyl, isovalerylthiomethyl, hexanoylthiomethyl, 1-formylthioethyl, 1-acetylthioethyl, 1-propionylthioethyl, 1-butyrylthioethyl, 1-pivaloylthioethyl, 1-valerylthioethyl, 1-isovalerylthioethyl, 1-hexanoylthioethyl, 1-formylthiopropyl, 1-acetylthiopropyl, 1-propionylthiopropyl, 1-butyrylthiopropyl, 1-pivaloylthiopropyl, 1-valerylthiopropyl, 1-isovalerylthiopropyl, 1-hexanoylthiopropyl, 1-acetylthiobutyl, 1-propionylthiobutyl, 1-butyrylthiobutyl, 1-pivaloylthiobutyl, 1-acetylthiopentyl, 1-propionylthiopentyl, 1-butyrylthiopentyl, 1-pivaloylthiopentyl, 1-pivaloylthiohexyl and etc.; a 1-(acyloxy) "lower alkyl group" such as a 1-("cycloalkyl"carbonyloxy) "lower alkyl group", e.g., cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl and 1-cyclohexylcarbonyloxybutyl, 1-("aromatic acyl"oxy) "lower alkyl group", e.g., benzoyloxymethyl; (alkoxycarbonyloxy)alkyl group such as methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1- (ethoxycarbonyloxy) ethyl, 1-(propoxycarbonyloxy)ethyl, 1-(isopropoxycarbonyloxy)ethyl, 1-(butoxycarbonyloxy)ethyl, 1-(isobutoxycarbonyloxy)ethyl, 1-(tert-butoxycarbonyloxy)ethyl, 1-(pentyloxycarbonyloxy)ethyl, 1-(hexyloxycarbonyloxy)ethyl, 1-(cyclopentyloxycarbonyloxy)ethyl, 1-(cyclopentyloxycarbonyloxy)propyl, 1-(cyclohexyloxycarbonyloxy)propyl, 1-(cyclopentyloxycarbonyloxy)butyl, 1-(cyclohexyloxycarbonyloxy)butyl, 1-(cyclohexyloxycarbonyloxy)ethyl, 1-(ethoxycarbonyloxy)propyl, 2-(methoxycarbonyloxy)ethyl, 2-(ethoxycarbonyloxy)ethyl, 2-(propoxycarbonyloxy)ethyl, 2-(isopropoxycarbonyloxy)ethyl, 2-(butoxycarbonyloxy)ethyl, 2-(isobutoxycarbonyloxy)ethyl, 2-(pentyloxycarbonyloxy)ethyl, 2-(hexyloxycarbonyloxy)ethyl, 1-(methoxycarbonyloxy)propyl, 2-(ethoxycarbonyloxy)propyl, 1-(propoxycarbonyloxy)propyl, 1- (isopropoxycarbonyloxy)propyl, 1-(butoxycarbonyloxy)propyl, 1- (isobutoxycarbonyloxy) propyl, 1-(pentyloxycarbonyloxy)propyl, 1-(hexyloxycarbonyloxy)propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, 1-(methoxycarbonyloxy)pentyl, 1-(ethoxycarbonyloxy)pentyl, 1-(methoxycarbonyloxy)hexyl, 1-(ethoxycarbonyloxy)hexyl and etc.; "a phthalidyl group" such as phthalidyl, dimethylphthalidyl and dimethoxyphthalidyl; "a carbonyloxyalkyl group" such as an oxodioxolenylmethyl group, e.g., (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl, (5-butyl-2-oxo-1,3-dioxolen-4-yl)methyl and etc.; the above-mentioned "aliphatic acyl group"; the above-mentioned "aromatic acyl group"; "a half ester salt residual group of succinic acid"; "a phosphoric acid ester salt residual group"; "ester-forming residual group of amino acid, and etc."; a carbamoyl group; a carbamoyl group substituted by 1 or 2 lower alkyl groups; a carboxyl "lower alkyl group" dithioethyl group such as 2-carboxyl ethyldithioethyl, 3-carboxyl propyldithioethyl, 4-carboxyl butyldithioethyl, 5-carboxyl pentyldithioethyl, 6-carboxyl hexyldithioethyl and etc.; and "a lower alkyl group" dithioethyl group such as methyldithioethyl, ethyldithioethyl, propyldithioethyl, butyldithioethyl, pentyldithioethyl, hexyldithioethyl and etc.

Whereas, "the protective group cleavable by a biological method such as hydrolysis in a living body" relating to the "ester of the carboxy group" may specifically include "alkoxy lower alkyl group" such as a lower alkoxy lower alkyl group, e.g., methoxymethyl, 1-ethoxyethyl, 1-methyl-1-methoxyethyl, 1-(isopropoxy)ethyl, 2-methoxyethyl, 2-ethoxyethyl, 1,1-dimethyl-1-methoxymethyl, ethoxymethyl, n-propoxymethyl, isopropoxymethyl, n-butoxymethyl, tert-butoxymethyl and etc., a lower alkoxylated lower alkoxy lower alkyl group, e.g., 2-methoxyethoxymethyl, an "aryl"oxy "lower alkyl group", e.g., phenoxymethyl and a halogenated lower alkoxy lower alkyl group, e.g., 2,2,2-trichloroethoxymethyl, bis(2-chloroethoxy)methyl and etc.; a "lower alkoxy" carbonyl "lower alkyl group" such as methoxycarbonylmethyl; a cyano "lower alkyl group" such as cyanomethyl and 2-cyanoethyl; a "lower alkyl" thiomethyl group such as methylthiomethyl and ethylthiomethyl; an "aryl" thiomethyl group such as phenylthiomethyl, naphthylthiomethyl and etc.; a "lower alkyl" sulfonyl "lower alkyl group" which may be substituted by halogen atoms such as 2-methanesulfonylethyl and 2-trifluoromethanesulfonylethyl; an "aryl" sulfonyl "lower alkyl group" such as 2-benzenesulfonylethyl and 2-toluenesulfonylethyl; an acyloxy "lower alkyl group" such as an "aliphatic acyl" oxy "lower alkyl group", e.g., formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 2-formyloxyethyl, 2-acetoxyethyl, 2-propionyloxyethyl, 2-butyryloxyethyl, 2-pivaloyloxyethyl, 2-valeryloxyethyl, 2-isovaleryloxyethyl, 2-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl, 1-pivaloyloxyhexyl and etc., a "cycloalkyl" carbonyloxy "lower alkyl group", e.g., cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl, 1-cyclohexylcarbonyloxybutyl and etc., and an "aromatic acyl" oxy "lower alkyl group", e.g., benzoyloxymethyl; an (alkoxycarbonyloxy)alkyl group such as methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1-(ethoxycarbonyloxy)ethyl, 1-(propoxycarbonyloxy)ethyl, 1- (isopropoxycarbonyloxy) ethyl, 1-(butoxycarbonyloxy)ethyl, 1-(isobutoxycarbonyloxy)ethyl, 1-(tert-butoxycarbonyloxy)ethyl, 1-(pentyloxycarbonyloxy)ethyl, 1-(hexyloxycarbonyloxy)ethyl, 1-(cyclopentyloxycarbonyloxy)ethyl, 1-(cyclopentyloxycarbonyloxy)propyl, 1-(cyclohexyloxycarbonyloxy)propyl, 1-(cyclopentyloxycarbonyloxy)butyl, 1-(cyclohexyloxycarbonyloxy)butyl, 1-(cyclohexyloxycarbonyloxy)ethyl, 1-(ethoxycarbonyloxy)propyl, 2-(methoxycarbonyloxy)ethyl, 2-(ethoxycarbonyloxy)ethyl, 2-(propoxycarbonyloxy)ethyl, 2-(isopropoxycarbonyloxy)ethyl, 2-(butoxycarbonyloxy)ethyl, 2-(isobutoxycarbonyloxy)ethyl, 2-(pentyloxycarbonyloxy)ethyl, 2-(hexyloxycarbonyloxy)ethyl, 1-(methoxycarbonyloxy)propyl, 1-(ethoxycarbonyloxy)propyl, 1-(propoxycarbonyloxy)propyl, 1- (isopropoxycarbonyloxy)propyl, 1-(butoxycarbonyloxy)propyl, 1-(isobutoxycarbonyloxy)propyl, 1-(pentyloxycarbonyloxy)propyl, 1-(hexyloxycarbonyloxy)propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, 1-(methoxycarbonyloxy)pentyl, 1- (ethoxycarbonyloxy)pentyl, 1-(methoxycarbonyloxy)hexyl, 1-(ethoxycarbonyloxy)hexyl and etc.; "a carbonyloxyalkyl group" such as an oxodioxolenylmethyl group, e.g., (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl, (5-butyl-2-oxo-1,3-dioxolen-4-yl)methyl and etc.; "a phthalidyl group" such as phthalidyl, dimethylphthalidyl and dimethoxyphthalidyl; "an aryl group" such as phenyl, indanyl and etc.; the above-mentioned "lower alkyl group"; and a straight or branched alkylthio group having 1 to 6 carbon atoms such as methylthio, ethylthio, n-propylthio, isopropylthio, n-butylthio, isobutylthio, s-butylthio, tert-butylthio, n-pentylthio, isopentylthio, 2-methylbutylthio, neopentylthio, 1-ethylpropylthio, n-hexylthio, isohexylthio, 4-methylpentylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, 1,3-dimethylbutylthio, 2,3-dimethylbutylthio, 2-ethylbutylthio and etc., preferably "an alkylthio group" having 1 to 4 carbon atoms; a "carboxyalkyl group" such as carboxymethyl; and an "amide-forming residual group of an amino acid" such as phenylalanine.

"Other derivatives" means an ether derivative or a carbamoyloxy derivative in the case where Compound (I) of the present invention has "a hydroxy group", or means an amide derivative in the case where the Compound (I) of the present invention has "an amino group", that are decomposed in the living body to form their respective original "hydroxy group" or "amino group". A determination whether or not a derivative is such a kind, can be made by administering it by intravenous injection to an experimental animal such as a rat or a mouse, analyzing the animal's body fluids afterward, to detect the original compound or its pharmacologically acceptable salt.

Compound (I) of the present invention may occasionally have an asymmetrical carbon in the molecule, and stereoisomers of R and S-configuration sometimes exist. Each of the stereoisomers or mixtures containing such stereoisomers in an arbitrary proportion thereof are all included in the present invention.

Moreover, the external preparation of the present invention is an external preparation which comprises at least one compound of the above nitroimidazole derivatives and at least one agent selected from the group consisting of an antimycotic agent, antibacterial agent, sulfa, immunosuppressant, anti-inflammatory drug, antibiotic, antiviral agent, metabolic antagonist, antihistamine, tissue repair promoter, vitamin, antiallergic, local anesthetic, hair agent and steroid being administered simultaneously or separately with an interval.

In the above, there are no particular restrictions on "administered simultaneously" provided it is an administration form that can be administered at nearly the same time, it is preferable to administer the preparation in the form of a single composition.

In the above, there are no particular restrictions on "administered separately with an interval" provided it is an administration form that can be administered separately with an interval. It refers to, for example, administration of a nitroimidazole derivative on day 1 followed by administration on day 2 of a preparation containing at least one agent selected from the group consisting of an antimycotic agent, antibacterial agent, sulfa, immunosuppressant, anti-inflammatory agent, antibiotic, antiviral agent, metabolic antagonist, antihistamine, tissue repair promoter, vitamin, antiallergic, local anesthetic, hair agent and steroid, or to initially administering a nitroimidazole derivative and then with a predetermined interval, administering a preparation containing at least one agent selected from the group consisting of an antimycotic agent, antibacterial agent, sulfa, immunosuppressant, anti-inflammatory agent, antibiotic, antiviral agent, metabolic antagonist, antihistamine, tissue repair promoter, vitamin, antiallergic, local anesthetic, hair agent, steroid and the like.

Among at least one agent selected from an antimycotic agent, antibacterial agent, sulfa, immunosuppressant, anti-inflammatory agent, antibiotic, antiviral agent, metabolic antagonist, antihistamine, tissue repair promoter, vitamin, antiallergic, local anesthetic, hair agent and steroid in the above description, an antimycotic agent, immunosuppressant, steroid and their combinations are preferable for an external preparation for the therapeutic or prophylactic treatment of atopic dermatitis, while an immunosuppressant, steroid and the combination of antimycotic agent and steroid are more preferable.

In the above, the agent of at least one agent selected from the group consisting of an antimycotic agent, antibacterial agent, sulfa, immunosuppressant, anti-inflammatory agent, antibiotic, antiviral agent, metabolic antagonist, antihistamine, tissue repair promoter, vitamin, antiallergic, local anesthetic, hair agent and steroid, is preferably used in a concentration at which the agent itself does not demonstrate any pharmacological effect. Determination on whether or not a concentration is at a level at which a pharmacological effect is demonstrated can be easily done by a person with ordinary skill in the art using commonly known means (such as comparative studies in humans or animals).

There are no particular restrictions on the content in a preparation in the case of containing in an external preparation an antimycotic agent, antibacterial agent, sulfa, immunosuppressant, anti-inflammatory agent, antibiotic, antiviral agent, metabolic antagonist, antihistamine, tissue repair promoter, vitamin, antiallergic, local anesthetic, hair agent or steroid, provided that the concentration is not that at which adverse side effects are exhibited. Based on the weight of the preparation, the content is preferably 0.0005 to 2 wt%, and more preferably 0.01 to 0.5 wt% for an antimycotic agent, preferably 0.001 to 5 wt%, and more preferably 0.01 to 0.5 wt% for an antibacterial agent, preferably 0.001 to 5 wt%, and more preferably 0.01 to 0.5 wt% for a sulfa, preferably 0.001 to 5 wt%, and more preferably 0.01 to 0.1 wt% for an immunosuppressant, preferably 0.001 to 5 wt%, and more preferably 0.005 to 0.5 wt% for an anti-inflammatory agent, preferably 0.0001 to 5 wt%, and more preferably 0.001 to 0.1 wt% for an antibiotic, preferably 0.01 to 5 wt%, and more preferably 0.1 to 1 wt% for an antiviral agent, preferably 0.01 to 5 wt%, and more preferably 0.01 to 0.5 wt% for a metabolic antagonist, preferably 0.001 to 10 wt%, and more preferably 0.01 to 5 wt% for an antihistamine, preferably 0.1 to 20 wt%, and more preferably 0.1 to 5 wt% for a tissue repair promoter, preferably 0.000001 to 0.005 wt%, and more preferably 0.00001 to 0.001 wt% for a vitamin, preferably 0.001 to 5 wt%, and more preferably 0.01 to 0.5 wt% for an antiallergic, preferably 0.001 to 5 wt%, and more preferably 0.01 to 1 wt% for a local anesthetic, preferably 0.01 to 10 wt%, and more preferably 0.1 to 2 wt% for a hair agent, and preferably 0.001 to 1 wt%, and more preferably 0.001 to 0.1 wt% for a steroid.

There are no particular restrictions on the above antimycotic agent provided it is an agent that is used for the treatment of pathogenic molds and deep mycoses, examples of which include imidazole compounds such as croconazole hydrochloride, neticonazole hydrochloride, clotrimazole, ketoconazole, isoconazole nitrate, econazole nitrate, oxiconazole nitrate, sulconazole nitrate, miconazole nitrate, thioconazole, bifonazole and lanoconazole, as well as amorolfine hydrochloride, terbinafine hydrochloride, butenafine hydrochloride, ciclopirox olamine, tolciclate, tolnaftate and the like.

There are no particular restrictions on the above antibacterial agent provided it is an agent that has efficacy against pathogenic microorganisms (including Gram positive cocci and bacilli, and Gram negative cocci and bacilli), examples of which include enoxacin, methyl rosaniline chloride, ciprofloxacin hydrochloride, lomefloxacin hydrochloride, ofloxacin, cinoxacin, sparfloxacin, tosufloxacin tosilate, nalidixic acid, norfloxacin, pipemidic acid trihydrate, piromidic acid, fleroxacin, levofloxacin, etc. and their derivatives.

There are no particular restrictions on the above sulfa provided it is used routinely, examples of which include acetylsulfamethoxazole, salazosulfapyridine, sulfadiazine, sulfadiazine silver, sulfadimethoxine, sulfathiazole, sulfaphenazole, sulfamethoxazole, sulfamethoxypyridazine, sulfamethopyradine, sulfamethomidine, sulfamethizole, sulfameradine, sulfamonomethoxine, sulfisoxazole, sulfisomidin, sulfisomidin sodium, homosulfamine, etc. and their derivatives.

There are no particular restrictions on the above immunosuppressant provided that an agent suppresses immune rejection reactions, examples of which include pimecrolimus, sirolimus, eberolimus, cyclosporin, tacrolimus, glibelimus hydrochloride, mizoribine, FTY-720 (2-amino-2-(2-(4-octylphenyl)ethyl)propane-1,3-diol hydrochloride) and etc.

There are no particular restrictions on the above anti-inflammatory agent provided it is used routinely, examples of which include actarit, azulene, acemetacin, aspirin, alclofenac, alminoprofen, amfenac sodium, ampiroxicam, ibuprofen, ibuprofenpiconol, indometacin, indometacin farnesil, ufenamate, etodolac, epirizol, emorfazone, tiaramide hydrochloride, tinoridine hydrochloride, buprenorphine hydrochloride, pentazocine hydrochloride, enfenam, oxaprozin, glycyrrhetic acid, crotamiton, ketoprofen, zaltoprofen, diflunisal, diclofenac sodium, suprofen, sulindac, tiaprofen, tenoxicam, trimethine sodium, nabumeton, naproxen, nifurmic acid, piroxicam, phenacetin, phenylbutazone, phenoprofen calcium, felbinac, fenbufen, bucolome, bufexamac, pranoprofen, flurbiprofen, floctafenine, dimethothiazine mesilate, methiazine, bendazac, heparin analogues, proglumetacin maleate, meclofenam, mefenamic acid, loxoprofen sodium, lobenzarit disodium, vaccinia virus inoculated rabbit inflammatory skin extract, etc. and derivatives thereof.

The above antibiotic means a substance that inhibits the growth of microorganisms, examples of which include acetylkitasamycin, acetylspiramycin, amphotericin B, amoxicillin, ampicillin, kanamycin monosulfate, erythromycin ethyl succinate, erythromycin, erythromycin estorate, aclarubicin hydrochloride, oxytetracycline hydrochloride, clindamycin hydrochloride, cefetamet pivoxil hydrochloride, cefotiam hexetil hydrochloride, cefcapene pivoxil hydrochloride, cefmenoxime hydrochloride, talampicillin hydrochloride, tetracycline hydrochloride, demethylchlortetracycline hydrochloride, tetracycline hydrochloride, vancomycin hydrochloride, doxycycline hydrochloride, doxorubicin hydrochloride, bacampicillin hydrochloride, clindamycin palmitate hydrochloride, vancomycin hydrochloride, pivmecillinam hydrochloride, bleomycin hydrochloride, minocycline hydrochloride, lincomycin hydrochloride, lenampicillin hydrochloride, carbenicillin sodium, kitasamycin, potassium clavulanate, clarithromycin, griseofulvin, cloxaciline sodium, chloramphenicol, colistin sodium methanesulfonate, cycloserine, midecamycin acetate, ciclacillin, dicloxacillin sodium, siccanin, josamycin, erythromycin stearate, sulbenicillin sodium, cefaclor, cefazolin, propylene glycol cefatrizine, cefadroxil, cefapirin, cefamandole sodium, cefalexin, cefalotin sodium, cefaloridine, cefixime, cefoxitin sodium, cefotaxime sodium, cefotetan, cefoperazone sodium, cefditoren pivoxil, cefdinir, cefsulodin sodium, ceftizoxime sodium, ceftibuten, cefteram pivoxil, cefpiramide sodium, cefbuperazone sodium, cefpodoxime proxetil, cefmetazole sodium, cefradine, cefroxadine, cefuroxime axetil, cefuroxime sodium, ticarcillin sodium, tetracycline, sultamicillin tosilate, tobramycin, trichomycin, nystatin, variotin, chloramphenicol palmitate, piperacillin sodium, pimaricin, faropenem sodium, josamycin propionate, phenethicillin potassium, phenoxymethylpenicillin potassium, benzylpenicillin potassium, benzylpenicillin benzathine, fosfomycin calcium, mitomycin C, midecamycin, tetracycline metaphosphate, latamoxef sodium, rifampicin, astromicin sulfate, amikacin sulfate, kanamycin sulfate, gentamicin sulfate, sisomicin sulfate, dibekacin sulfate, streptomycin sulfate, netilmicin sulfate, fradiomycin sulfate, bleomycin sulfate, bekanamycin sulfate, peplomycin sulfate, polymyxin B sulfate, micronomicin sulfate, ribostamycin sulfate, clindamycin phosphate, roxithromycin, rokitamycin, etc. and derivatives thereof.

The above antiviral agent means an agent that is specific for viruses, examples of which include aciclovir, ganciclovir, sanilvudine, zalcitabine, didanosine, zidovudine, nevirapine, saquinavir mesilate, nelfinavir mesilate, lamivudine, ritonavir, indinavir sulfate, etc. and the addition and substitution products of their salts.

There are no particular restrictions on the above metabolic antagonist provided it is used routinely, examples of which include actinomycin D, L-asparaginase, aceglatone, ubenimex, uracil, etoposide, enocitabine, aclarubicin hydrochloride, idarubicin hydrochloride, irinotecan hydrochloride, epirubicin hydrochloride, dunorubicin hydrochloride, doxorubicin hydrochloride, pirarubicin hydrochloride, fadrozole hydrochloride hydrate, bleomycin hydrochloride, procarbazine hydrochloride, mitoxantrone hydrochloride, carboplatin, carmofur, tamoxifen citrate, toremifene citrate, cyclophosphamide, cisplatin, sizofiran, cytarabine, cytarabine ocfosfate, zinostatin stimalamer, vinorelbine ditartrate, sobuzoxane, thiotepa, tegafur, doxifluridine, docetaxel hydrate, tretinoin, neocarzinostatin, nedaplatin, paclitaxel, bicalutamide, hydroxycarbamide, fosfestrol, busulfan, fluorouracil, flutamide, propylthiouracil, pentostatin, porfimer sodium, methyltestosterone, mepitiostane, G-mercaptopurine riboside, mercaptopurine, methotrexate, melphalan, Streptococcus extract, peplomycin sulfate, vincristine sulfate, vinblastine sulfate, lentinan, etc. and derivatives thereof.

There are no particular restrictions on the above antihistamine provided it is an agent that is specifically antagonistic for histamine, examples of which include cyproheptadine hydrochloride, diphenhydramine hydrochloride, triprolidine hydrochloride, hydroxidine hydrochloride, promethazine hydrochloride, homochlorcyclizine hydrochloride, cimetidine, alimemazine tartrate, diphenhydramine tannate, diphenylpyraline teoclate, hydroxidine pamoate, famotidine, chlorpheniramine maleate, clemastine fumarate, mequitazine, etc. and derivatives thereof.

There are no particular restrictions on the above tissue repair promoter provided it is an agent that promotes tissue repair, examples of which include extract of calves blood, EGF and their derivatives.

The above vitamin refers to vitamins which demonstrate vitamin-like action, examples of which include vitamin D3 analogues such as tacalcitol, mexacalcitol, calcipotriol, and ferecalcitol, vitamin A analogues such as adaparene, tazarotene, alitretinoin, and etretinate, as well as vitamin A, vitamin B group, vitamin C, vitamin D and vitamin E.

There are no particular restrictions on the above antiallergic provided it is used routinely, examples of which include astemizole, amlexanox, ibudilast, ebastine, azelastine hydrochloride, epinastine hydrochloride, ozagrel hydrochloride, ceterizine hydrochloride, oxatomide, sodium cromoglicate, seratrodast, tazanolast, terfenadine, suplatast tosilate, tranilast, emedastine difumarate, ketotifen fumarate, pranlukast hydrate, pemirolast potassium, repirinast, etc. and derivatives thereof.

The above local anesthetic means a drug that is able to anesthetize perception and movement at the location at which it is applied, examples of which include ethyl aminobenzoate, oxybuprocaine hydrochloride, dibucaine hydrochloride, tetracaine hydrochloride, diethylaminoethyl parabutyl-aminobenzoate hydrochloride, procaine hydrochloride, mepivacaine hydrochloride, lidocaine hydrochloride, oxethazaine, lidocaine and etc. and derivatives thereof.

There are no particular restrictions on the above hair agent provided it is used routinely, examples of which include asunaron, carpronium chloride and minoxidil.

There are no particular restrictions on the above steroid provided it is an agent that exhibits action resembling steroid hormones secreted from the adrenal cortex, examples of which include amcinonide, oxymetholone, potassium canrenoate, prednisolone valerate acetate, diflucortolone valerate, dexamethasone valerate, betamethasone valerate, hydrocortisone succinate, prednisolone succinate, chlormadinone acetate, cortisone acetate, diflorasone diacetate, hydrocortisone acetate, paramethasone acetate, fludrocortisone acetate, prednisolone acetate, methenolone acetate, difluprednate, betamethasone dipropionate, dexamethasone, triamcinolone, triamcinolone acetonide, halcinonide, hydrocortisone, flumetasone pivalate, prednisolone farnesylate gel, budesonide, mometasone furoate, fluocinonide, fluocinolone acetonide, fluorometholone, fludroxycortide, prednisolone, alclometasone dipropionate, clobetasol propionate, dexamethasone propionate, deprodone propionate, beclometasone dipropionate, betamethasone, methylprednisolone, clobetasone butyrate, hydrocortisone butyrate, hydrocortisone butyrate propionate, betamethasone butyrate propionate, hydrocortisone sodium phosphate, betamethasone sodium phosphate, etc. and derivatives thereof.

Preferable examples of the above nitroimidazole derivatives include the above-mentioned (1) through (9), and more preferable examples include metronidazole and tinidazole.

In addition, the external preparation for atopic dermatitis of the present invention is preferably an external preparation that contains crotamiton. The containing of crotamiton has the effect of fast-action of antiamyctic effects, increasing solubility of nitroimidazole derivative, and improving stability of the external preparation.

Preferable examples of the present invention include:
(10) atopic dermatitis,
(11) facial atopic dermatitis,
(12) pediatric atopic dermatitis.
In addition, during the above therapeutic or prophylactic treatment or amelioration of skin diseases, an external preparation is also preferable in which a nitroimidazole derivative is arbitrarily selected, and at least one compound of the nitroimidazole derivatives and at least one agent selected from the group consisting of an antimycotic agent, antibacterial agent, sulfa, immunosuppressant, anti-inflammatory agent, antibiotic, antiviral agent, metabolic antagonist, antihistamine, tissue repair promoter, vitamin, antiallergic, local anesthetic, hair agent and steroid are administered simultaneously or separately with an interval, while more preferable examples of external preparations include:
an external preparation for a therapeutic or prophylactic treatment of (10) atopic dermatitis in which the nitroimidazole derivative is metronidazole,
an external preparation for a therapeutic or prophylactic treatment of (11) facial atopic dermatitis in which the nitroimidazole derivative is metronidazole,
an external preparation for a therapeutic or prophylactic treatment of (12) pediatric atopic dermatitis in which the nitroimidazole derivative is metronidazole,
an external preparation for a therapeutic or prophylactic treatment of (10) atopic dermatitis in which the nitroimidazole derivative is metronidazole, and metronidazole and an antimycotic agent, immunosuppressant, steroid or their combination being administered simultaneously or separately with an interval,
an external preparation for a therapeutic or prophylactic treatment of (10) atopic dermatitis in which the nitroimidazole derivative is metronidazole, and metronidazole and immunosuppressant, steroid, or a combination of antimycotic agent and steroid being administered simultaneously or separately with an interval,
an external preparation for a therapeutic or prophylactic treatment of (10) atopic dermatitis in which the nitroimidazole derivative is tinidazole,
an external preparation for a therapeutic or prophylactic treatment of (11) facial atopic dermatitis in which the nitroimidazole derivative is tinidazole,
an external preparation for a therapeutic or prophylactic treatment of (12) pediatric atopic dermatitis in which the nitroimidazole derivative is tinidazole,
an external preparation for a therapeutic or prophylactic treatment of (10) atopic dermatitis in which the nitroimidazole derivative is tinidazole, and tinidazole and an antimycotic agent, immunosuppressant, steroid or their combination being administered simultaneously or separately with an interval,
an external preparation for a therapeutic or prophylactic treatment of (10) atopic dermatitis in which the nitroimidazole derivative is tinidazole, and tinidazole and immunosuppressant, steroid, or a combination of antimycotic agents and steroid being administered simultaneously or separately with an interval,
in addition to diseases of humans, diseases of other mammals (such as dogs or cats) are also included in the targets of the above amelioration, therapeutic or prophylactic treatment.

There are no particular restrictions on the form of the external preparation for skin diseases of the present invention provided it is used routinely, preferable examples of which include cream, lotion, shampoo, gel, rinse, face lotion, milky lotion, paste, shaving cream, foundation, cologne, pack, ointment, patch, semi-solid, solid or liquid. In the treatment of atopic dermatitis of the head region in particular, an external preparation such as shampoo, gel or rinse is useful since cream or ointment and so forth is difficult to use.

Although there are no particular restrictions on the concentration of the nitroimidazole derivative in the external preparation for skin diseases of the present invention provided it is a concentration at which effects are demonstrated, the concentration is preferably 0.1 to 20 wt%, more preferably 1.0 to 10 wt%, further more preferably 1.5 to 10 wt%, and most preferably 1.5 to 5 wt%, based on the weight of the preparation.

Although there are no particular restrictions on the overall pH of the external preparation for skin diseases of the present invention provided it is pH that is used routinely, pH is preferably 2.0 to 9.0, more preferably 3.0 to 9.0 and particularly preferably 4.0 to 9.0.

In the present invention, a nitroimidazole derivative is used to produce an external preparation for therapeutic or prophylactic treatment of atopic dermatitis.

In the present invention, therapeutic or prophylactic treatment of atopic dermatitis, is performed using an external preparation for skin diseases that contains the nitroimidazole derivative.

Specific compounds included in the nitroimidazole derivatives of the present invention are exemplified in Table 1, but are not limited thereto.

In Table 1, Me represents a methyl group, Et an ethyl group, Pr a propyl group, iPr an isopropyl group, Bu a butyl group, Pn a pentyl group, Hx a hexyl group, Ac an acetyl group, Bn a benzyl group, Bz a benzoyl group, Car a carbamoyl group and Mor a morpholino group.

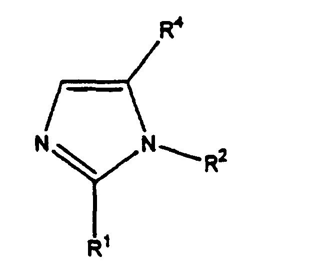

**[Table 1]**

| Compound No. | R¹ | R⁴ | R² |
|---|---|---|---|
| 1 | H | NO₂ | Me |
| 2 | H | NO₂ | CH₂OH |
| 3 | H | NO₂ | CH₂OAc |
| 4 | H | NO₂ | CH₂OBn |
| 5 | H | NO₂ | CH₂OBz |
| 6 | H | NO₂ | CH₂SH |
| 7 | H | NO₂ | CH₂SO₂Me |
| 8 | H | NO₂ | CH₂SO₂Et |
| 9 | H | NO₂ | CH₂SO₂CH₂CH₂OH |
| 10 | H | NO₂ | CH₂SO₂CH₂CH₂OAc |
| 11 | H | NO₂ | CH₂SO₂CH₂CH₂OBz |
| 12 | H | NO₂ | CH₂SO₂CH₂CH₂OBn |
| 13 | H | NO₂ | CH₂SO₂CH₂CH₂SH |
| 14 | H | NO₂ | CH₂SO₂Pr |
| 15 | H | NO₂ | CH₂SO₂iPr |
| 16 | H | NO₂ | CH₂SO₂Bu |
| 17 | H | NO₂ | CH₂SO₂Pn |
| 18 | H | NO₂ | CH₂SO₂Hx |
| 19 | H | NO₂ | CH₂OCar |
| 20 | H | NO₂ | CH₂NHCO₂Me |
| 21 | H | NO₂ | CH₂NHC(S)OMe |
| 22 | H | NO₂ | Et |
| 23 | H | NO₂ | CH₂CH₂OH |
| 24 | H | NO₂ | CH₂CH₂OAc |
| 25 | H | NO₂ | CH₂CH₂OBn |
| 26 | H | NO₂ | CH₂CH₂OBz |
| 27 | H | NO₂ | CH₂CH₂SH |
| 28 | H | NO₂ | CH₂CH₂SO₂Me |
| 29 | H | NO₂ | CH₂CH₂SO₂Et |
| 30 | H | NO₂ | CH₂CH₂SO₂CH₂CH₂OH |
| 31 | H | NO₂ | CH₂CH₂SO₂CH₂CH₂OAc |
| 32 | H | NO₂ | CH₂CH₂SO₂CH₂CH₂OBz |
| 33 | H | NO₂ | CH₂CH₂SO₂CH₂CH₂OBn |
| 34 | H | NO₂ | CH₂CH₂SO₂CH₂CH₂SH |
| 35 | H | NO₂ | CH₂CH₂SO₂Pr |
| 36 | H | NO₂ | CH₂CH₂SO₂iPr |
| 37 | H | NO₂ | CH₂CH₂SO₂Bu |
| 38 | H | NO₂ | CH₂CH₂SO₂Pn |
| 39 | H | NO₂ | CH₂CH₂SO₂Hx |
| 40 | H | NO₂ | CH₂CH₂OCar |
| 41 | H | NO₂ | CH₂CH₂NHCO₂Me |
| 42 | H | NO₂ | CH₂CH₂NHC(S)OMe |
| 43 | H | NO₂ | CH₂CH₂Mor |
| 44 | H | NO₂ | Pr |
| 45 | H | NO₂ | CH₂CH₂CH₂OH |
| 46 | H | NO₂ | CH₂CH₂CH₂OAc |
| 47 | H | NO₂ | CH₂CH₂CH₂OBn |
| 48 | H | NO₂ | CH₂CH₂CH₂OBz |
| 49 | H | NO₂ | CH₂CH₂CH₂SH |
| 50 | H | NO₂ | CH₂CH(OH)CH₃ |
| 51 | H | NO₂ | CH₂CH(OAc)CH₃ |
| 52 | H | NO₂ | CH₂CH(OBz)CH₃ |
| 53 | H | NO₂ | CH₂CH(SH)CH₃ |
| 54 | H | NO₂ | CH₂CH(OH)CH₂OMe |
| 55 | H | NO₂ | CH₂CH(OAc)CH₂OMe |
| 56 | H | NO₂ | CH₂CH(OBz)CH₂OMe |
| 57 | H | NO₂ | CH₂CH(SH)CH₂OMe |
| 58 | H | NO₂ | CH₂CH(OH)CH₂Cl |
| 59 | H | NO₂ | CH₂CH(OAc)CH₂Cl |
| 60 | H | NO₂ | CH₂CH(OBz)CH₂Cl |
| 61 | H | NO₂ | CH₂CH(SH)CH₂Cl |
| 62 | H | NO₂ | CH₂CH(OH)CH₂F |
| 63 | H | NO₂ | CH₂CH₂CH₂SO₂Me |
| 64 | H | NO₂ | CH₂CH₂CH₂SO₂Et |
| 65 | H | NO₂ | CH₂CH(CH₃)SO₂Et |
| 66 | H | NO₂ | CH₂CH₂CH₂SO₂CH₂CH₂OH |
| 67 | H | NO₂ | CH₂CH₂CH₂SO₂CH₂CH₂OAc |
| 68 | H | NO₂ | CH₂CH₂CH₂SO₂CH₂CH₂OBz |
| 69 | H | NO₂ | CH₂CH₂CH₂SO₂CH₂CH₂OBn |
| 70 | H | NO₂ | CH₂CH₂CH₂SO₂CH₂CH₂SH |
| 71 | H | NO₂ | CH₂CH₂CH₂SO₂Pr |
| 72 | H | NO₂ | CH₂CH₂CH₂SO₂iPr |
| 73 | H | NO₂ | CH₂CH₂CH₂SO₂Bu |
| 74 | H | NO₂ | CH₂CH₂CH₂SO₂Pn |
| 75 | H | NO₂ | CH₂CH₂CH₂SO₂Hx |
| 76 | H | NO₂ | CH₂CH₂CH₂NHCO₂Me |
| 77 | H | NO₂ | CH₂CH₂CH₂NHC(S)OMe |
| 78 | H | NO₂ | Bu |
| 79 | H | NO₂ | CH₂CH₂CH₂CH₂OH |
| 80 | H | NO₂ | CH₂CH₂CH₂CH₂OAc |
| 81 | H | NO₂ | CH₂CH₂CH₂CH₂OBn |
| 82 | H | NO₂ | CH₂CH₂CH₂CH₂OBz |
| 83 | H | NO₂ | CH₂CH₂CH₂CH₂SH |
| 84 | H | NO₂ | CH₂CH(OH)CH₂CH₃ |
| 85 | H | NO₂ | CH₂CH(OAc)CH₂CH₃ |
| 86 | H | NO₂ | CH₂CH(OBz)CH₂CH₃ |
| 87 | H | NO₂ | CH₂CH(SH)CH₂CH₃ |
| 88 | H | NO₂ | CH₂CH(OH)CH₂CH₂OMe |
| 89 | H | NO₂ | CH₂CH₂CH₂CH₂SO₂Me |
| 90 | H | NO₂ | CH₂CH₂CH₂CH₂SO₂Et |
| 91 | H | NO₂ | CH₂CH₂CH₂CH₂SO₂CH₂CH₂OH |
| 92 | H | NO₂ | |
| CH₂CH₂CH₂CH₂SO₂CH₂CH₂OAc | | | |
| 93 | H | NO₂ | |
| CH₂CH₂CH₂CH₂SO₂CH₂CH₂OBz | | | |
| 94 | H | NO₂ | |
| CH₂CH₂CH₂CH₂SO₂CH₂CH₂OBn | | | |
| 95 | H | NO₂ | CH₂CH₂CH₂CH₂SO₂CH₂CH₂SH |
| 96 | H | NO₂ | CH₂CH₂CH₂CH₂SO₂iPr |
| 97 | H | NO₂ | Pn |
| 98 | H | NO₂ | (CH₂)₅OH |
| 99 | H | NO₂ | (CH₂)₅OAc |
| 100 | H | NO₂ | (CH₂)₅OBn |
| 101 | H | NO₂ | (CH₂)₅OBz |
| 102 | H | NO₂ | (CH₂)₅SH |
| 103 | H | NO₂ | CH₂CH(OH)CH₂CH₂CH₃ |
| 104 | H | NO₂ | CH₂CH(OAc)CH₂CH₂CH₃ |
| 105 | H | NO₂ | CH₂CH(OBz)CH₂CH₂CH₃ |
| 106 | H | NO₂ | CH₂CH(SH)CH₂CH₂CH₃ |
| 107 | H | NO₂ | CH₂CH(OH)CH₂CH₂CH₂OMe |
| 108 | H | NO₂ | (CH₂)₅SO₂Me |
| 109 | H | NO₂ | (CH₂)₅SO₂Et |
| 110 | H | NO₂ | (CH₂)₅SO₂CH₂CH₂OH |
| 111 | H | NO₂ | (CH₂)₅SO₂CH₂CH₂OAc |
| 112 | H | NO₂ | (CH₂)₅SO₂CH₂CH₂OBz |
| 113 | H | NO₂ | (CH₂)₅SO₂CH₂CH₂OBn |
| 114 | H | NO₂ | (CH₂)₅SO₂CH₂CH₂SH |
| 115 | H | NO₂ | (CH₂)₅SO₂iPr |
| 116 | H | NO₂ | Hx |
| 117 | H | NO₂ | (CH₂)₆OH |
| 118 | H | NO₂ | (CH₂)₆OAc |
| 119 | H | NO₂ | (CH₂)₆OBn |
| 120 | H | NO₂ | (CH₂)₆OBz |
| 121 | H | NO₂ | (CH₂)₆SH |
| 122 | H | NO₂ | CH₂CH(OH)CH₂CH₂CH₂CH₃ |
| 123 | H | NO₂ | CH₂CH(OAc)CH₂CH₂CH₂CH3 |
| 124 | H | NO₂ | CH₂CH(OBz)CH₂CH₂CH₂CH₃ |
| 125 | H | NO₂ | CH₂CH(SH)CH₂CH₂CH₂CH₃ |
| 126 | H | NO₂ | |
| CH₂CH(OH)CH_{Z}CH₂CH_{Z}CH₂OMe | | | |
| 127 | H | NO₂ | (CH₂)₆SO₂Me |
| 128 | H | NO₂ | (CH₂)₆SO₂Et |
| 129 | H | NO₂ | (CH₂)₆SO₂CH₂CH₂OH |
| 130 | H | NO₂ | (CH₂)₆SO₂CH₂CH₂OAc |
| 131 | H | NO₂ | (CH₂)₆SO₂CH₂CH₂OBz |
| 132 | H | NO₂ | (CH₂)₆SO₂CH₂CH₂OBn |
| 133 | H | NO₂ | (CH₂)₆SO₂CH₂CH₂SH |
| 134 | H | NO₂ | (CH₂)₆SO₂iPr |
| 135 | Me | NO₂ | Me |
| 136 | Me | NO₂ | CH₂OH |
| 137 | Me | NO₂ | CH₂OAc |
| 138 | Me | NO₂ | CH₂OBn |
| 139 | Me | NO₂ | CH₂OBz |
| 140 | Me | NO₂ | CH₂SH |
| 141 | Me | NO₂ | CH₂SO₂Me |
| 142 | Me | NO₂ | CH₂SO₂Et |
| 143 | Me | NO₂ | CH₂SO₂CH₂CH₂OH |
| 144 | Me | NO₂ | CH₂SO₂CH₂CH₂OAc |
| 145 | Me | NO₂ | CH₂SO₂CH₂CH₂OBz |
| 146 | Me | NO₂ | CH₂SO₂CH₂CH₂OBn |
| 147 | Me | NO₂ | CH₂SO₂CH₂CH₂SH |
| 148 | Me | NO₂ | CH₂SO₂Pr |
| 149 | Me | NO₂ | CH₂SO₂iPr |
| 150 | Me | NO₂ | CH₂SO₂Bu |
| 151 | Me | NO₂ | CH₂SO₂Pn |
| 152 | Me | NO₂ | CH₂SO₂Hx |
| 153 | Me | NO₂ | CH₂OCar |
| 154 | Me | NO₂ | CH₂NHCO₂Me |
| 155 | Me | NO₂ | CH₂NHC(S)OMe |
| 156 | Me | NO₂ | Et |
| 157 | Me | NO₂ | CH₂CH₂OH |
| 158 | Me | NO₂ | CH₂CH₂OAc |
| 159 | Me | NO₂ | CH₂CH₂OBn |
| 160 | Me | NO₂ | CH₂CH₂OBz |
| 161 | Me | NO₂ | CH₂CH₂SH |
| 162 | Me | NO₂ | CH₂CH₂SO₂Me |
| 163 | Me | NO₂ | CH₂CH₂SO₂Et |
| 164 | Me | NO₂ | CH₂CH₂SO₂CH₂CH₂OH |
| 165 | Me | NO₂ | CH₂CH₂SO₂CH₂CH₂OAc |
| 166 | Me | NO₂ | CH₂CH₂SO₂CH₂CH₂OBz |
| 167 | Me | NO₂ | CH₂CH₂SO₂CH₂CH₂OBn |
| 168 | Me | NO₂ | CH₂CH₂SO₂CH₂CH₂SH |
| 169 | Me | NO₂ | CH₂CH₂SO₂Pr |
| 170 | Me | NO₂ | CH₂CH₂SO₂iPr |
| 171 | Me | NO₂ | CH₂CH₂SO₂Bu |
| 172 | Me | NO₂ | CH₂CH₂SO₂Pn |
| 173 | Me | NO₂ | CH₂CH₂SO₂Hx |
| 174 | Me | NO₂ | CH₂CH₂OCar |
| 175 | Me | NO₂ | CH₂CH₂NHCO₂Me |
| 176 | Me | NO₂ | CH₂CH₂NHC(S)OMe |
| 177 | Me | NO₂ | Pr |
| 178 | Me | NO₂ | CH₂CH₂CH₂OH |
| 179 | Me | NO₂ | CH₂CH₂CH₂OAc |
| 180 | Me | NO₂ | CH₂CH₂CH₂OBn |
| 181 | Me | NO₂ | CH₂CH₂CH₂OBz |
| 182 | Me | NO₂ | CH₂CH₂CH₂SH |
| 183 | Me | NO₂ | CH₂CH(OH)CH₃ |
| 184 | Me | NO₂ | CH₂CH(OAc)CH₃ |
| 185 | Me | NO₂ | CH₂CH(OBz)CH₃ |
| 186 | Me | NO₂ | CH₂CH(SH)CH₃ |
| 187 | Me | NO₂ | CH₂CH(OH)CH₂OMe |
| 188 | Me | NO₂ | CH₂CH(OAc)CH₂OMe |
| 189 | Me | NO₂ | CH₂CH(OBz)CH₂OMe |
| 190 | Me | NO₂ | CH₂CH(SH)CH₂OMe |
| 191 | Me | NO₂ | CH₂CH(OH)CH₂Cl |
| 192 | Me | NO₂ | CH₂CH(OAc)CH₂Cl |
| 193 | Me | NO₂ | CH₂CH(OBz)CH₂Cl |
| 194 | Me | NO₂ | CH₂CH(SH)CH₂Cl |
| 195 | Me | NO₂ | CH₂CH(OH)CH₂F |
| 196 | Me | NO₂ | CH₂CH₂CH₂SO₂Me |
| 197 | Me | NO₂ | CH₂CH₂CH₂SO₂Et |
| 198 | Me | NO₂ | CH₂CH(CH₃)SO₂Et |
| 199 | Me | NO₂ | CH₂CH₂CH₂SO₂CH₂CH₂OH |
| 200 | Me | NO₂ | CH₂CH₂CH₂SO₂CH₂CH₂OAc |
| 201 | Me | NO₂ | CH₂CH₂CH₂SO₂CH₂CH₂OBz |
| 202 | Me | NO₂ | CH₂CH₂CH₂SO₂CH₂CH₂OBn |
| 203 | Me | NO₂ | CH₂CH₂CH₂SO₂CH₂CH₂SH |
| 204 | Me | NO₂ | CH₂CH₂CH₂SO₂Pr |
| 205 | Me | NO₂ | CH₂CH₂CH₂SO₂iPr |
| 206 | Me | NO₂ | CH₂CH₂CH₂SO₂Bu |
| 207 | Me | NO₂ | CH₂CH₂CH₂SO₂PnH₃ |
| 208 | Me | NO₂ | CH₂CH₂CH₂SO₂Hx |
| 209 | Me | NO₂ | CH₂CH₂CH₂NHCO₂Me |
| 210 | Me | NO₂ | CH₂CH₂CH₂NHC(S)OMe |
| 211 | Me | NO₂ | Bu |
| 212 | Me | NO₂ | CH₂CH₂CH₂CH₂OH |
| 213 | Me | NO₂ | CH₂CH₂CH₂CH₂OAc |
| 214 | Me | NO₂ | CH₂CH₂CH₂CH₂OBn |
| 215 | Me | NO₂ | CH₂CH₂CH₂CH₂OBz |
| 216 | Me | NO₂ | CH₂CH₂CH₂CH₂SH |
| 217 | Me | NO₂ | CH₂CH(OH)CH₂CH₃ |
| 218 | Me | NO₂ | CH₂CH(OAc)CH₂CH₃ |
| 219 | Me | NO₂ | CH₂CH(OBz)CH₂CH₃ |
| 220 | Me | NO₂ | CH₂CH(SH)CH₂CH₃ |
| 221 | Me | NO₂ | CH₂CH(OH)CH₂CH₂OMe |
| 222 | Me | NO₂ | CH₂CH₂CH₂CH₂SO₂Me |
| 223 | Me | NO₂ | CH₂CH₂CH₂CH₂SO₂Et |
| 224 | Me | NO₂ | CH₂CH₂CH₂CH₂SO₂CH₂CH₂OH |
| 225 | Me | NO₂ | |
| CH₂CH₂CH₂CH₂SO₂CH₂CH₂OAc | | | |
| 226 | Me | NO₂ | |
| CH₂CH₂CH₂CH₂SO₂CH₂CH₂OBz | | | |
| 227 | Me | NO₂ | |
| CH₂CH₂CH₂CH₂SO₂CH₂CH₂OBn | | | |
| 228 | Me | NO₂ | CH₂CH₂CH₂CH₂SO₂CH₂CH₂SH |
| 229 | Me | NO₂ | CH₂CH₂CH₂CH₂SO₂iPr |
| 230 | Me | NO₂ | Pn |
| 231 | Me | NO₂ | (CH₂)₅OH |
| 232 | Me | NO₂ | (CH₂)₅OAc |
| 233 | Me | NO₂ | (CH₂)₅OBn |
| 234 | Me | NO₂ | (CH₂)₅OBz |
| 235 | Me | NO₂ | (CH₂)₅SH |
| 236 | Me | NO₂ | CH₂CH(OH)CH₂CH₂CH₃ |
| 237 | Me | NO₂ | CH₂CH(OAc)CH₂CH₂CH₃ |
| 238 | Me | NO₂ | CH₂CH(OBz)CH₂CH₂CH₃ |
| 239 | Me | NO₂ | CH₂CH(SH)CH₂CH₂CH₃ |
| 240 | Me | NO₂ | CH₂CH(OH)CH₂CH₂CH₂OMe |
| 241 | Me | NO₂ | (CH₂)₅SO₂Me |
| 242 | Me | NO₂ | (CH₂)₅SO₂Et |
| 243 | Me | NO₂ | (CH₂)₅SO₂CH₂CH₂OH |
| 244 | Me | NO₂ | (CH₂)₅SO₂CH₂CH₂OAc |
| 245 | Me | NO₂ | (CH₂)₅SO₂CH₂CH₂OBz |
| 246 | Me | NO₂ | (CH₂)₅SO₂CH₂CH₂OBn |
| 247 | Me | NO₂ | (CH₂)₅SO₂CH₂CH₂SH |
| 248 | Me | NO₂ | (CH₂)₅SO₂iPr |
| 249 | Me | NO₂ | Hx |
| 250 | Me | NO₂ | (CH₂)₆OH |
| 251 | Me | NO₂ | (CH₂)₆OAc |
| 252 | Me | NO₂ | (CH₂)₆OBn |
| 253 | Me | NO₂ | (CH₂)₆OBz |
| 254 | Me | NO₂ | (CH₂)₆SH |
| 255 | Me | NO₂ | CH₂CH(OH)CH₂CH₂CH₂CH₃ |
| 256 | Me | NO₂ | CH₂CH(OAc)CH₂CH₂CH₂CH₃ |
| 257 | Me | NO₂ | CH₂CH(OBz)CH₂CH₂CH₂CH₃ |
| 258 | Me | NO₂ | CH₂CH(SH)CH₂CH₂CH₂CH₃ |
| 259 | Me | NO₂ | |
| CH₂CH(OH)CH₂CH₂CH₂CH₂OMe | | | |
| 260 | Me | NO₂ | (CH₂)₆SO₂Me |
| 261 | Me | NO₂ | (CH₂)₆SO₂Et |
| 262 | Me | NO₂ | (CH₂)₆SO₂CH₂CH₂OH |
| 263 | Me | NO₂ | (CH₂)₆SO₂CH₂CH₂OAc |
| 264 | Me | NO₂ | (CH₂)₆SO₂CH₂CH₂OBz |
| 265 | Me | NO₂ | (CH₂)₆SO₂CH₂CH₂OBn |
| 266 | Me | NO₂ | (CH₂)₆SO₂CH₂CH₂SH |
| 267 | Me | NO₂ | (CH₂)₆SO₂iPr |
| 268 | Et | NO₂ | Me |
| 269 | Et | NO₂ | CH₂OH |
| 270 | Et | NO₂ | CH₂OAc |
| 271 | Et | NO₂ | CH₂OBn |
| 272 | Et | NO₂ | CH₂OBz |
| 273 | Et | NO₂ | CH₂SH |
| 274 | Et | NO₂ | CH₂SO₂Me |
| 275 | Et | NO₂ | CH₂SO₂Et |
| 276 | Et | NO₂ | CH₂SO₂CH₂CH₂OH |
| 277 | Et | NO₂ | CH₂SO₂CH₂CH₂OAc |
| 278 | Et | NO₂ | CH₂SO₂CH₂CH₂OBz |
| 279 | Et | NO₂ | CH₂SO₂CH₂CH₂OBn |
| 280 | Et | NO₂ | CH₂SO₂CH₂CH₂SH |
| 281 | Et | NO₂ | CH₂SO₂Pr |
| 282 | Et | NO₂ | CH₂SO₂iPr |
| 283 | Et | NO₂ | CH₂SO₂Bu |
| 284 | Et | NO₂ | CH₂SO₂Pn |
| 285 | Et | NO₂ | CH₂SO₂Hx |
| 286 | Et | NO₂ | CH₂OCar |
| 287 | Et | NO₂ | CH₂NHCO₂Me |
| 288 | Et | NO₂ | CH₂NHC(S)OMe |
| 289 | Et | NO₂ | Et |
| 290 | Et | NO₂ | CH₂CH₂OH |
| 291 | Et | NO₂ | CH₂CH₂OAc |
| 292 | Et | NO₂ | CH₂CH₂OBn |
| 293 | Et | NO₂ | CH₂CH₂OBz |
| 294 | Et | NO₂ | CH₂CH₂SH |
| 295 | Et | NO₂ | CH₂CH₂SO₂Me |
| 296 | Et | NO₂ | CH₂CH₂SO₂Et |
| 297 | Et | NO₂ | CH₂CH₂SO₂CH₂CH₂OH |
| 298 | Et | NO₂ | CH₂CH₂SO₂CH₂CH₂OAc |
| 299 | Et | NO₂ | CH₂CH₂SO₂CH₂CH₂OBz |
| 300 | Et | NO₂ | CH₂CH₂SO₂CH₂CH₂OBn |
| 301 | Et | NO₂ | CH₂CH₂SO₂CH₂CH₂SH |
| 302 | Et | NO₂ | CH₂CH₂SO₂Pr |
| 303 | Et | NO₂ | CH₂CH₂SO₂iPr |
| 304 | Et | NO₂ | CH₂CH₂SO₂Bu |
| 305 | Et | NO₂ | CH₂CH₂SO₂Pn |
| 306 | Et | NO₂ | CH₂CH₂SO₂Hx |
| 307 | Et | NO₂ | CH₂CH₂OCar |
| 308 | Et | NO₂ | CH₂CH₂NHCO₂Me |
| 309 | Et | NO₂ | CH₂CH₂NHC(S)OMe |
| 310 | Et | NO₂ | Pr |
| 311 | Et | NO₂ | CH₂CH₂CH₂OH |
| 312 | Et | NO₂ | CH₂CH₂CH₂OAc |
| 313 | Et | NO₂ | CH₂CH₂CH₂OBn |
| 314 | Et | NO₂ | CH₂CH₂CH₂OBz |
| 315 | Et | NO₂ | CH₂CH₂CH₂SH |
| 316 | Et | NO₂ | CH₂CH(OH)CH₃ |
| 317 | Et | NO₂ | CH₂CH(OAc)CH₃ |
| 318 | Et | NO₂ | CH₂CH(OBz)CH₃ |
| 319 | Et | NO₂ | CH₂CH(SH)CH₃ |
| 320 | Et | NO₂ | CH₂CH(OH)CH₂OMe |
| 321 | Et | NO₂ | CH₂CH(OAc)CH₂OMe |
| 322 | Et | NO₂ | CH₂CH(OBz)CH₂OMe |
| 323 | Et | NO₂ | CH₂CH(SH)CH₂OMe |
| 324 | Et | NO₂ | CH₂CH(OH)CH₂Cl |
| 325 | Et | NO₂ | CH₂CH(OAc)CH₂Cl |
| 326 | Et | NO₂ | CH₂CH(OBz)CH₂Cl |
| 327 | Et | NO₂ | CH₂CH(SH)CH₂Cl |
| 328 | Et | NO₂ | CH₂CH(OH)CH₂F |
| 329 | Et | NO₂ | CH₂CH₂CH₂SO₂Me |
| 330 | Et | NO₂ | CH₂CH₂CH₂SO₂Et |
| 331 | Et | NO₂ | CH₂CH(CH₃)SO₂Et |
| 332 | Et | NO₂ | CH₂CH₂CH₂SO₂CH₂CH₂OH |
| 333 | Et | NO₂ | CH₂CH₂CH₂SO₂CH₂CH₂OAc |
| 334 | Et | NO₂ | CH₂CH₂CH₂SO₂CH₂CH₂OBz |
| 335 | Et | NO₂ | CH₂CH₂CH₂SO₂CH₂CH₂OBn |
| 336 | Et | NO₂ | CH₂CH₂CH₂SO₂CH₂CH₂SH |
| 337 | Et | NO₂ | CH₂CH₂CH₂SO₂Pr |
| 338 | Et | NO₂ | CH₂CH₂CH₂SO₂iPr |
| 339 | Et | NO₂ | CH₂CH₂CH₂SO₂Bu |
| 340 | Et | NO₂ | CH₂CH₂CH₂SO₂Pn |
| 341 | Et | NO₂ | CH₂CH₂CH₂SO₂Hx |
| 342 | Et | NO₂ | CH₂CH₂CH₂NHCO₂Me |
| 343 | Et | NO₂ | CH₂CH₂CH₂NHC(S)OMe |
| 344 | Et | NO₂ | Bu |
| 345 | Et | NO₂ | CH₂CH₂CH₂CH₂OH |
| 346 | Et | NO₂ | CH₂CH₂CH₂CH₂OAc |
| 347 | Et | NO₂ | CH₂CH₂CH₂CH₂OBn |
| 348 | Et | NO₂ | CH₂CH₂CH₂CH₂OBz |
| 349 | Et | NO₂ | CH₂CH₂CH₂CH₂SH |
| 350 | Et | NO₂ | CH₂CH(OH)CH₂CH₃ |
| 351 | Et | NO₂ | CH₂CH(OAc)CH₂CH₃ |
| 352 | Et | NO₂ | CH₂CH(OBz)CH₂CH₃ |
| 353 | Et | NO₂ | CH₂CH(SH)CH₂CH₃ |
| 354 | Et | NO₂ | CH₂CH(OH)CH₂CH₂OMe |
| 355 | Et | NO₂ | CH₂CH₂CH₂CH₂SO₂Me |
| 356 | Et | NO₂ | CH₂CH₂CH₂CH₂SO₂Et |
| 357 | Et | NO₂ | CH₂CH₂CH₂CH₂SO₂CH₂CH₂OH |
| 358 | Et | NO₂ | |
| CH₂CH₂CH₂CH₂SO₂CH₂CH₂OAc | | | |
| 359 | Et | NO₂ | |
| CH₂CH₂CH₂CH₂SO₂CH₂CH₂OBz | | | |
| 360 | Et | NO₂ | |
| CH₂CH₂CH₂CH₂SO₂CH₂CH₂OBn | | | |
| 361 | Et | NO₂ | CH₂CH₂CH₂CH₂SO₂CH₂CH₂SH |
| 362 | Et | NO₂ | CH₂CH₂CH₂CH₂SO₂iPr |
| 363 | Et | NO₂ | Pn |
| 364 | Et | NO₂ | (CH₂)₅OH |
| 365 | Et | NO₂ | (CH₂)₅OAc |
| 366 | Et | NO₂ | (CH₂)₅OBn |
| 367 | Et | NO₂ | (CH₂)₅OBz |
| 368 | Et | NO₂ | (CH₂)₅SH |
| 369 | Et | NO₂ | CH₂CH(OH)CH₂CH₂CH₃ |
| 370 | Et | NO₂ | CH₂CH(OAc)CH₂CH₂CH₃ |
| 371 | Et | NO₂ | CH₂CH(OBz)CH₂CH₂CH₃ |
| 372 | Et | NO₂ | CH₂CH(SH)CH₂CH₂CH₃ |
| 373 | Et | NO₂ | CH₂CH(OH)CH₂CH₂CH₂OMe |
| 374 | Et | NO₂ | (CH₂)₅SO₂Me |
| 375 | Et | NO₂ | (CH₂)₅SO₂Et |
| 376 | Et | NO₂ | (CH₂)₅SO₂CH₂CH₂OH |
| 377 | Et | NO₂ | (CH₂)₅SO₂CH₂CH₂OAc |
| 378 | Et | NO₂ | (CH₂)₅SO₂CH₂CH₂OBz |
| 379 | Et | NO₂ | (CH₂)₅SO₂CH₂CH₂OBn |
| 380 | Et | NO₂ | (CH₂)₅SO₂CH₂CH₂SH |
| 381 | Et | NO₂ | (CH₂)₅SO₂iPr |
| 382 | Et | NO₂ | Hx |
| 383 | Et | NO₂ | (CH₂)₆OH |
| 384 | Et | NO₂ | (CH₂)₆OAc |
| 385 | Et | NO₂ | (CH₂)₆OBn |
| 386 | Et | NO₂ | (CH₂)₆OBz |
| 387 | Et | NO₂ | (CH₂)₆SH |
| 388 | Et | NO₂ | CH₂CH(OH)CH₂CH₂CH₂CH₃ |
| 389 | Et | NO₂ | CH₂CH(OAc)CH₂CH₂CH₂CH₃ |
| 390 | Et | NO₂ | CH₂CH(OBz)CH₂CH₂CH₂CH₃ |
| 391 | Et | NO₂ | CH₂CH(SH)CH₂CH₂CH₂CH₃ |
| 392 | Et | NO₂ | |
| CH₂CH(OH)CH₂CH₂CH₂CH₂OMe | | | |
| 393 | Et | NO₂ | (CH₂)₆SO₂Me |
| 394 | Et | NO₂ | (CH₂)₆SO₂Et |
| 395 | Et | NO₂ | (CH₂)₆SO₂CH₂CH₂OH |
| 396 | Et | NO₂ | (CH₂)₆SO₂CH₂CH₂OAc |
| 397 | Et | NO₂ | (CH₂)₆SO₂CH₂CH₂OBz |
| 398 | Et | NO₂ | (CH₂)₆SO₂CH₂CH₂OBn |
| 399 | Et | NO₂ | (CH₂)₆SO₂CH₂CH₂SH |
| 400 | Et | NO₂ | (CH₂)₆SO₂iPr |
| 401 | CH₂OCar | NO₂ | H |
| 402 | CH₂OCar | NO₂ | Me |
| 403 | CH₂OCar | NO₂ | Et |
| 404 | CH₂CH₂OCar | NO₂ | H |
| 405 | CH₂CH₂OCar | NO₂ | Me |
| 406 | CH₂CH₂OCar | NO₂ | Et |
| 407 | NO₂ | H | Me |
| 408 | NO₂ | H | CH₂OH |
| 409 | NO₂ | H | CH₂OAc |
| 410 | NO₂ | H | CH₂OBn |
| 411 | NO₂ | H | CH₂OBz |
| 412 | NO₂ | H | CH₂SH |
| 413 | NO₂ | H | CH₂SO₂Me |
| 414 | NO₂ | H | CH₂SO₂Et |
| 415 | NO₂ | H | CH₂SO₂CH₂CH₂OH |
| 416 | NO₂ | H | CH₂SO₂CH₂CH₂OAc |
| 417 | NO₂ | H | CH₂SO₂CH₂CH₂OBz |
| 418 | NO₂ | H | CH₂SO₂CH₂CH₂OBn |
| 419 | NO₂ | H | CH₂SO₂CH₂CH₂SH |
| 420 | NO₂ | H | CH₂SO₂Pr |
| 421 | NO₂ | H | CH₂SO₂iPr |
| 422 | NO₂ | H | CH₂SO₂Bu |
| 423 | NO₂ | H | CH₂SO₂Pn |
| 424 | NO₂ | H | CH₂SO₂Hx |
| 425 | NO₂ | H | CH₂OCar |
| 426 | NO₂ | H | CH₂NHCO₂Me |
| 427 | NO₂ | H | CH₂NHC(S)OMe |
| 428 | NO₂ | H | Et |
| 429 | NO₂ | H | CH₂CH₂OH |
| 430 | NO₂ | H | CH₂CH₂OAc |
| 431 | NO₂ | H | CH₂CH₂OBn |
| 432 | NO₂ | H | CH₂CH₂OBz |
| 433 | NO₂ | H | CH₂CH₂SH |
| 434 | NO₂ | H | CH₂CH₂SO₂Me |
| 435 | NO₂ | H | CH₂CH₂SO₂Et |
| 436 | NO₂ | H | CH₂CH₂SO₂CH₂CH₂OH |
| 437 | NO₂ | H | CH₂CH₂SO₂CH₂CH₂OAc |
| 438 | NO₂ | H | CH₂CH₂SO₂CH₂CH₂OBz |
| 439 | NO₂ | H | CH₂CH₂SO₂CH₂CH₂OBn |
| 440 | NO₂ | H | CH₂CH₂SO₂CH₂CH₂SH |
| 441 | NO₂ | H | CH₂CH₂SO₂Pr |
| 442 | NO₂ | H | CH₂CH₂SO₂iPr |
| 443 | NO₂ | H | CH₂CH₂SO₂Bu |
| 444 | NO₂ | H | CH₂CH₂SO₂Pn |
| 445 | NO₂ | H | CH₂CH₂SO₂Hx |
| 446 | NO₂ | H | CH₂CH₂OCar |
| 447 | NO₂ | H | CH₂CH₂NHCO₂Me |
| 448 | NO₂ | H | CH₂CH₂NHC(S)OMe |
| 449 | NO₂ | H | Pr |
| 450 | NO₂ | H | CH₂CH₂CH₂OH |
| 451 | NO₂ | H | CH₂CH₂CH₂OAc |
| 452 | NO₂ | H | CH₂CH₂CH₂OBn |
| 453 | NO₂ | H | CH₂CH₂CH₂OBz |
| 454 | NO₂ | H | CH₂CH₂CH₂SH |
| 455 | NO₂ | H | CH₂CH(OH)CH₃ |
| 456 | NO₂ | H | CH₂CH(OAc)CH₃ |
| 457 | NO₂ | H | CH₂CH(OBz)CH₃ |
| 458 | NO₂ | H | CH₂CH(SH)CH₃ |
| 459 | NO₂ | H | CH₂CH(OH)CH₂OMe |
| 460 | NO₂ | H | CH₂CH(OAc)CH₂OMe |
| 461 | NO₂ | H | CH₂CH(OBz)CH₂OMe |
| 462 | NO₂ | H | CH₂CH(SH)CH₂OMe |
| 463 | NO₂ | H | CH₂CH(OH)CH₂Cl |
| 464 | NO₂ | H | CH₂CH(OAc)CH₂Cl |
| 465 | NO₂ | H | CH₂CH(OBz)CH₂Cl |
| 466 | NO₂ | H | CH₂CH(SH)CH₂Cl |
| 467 | NO₂ | H | CH₂CH(OH)CH₂F |
| 468 | NO₂ | H | CH₂CH₂CH₂SO₂Me |
| 469 | NO₂ | H | CH₂CH₂CH₂SO₂Et |
| 470 | NO₂ | H | CH₂CH(CH₃)SO₂Et |
| 471 | NO₂ | H | CH₂CH₂CH₂SO₂CH₂CH₂OH |
| 472 | NO₂ | H | CH₂CH₂CH₂SO₂CH₂CH₂OAc |
| 473 | NO₂ | H | CH₂CH₂CH₂SO₂CH₂CH₂OBz |
| 474 | NO₂ | H | CH₂CH₂CH₂SO₂CH₂CH₂OBn |
| 475 | NO₂ | H | CH₂CH₂CH₂SO₂CH₂CH₂SH |
| 476 | NO₂ | H | CH₂CH₂CH₂SO₂Pr |
| 477 | NO₂ | H | CH₂CH₂CH₂SO₂iPr |
| 478 | NO₂ | H | CH₂CH₂CH₂SO₂Bu |
| 479 | NO₂ | H | CH₂CH₂CH₂SO₂Pn |
| 480 | NO₂ | H | CH₂CH₂CH₂SO₂Hx |
| 481 | NO₂ | H | CH₂CH₂CH₂NHCO₂Me |
| 482 | NO₂ | H | CH₂CH₂CH₂NHC(S)OMe |
| 483 | NO₂ | H | Bu |
| 484 | NO₂ | H | CH₂CH₂CH₂CH₂OH |
| 485 | NO₂ | H | CH₂CH₂CH₂CH₂OAc |
| 486 | NO₂ | H | CH₂CH₂CH₂CH₂OBn |
| 487 | NO₂ | H | CH₂CH₂CH₂CH₂OBz |
| 488 | NO₂ | H | CH₂CH₂CH₂CH₂SH |
| 489 | NO₂ | H | CH₂CH(OH)CH₂CH₃ |
| 490 | NO₂ | H | CH₂CH(OAc)CH₂CH₃ |
| 491 | NO₂ | H | CH₂CH(OBz)CH₂CH₃ |
| 492 | NO₂ | H | CH₂CH(SH)CH₂CH₃ |
| 493 | NO₂ | H | CH₂CH(OH)CH₂CH₂OMe |
| 494 | NO₂ | H | CH₂CH₂CH₂CH₂SO₂Me |
| 495 | NO₂ | H | CH₂CH₂CH₂CH₂SO₂Et |
| 496 | NO₂ | H | CH₂CH₂CH₂CH₂SO₂CH₂CH₂OH |
| 497 | NO₂ | H | |
| CH₂CH₂CH₂CH₂SO₂CH₂CH₂OAc | | | |
| 498 | NO₂ | H | |
| CH₂CH₂CH₂CH₂SO₂CH₂CH₂OBz | | | |
| 499 | NO₂ | H | |
| CH₂*C*H₂CH₂CH₂SO₂CH₂CH₂OBn | | | |
| 500 | NO₂ | H | CH₂CH₂CH₂CH₂SO₂CH₂CH₂SH |
| 501 | NO₂ | H | CH₂CH₂CH₂CH₂SO₂iPr |
| 502 | NO₂ | H | Pn |
| 503 | NO₂ | H | (CH₂)₅OH |
| 504 | NO₂ | H | (CH₂)₅OAc |
| 505 | NO₂ | H | (CH₂)₅OBn |
| 506 | NO₂ | H | (CH₂)₅OBz |
| 507 | NO₂ | H | (CH₂)₅SH |
| 508 | NO₂ | H | CH₂CH(OH)CH₂CH₂CH₃ |
| 509 | NO₂ | H | CH₂CH(OAc)CH₂CH₂CH₃ |
| 510 | NO₂ | H | CH₂CH(OBz)CH₂CH₂CH₃ |
| 511 | NO₂ | H | CH₂CH(SH)CH₂CH₂CH₃ |
| 512 | NO₂ | H | CH₂CH(OH)CH₂CH₂CH₂OMe |
| 513 | NO₂ | H | (CH₂)₅SO₂Me |
| 514 | NO₂ | H | (CH₂)₅SO₂Et |
| 515 | NO₂ | H | (CH₂)₅SO₂CH₂CH₂OH |
| 516 | NO₂ | H | (CH₂)₅SO₂CH₂CH₂OAc |
| 517 | NO₂ | H | (CH₂)₅SO₂CH₂CH₂OBz |
| 518 | NO₂ | H | (CH₂)₅SO₂CH₂CH₂OBn |
| 519 | NO₂ | H | (CH₂)₅SO₂CH₂CH₂SH |
| 520 | NO₂ | H | (CH₂)₅SO₂iPr |
| 521 | NO₂ | H | Hx |
| 522 | NO₂ | H | (CH₂)₆OH |
| 523 | NO₂ | H | (CH₂)₆OAc |
| 524 | NO₂ | H | (CH₂)₆OBn |
| 525 | NO₂ | H | (CH₂)₆OBz |
| 526 | NO₂ | H | (CH₂)₆SH |
| 527 | NO₂ | H | CH₂CH(OH)CH₂CH₂CH₂CH₃ |
| 528 | NO₂ | H | CH₂CH(OAc)CH₂CH₂CH₂CH₃ |
| 529 | NO₂ | H | CH₂CH(OBz)CH₂CH₂CH₂CH₃ |
| 530 | NO₂ | H | CH₂CH(SH)CH₂CH₂CH₂CH₃ |
| 531 | NO₂ | H | |
| CH₂CH(OH)CH₂CH₂CH₂CH₂OMe | | | |
| 532 | NO₂ | H | (CH₂)₆SO₂Me |
| 533 | NO₂ | H | (CH₂)₆SO₂Et |
| 534 | NO₂ | H | (CH₂)₆SO₂CH₂CH₂OH |
| 535 | NO₂ | H | (CH₂)₆SO₂CH₂CH₂OAc |
| 536 | NO₂ | H | (CH₂)₆SO₂CH₂CH₂OBz |
| 537 | NO₂ | H | (CH₂)₆SO₂CH₂CH₂OBn |
| 538 | NO₂ | H | (CH₂)₆SO₂CH₂CH₂SH |
| 539 | NO₂ | H | (CH₂)₆SO₂iPr |
| 540 | NO₂ | Me | Me |
| 541 | NO₂ | Me | CH₂OH |
| 542 | NO₂ | Me | CH₂OAc |
| 543 | NO₂ | Me | CH₂OBn |
| 544 | NO₂ | Me | CH₂OBz |
| 545 | NO₂ | Me | CH₂SH |
| 546 | NO₂ | Me | CH₂SO₂Me |
| 547 | NO₂ | Me | CH₂SO₂Et |
| 548 | NO₂ | Me | CH₂SO₂CH₂CH₂OH |
| 549 | NO₂ | Me | CH₂SO₂CH₂CH₂OAc |
| 550 | NO₂ | Me | CH₂SO₂CH₂CH₂OBz |
| 551 | NO₂ | Me | CH₂SO₂CH₂CH₂OBn |
| 552 | NO₂ | Me | CH₂SO₂CH₂CH₂SH |
| 553 | NO₂ | Me | CH₂SO₂Pr |
| 554 | NO₂ | Me | CH₂SO₂iPr |
| 555 | NO₂ | Me | CH₂SO₂Bu |
| 556 | NO₂ | Me | CH₂SO₂Pn |
| 557 | NO₂ | Me | CH₂SO₂Hx |
| 558 | NO₂ | Me | CH₂OCar |
| 559 | NO₂ | Me | CH₂NHCO₂Me |
| 560 | NO₂ | Me | CH₂NHC(S)OMe |
| 561 | NO₂ | Me | Et |
| 562 | NO₂ | Me | CH₂CH₂OH |
| 563 | NO₂ | Me | CH₂CH₂OAc . |
| 564 | NO₂ | Me | CH₂CH₂OBn |
| 565 | NO₂ | Me | CH₂CH₂OBz |
| 566 | NO₂ | Me | CH₂CH₂SH |
| 567 | NO₂ | Me | CH₂CH₂SO₂Me |
| 568 | NO₂ | Me | CH₂CH₂SO₂Et |
| 569 | NO₂ | Me | CH₂CH₂SO₂CH₂CH₂OH |
| 570 | NO₂ | Me | CH₂CH₂SO₂CH₂CH₂OAc |
| 571 | NO₂ | Me | CH₂CH₂SO₂CH₂CH₂OBz |
| 572 | NO₂ | Me | CH₂CH₂SO₂CH₂CH₂OBn |
| 573 | NO₂ | Me | CH₂CH₂SO₂CH₂CH₂SH |
| 574 | NO₂ | Me | CH₂CH₂SO₂Pr |
| 575 | NO₂ | Me | CH₂CH₂SO₂iPr |
| 576 | NO₂ | Me | CH₂CH₂SO₂Bu |
| 577 | NO₂ | Me | CH₂CH₂SO₂Pn |
| 578 | NO₂ | Me | CH₂CH₂SO₂Hx |
| 579 | NO₂ | Me | CH₂CH₂OCar |
| 580 | NO₂ | Me | CH₂CH₂NHCO₂Me |
| 581 | NO₂ | Me | CH₂CH₂NHC(S)OMe |
| 582 | NO₂ | Me | Pr |
| 583 | NO₂ | Me | CH₂CH₂CH₂OH |
| 584 | NO₂ | Me | CH₂CH₂CH₂OAc |
| 585 | NO₂ | Me | CH₂CH₂CH₂OBn |
| 586 | NO₂ | Me | CH₂CH₂CH₂OBz |
| 587 | NO₂ | Me | CH₂CH₂CH₂SH |
| 588 | NO₂ | Me | CH₂CH(OH)CH₃ |
| 589 | NO₂ | Me | CH₂CH(OAc)CH₃ |
| 590 | NO₂ | Me | CH₂CH(OBz)CH₃ |
| 591 | NO₂ | Me | CH₂CH(SH)CH₃ |
| 592 | NO₂ | Me | CH₂CH(OH)CH₂OMe |
| 593 | NO₂ | Me | CH₂CH(OAc)CH₂OMe |
| 594 | NO₂ | Me | CH₂CH(OBz)CH₂OMe |
| 595 | NO₂ | Me | CH₂CH(SH)CH₂OMe |
| 596 | NO₂ | Me | CH₂CH(OH)CH₂Cl |
| 597 | NO₂ | Me | CH₂CH(OAc)CH₂Cl |
| 598 | NO₂ | Me | CH₂CH(OBz)CH₂Cl |
| 599 | NO₂ | Me | CH₂CH(SH)CH₂Cl |
| 600 | NO₂ | Me | CH₂CH(OH)CH₂F |
| 601 | NO₂ | Me | CH₂CH₂CH₂SO₂Me |
| 602 | NO₂ | Me | CH₂CH₂CH₂SO₂Et |
| 603 | NO₂ | Me | CH₂CH(CH₃)SO₂Et |
| 604 | NO₂ | Me | CH₂CH₂CH₂SO₂CH₂CH₂OH |
| 605 | NO₂ | Me | CH₂CH₂CH₂SO₂CH₂CH₂OAc |
| 606 | NO₂ | Me | CH₂CH₂CH₂SO₂CH₂CH₂OBz |
| 607 | NO₂ | Me | CH₂CH₂CH₂SO₂CH₂CH₂OBn |
| 608 | NO₂ | Me | CH₂CH₂CH₂SO₂CH₂CH₂SH |
| 609 | NO₂ | Me | CH₂CH₂CH₂SO₂Pr |
| 610 | NO₂ | Me | CH₂CH₂CH₂SO₂iPr |
| 611 | NO₂ | Me | CH₂CH₂CH₂SO₂Bu |
| 612 | NO₂ | Me | CH₂CH₂CH₂SO₂Pn |
| 613 | NO₂ | Me | CH₂CH₂CH₂SO₂Hx |
| 614 | NO₂ | Me | CH₂CH₂CH₂NHCO₂Me |
| 615 | NO₂ | Me | CH₂CH₂CH₂NHC(S)OMe |
| 616 | NO₂ | Me | Bu |
| 617 | NO₂ | Me | CH₂CH₂CH₂CH₂OH |
| 618 | NO₂ | Me | CH₂CH₂CH₂CH₂OAc |
| 619 | NO₂ | Me | CH₂CH₂CH₂CH₂OBn |
| 620 | NO₂ | Me | CH₂CH₂CH₂CH₂OBz |
| 621 | NO₂ | Me | CH₂CH₂CH₂CH₂SH |
| 622 | NO₂ | Me | CH₂CH(OH)CH₂CH₃ |
| 623 | NO₂ | Me | CH₂CH(OAc)CH₂CH₃ |
| 624 | NO₂ | Me | CH₂CH(OBz)CH₂CH₃ |
| 625 | NO₂ | Me | CH₂CH(SH)CH₂CH₃ |
| 626 | NO₂ | Me | CH₂CH(OH)CH₂CH₂OMe |
| 627 | NO₂ | Me | CH₂CH₂CH₂CH₂SO₂Me |
| 628 | NO₂ | Me | CH₂CH₂CH₂CH₂SO₂Et |
| 629 | NO₂ | Me | CH₂CH₂CH₂CH₂SO₂CH₂CH₂OH |
| 630 | NO₂ | Me | |
| CH₂CH₂CH₂CH₂SO₂CH₂CH₂OAc | | | |
| 631 | NO₂ | Me | |
| CH₂CH₂CH₂CH₂SO₂CH₂CH₂OBz | | | |
| 632 | NO₂ | Me | |
| CH₂CH₂CH₂CH₂SO₂CH₂CH₂OBn | | | |
| 633 | NO₂ | Me | CH₂CH₂CH₂CH₂SO₂CH₂CH₂SH |
| 634 | NO₂ | Me | CH₂CH₂CH₂CH₂SO₂iPr |
| 635 | NO₂ | Me | Pn |
| 636 | NO₂ | Me | (CH₂)₅OH |
| 637 | NO₂ | Me | (CH₂)₅OAc |
| 638 | NO₂ | Me | (CH₂)₅OBn |
| 639 | NO₂ | Me | (CH₂)₅OBz |
| 640 | NO₂ | Me | (CH₂)₅SH |
| 641 | NO₂ | Me | CH₂CH(OH)CH₂CH₂CH₃ |
| 642 | NO₂ | Me | CH₂CH(OAc)CH₂CH₂CH₃ |
| 643 | NO₂ | Me | CH₂CH(OBz)CH₂CH₂CH₃ |
| 644 | NO₂ | Me | CH₂CH(SH)CH₂CH₂CH₃ |
| 645 | NO₂ | Me | CH₂CH(OH)CH₂CH₂CH₂OMe |
| 646 | NO₂ | Me | (CH₂)₅SO₂Me |
| 647 | NO₂ | Me | (CH₂)₅SO₂Et |
| 648 | NO₂ | Me | (CH₂)₅SO₂CH₂CH₂OH |
| 649 | NO₂ | Me | (CH₂)₅SO₂CH₂CH₂OAc |
| 650 | NO₂ | Me | (CH₂)₅SO₂CH₂CH₂OBz |
| 651 | NO₂ | Me | (CH₂)₅SO₂CH₂CH₂OBn |
| 652 | NO₂ | Me | (CH₂)₅SO₂CH₂CH₂SH |
| 653 | NO₂ | Me | (CH₂)₅SO₂iPr |
| 654 | NO₂ | Me | Hx |
| 655 | NO₂ | Me | (CH₂)₆OH |
| 656 | NO₂ | Me | (CH₂)₆OAc |
| 657 | NO₂ | Me | (CH₂)₆OBn |
| 658 | NO₂ | Me | (CH₂)₆OBz |
| 659 | NO₂ | Me | (CH₂)₆SH |
| 660 | NO₂ | Me | CH₂CH(OH)CH₂CH₂CH₂CH₃ |
| 661 | NO₂ | Me | CH₂CH(OAc)CH₂CH₂CH₂CH₃ |
| 662 | NO₂ | Me | CH₂CH(OBz)CH₂CH₂CH₂CH₃ |
| 663 | NO₂ | Me | CH₂CH(SH)CH₂CH₂CH₂CH₃ |
| 664 | NO₂ | Me | |
| CH₂CH(OH)CH₂CH₂CH₂CH₂OMe | | | |
| 665 | NO₂ | Me | (CH₂)₆SO₂Me |
| 666 | NO₂ | Me | (CH₂)₆SO₂Et |
| 667 | NO₂ | Me | (CH₂)₆SO₂CH₂CH₂OH |
| 668 | NO₂ | Me | (CH₂)₆SO₂CH₂CH₂OAc |
| 669 | NO₂ | Me | (CH₂)₆SO₂CH₂CH₂OBz |
| 670 | NO₂ | Me | (CH₂)₆SO₂CH₂CH₂OBn |
| 671 | NO₂ | Me | (CH₂)₆SO₂CH₂CH₂SH |
| 672 | NO₂ | Me | (CH₂)₆SO₂iPr |
| 673 | iPr | NO₂ | Me |

In Table 1, Compound No. 1, 21, 23, 28, 29, 43, 50, 54, 55, 56, 57, 58, 135, 136, 137, 138, 139, 140, 141, 142, 153, 154, 155, 156, 157, 158, 159, 160, 161, 162, 163, 164, 165, 166, 167, 168, 169, 170, 175, 176, 178, 179, 180, 181, 182, 183, 184, 185, 186, 187, 191, 192, 193, 194, 195, 196, 197, 198, 209, 210, 268, 290, 291, 292, 293, 294, 295, 296, 308, 309, 316, 324, 401, 402, 403, 404, 405, 406, 459, 460, 461, 462, 493, 562, 568 or 673 is preferred; Compound No. 28, 29, 43, 54, 135, 136, 137, 138, 139, 140, 141, 142, 157, 158, 159, 160, 161, 162, 163, 175, 177, 178, 179, 180, 181, 182, 183, 191, 196, 197, 198, 290, 291, 292, 293, 294, 295, 296, 402, 459 or 673 is more preferred;
43) 4-(2-nitro-1H-imidazol-1-yl)ethylmorpholine (general name: Nimorazole), 135) 1,2-dimethyl-5-nitro-1H-imidazole (general name: Dimetridazole), 157) 2-(2-methyl-5-nitroimidazol-1-yl)ethanol (general name: Metronidazole), 163) 1-(2-ethylsulfonylethyl)-2-methyl-5-nitroimidazole (general name: Tinidazole),
176) methyl (2-(2-methyl-5-nitroimidazol-1-yl)ethylthiocarbamate (general name: Carnidazole),
183) 1-(2-methyl-5-nitroimidazol-1-yl)propan-2-ol (general name: Secnidazole), 191) 1-chloro-3-methyl-5-nitroimidazol-1-yl)propan-2-ol (general name: Ornidazole),
402) 2-carbamoyloxymethyl-1-methyl-5-nitro-1H-imidazole (general name: Ronidazole),
459) α-methoxymethyl-2-nitroimidazole-1-ethanol (general name: Misonidazole) or
673) 1-methyl-2-(1-methylethyl)-5-nitroimidazole (general name: Ipronidazole) is further more preferred; and
157) 2-(2-methyl-5-nitroimidazol-1-yl)ethanol (general name: Metronidazole) or
163) 1-(2-ethylsulfonylethyl)-2-methyl-5-nitroimidazole (general name: Tinidazole) is most preferred.

### Detailed description of a preferred embodiment of the invention

The nitroimidazole derivative contained in the external preparation of the present invention is a known compound or can be also obtained by the following Process A to Process C.

### Process A

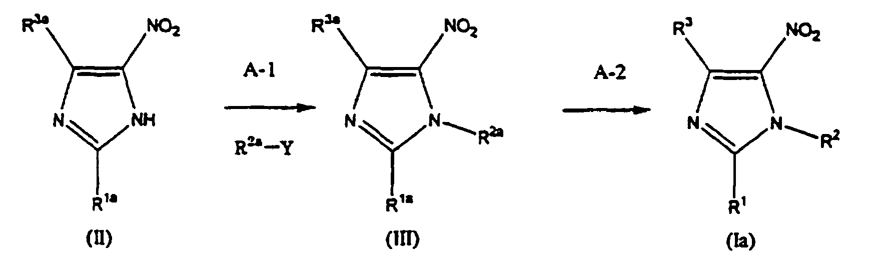

### Process B

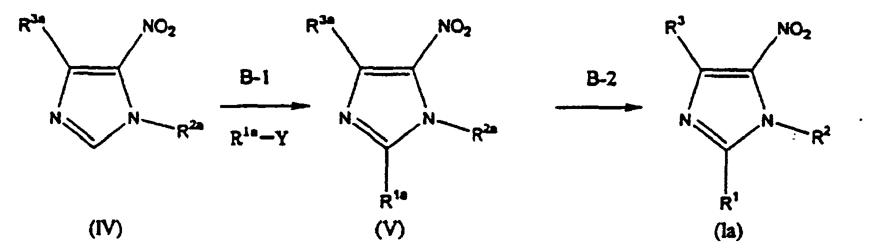

### Process C

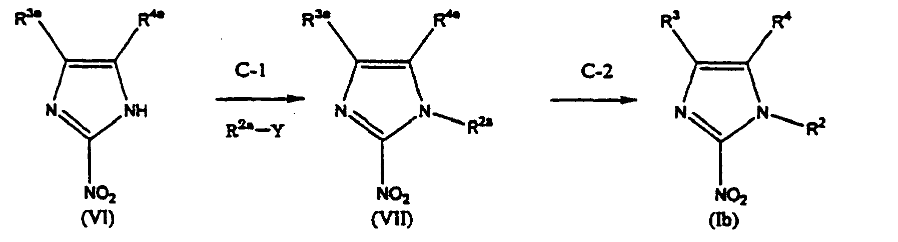

In the above Processes A to Process C, R¹ to R⁴ represent the same meanings as defined above and R³ and R⁴ are the same or different and represent a hydrogen atom or an optional organic group. R^{1a} represents the above R¹ or a group in which a functional group on R¹ is appropriately protected, if necessary, according to a well-known method (for example, "Protective Groups in Organic Synthesis", Greene, T.W.; Wuts, P.G.M. John Wiley & Sons; New York, 1999, etc.) and the functional group on R¹ is substituted, if necessary, to an appropriate functional group capable of obtaining a desired functional group by a well-known substitution reaction. R^{2a} represents the above R² or a group in which a functional group on R² is appropriately protected, if necessary, according to a well-known method and the functional group on R² is substituted, if necessary, to an appropriate functional group capable of obtaining a desired functional group by a well-known substitution reaction. R^{3a} represents the above R³ or a group in which a functional group on R³ is appropriately protected, if necessary, according to a well-known method and the functional group on R³ is substituted, if necessary, to an appropriate functional group capable of obtaining a desired functional group by a well-known substitution reaction. R^{4a} represents the above R⁴ or a group in which a functional group on R⁴ is appropriately protected, if necessary, according to a well-known method and the functional group on R⁴ is substituted, if necessary, to an appropriate functional group capable of obtaining a desired functional group by a well-known substitution reaction. Y represents a group to be eliminated.

In the following, respective steps of Process A to Process C are described in more detail.

### (Process A)

### (Step A-1)

The present step is to prepare Compound (III) by reacting Compound (II), which is well-known or can be easily obtained from the well-known compound with R^{2a}-Y in the presence or absence of a base catalyst in an inert solvent.

The solvent employable here may include, for example, water; aliphatic hydrocarbons such as hexane, heptane, ligroin, petroleum ether,etc.; aromatic hydrocarbons, such as benzene, toluene, xylene, etc.; halogenated hydrocarbons such as methylene chloride, chloroform, carbon tetrachloride, dichloroethane, chlorobenzene, dichlorobenzene, etc.; esters such as ethyl formate, ethyl acetate, propyl acetate, butyl acetate, diethyl carbonate, etc.; ethers, such as diethyl ether, diisopropyl ether, tetrahydrofuran, dioxane, dimethoxyethane ,diethylene glycol dimethyl ether, etc.; ketones, such as acetone, methyl ethyl ketone, methyl isobutyl ketone, isophorone, cyclohexanone, etc.; nitro compounds, such as nitroethane, nitrobenzene,etc.; nitriles, such as acetonitrile, isobutyronitrile, etc.; amides, such as formamide, N,N-dimethylformamide, N,N-dimethylacetamide, N-methyl-2-pyrrolidone, N-methylpyrrolidinone, hexamethylphosphoric triamide, etc; sulfoxides, such as sulfolan, etc.; and pyridines, preferably water and the pyridine.

The base catalyst employable here may include, for example, alkali metal hydroxides, such as sodium hydroxide, potassium hydroxide, etc.; alkali metal carbonates, such as sodium carbonate, potassium carbonate, etc; alkali metal alkoxides, such as sodium methoxide, sodium ethoxide,etc.; organic bases, such as triethylamine, pyridine, dimethylaminopyridine, etc.; and ammonia water, preferably the alkali metal hydroxides.

A reaction temperature varies depending on the raw material compound, the solvent, the reagent and the base catalyst, and is usually 0°C to 200°C, preferably 20°C to 130°C.

A reaction time varies depending on the raw material compound, the solvent, the reagent, the base catalyst and the reaction temperature, and is usually 10 minutes to 3 days, preferably 1 to 10 hours.

After completion of the reaction, the desired Compound (III) of the present reaction is obtained, for example, by neutralizing the reaction mixture, concentrating the reaction mixture, adding an organic solvent immiscible with water such as ethyl acetate, washing with water, separating an organic layer or an aqueous layer containing the desired compound and distilling off the solvent.

The compound, thus obtained, can be further purified, if necessary, by a conventional method, for example, recrystallization and silica gel column chromatography.

In the case where the compound, thus obtained, is the desired Compound (Ia) and deprotection and conversion of a functional group are not required, Compound (Ia) can be obtained without conducting Step A-2 described below.

### (Step A-2)

The present step is to prepare Compound (Ia) by carrying out a substitution reaction of Compound (III) which is well-known or is obtained in (A-1), if necessary, in an inert solvent, subsequently or concurrently carrying out the deprotection reaction if necessary.

The substitution reaction of the present step varies depending on the desired substituents and is not particularly limited so long as it is a reaction in which the desired functional group is obtained and is carried out according to a method described in references (for example, "Aliphatic Nucleophilic Substitution", Hartshorn, Cambridge University Press: Cambridge (1973), Chem. Soc. Rev., 19, 83 (1990), Carbocation Chem., 1, 121 (1989), etc.).

The deprotection reaction of the present step varies depending on the protective group and is not particularly limited so long as it is the reaction in which the desired functional group is obtained and is carried out according to a method described in references (for example, "Protective Groups in Organic Synthesis", Greene, T.W.; Wuts, P.G.M. John Wiley & Sons; New York, 1999, etc.).

### (Process B)

### (Step B-1)

The present step is to prepare Compound (V) by reacting Compound (IV) which is publicly known or can be easily obtained from the publicly known compound with R^{1a}-Y in the presence or absence of a base catalyst in an inert solvent.

The solvent employable here may include, for example, water; aliphatic hydrocarbons such as hexane, heptane, ligroin and petroleum ether; aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; ketones such as acetone, methyl ethyl ketone, methyl isobutyl ketone, isophorone and cyclohexanone; nitro compounds such as nitroethane and nitrobenzene; nitriles such as acetonitrile and isobutyronitrile; amides such as formamide, N,N-dimethylformamide, N,N-dimethylacetamide, N-methyl-2-pyrrolidone, N-methylpyrrolidinone and hexamethylphosphoric triamide; sulfoxides such as sulfolan; and pyridines, preferably water and the pyridine.

The base catalyst employable here may include, for example, alkali metal hydroxides such as sodium hydroxide and potassium hydroxide; alkali metal carbonates such as sodium carbonate and potassium carbonate; alkali metal alkoxides such as sodium methoxide and sodium ethoxide; organic bases such as triethylamine, pyridine and dimethylaminopyridine; and ammonia water, preferably the alkali metal hydroxides.

The reaction temperature varies depending on the material compound, the solvent, the reagent and the base catalyst and is usually 0°C to 200°C, preferably 20°C to 130°C.

The reaction time varies depending on the material compound, the solvent, the reagent, the base catalyst and the reaction temperature and is usually 10 minutes to 3 days, preferably 1 to 10 hours.

After completion of the reaction, the desired Compound (V) of the present reaction is obtained, for example, by neutralizing the reaction mixture, concentrating the reaction mixture, adding an organic solvent immiscible with water such as ethyl acetate, washing with water, separating an organic layer or an aqueous layer containing the desired compound and distilling off the solvent.

The compound thus obtained can be further purified, if necessary, by a conventional method, for example, recrystallization and silica gel column chromatography.

In the case where the compound thus obtained is the desired Compound (Ia) and deprotection and conversion of a functional group are not required, Compound (Ia) can be obtained without conducting Step B-2 described below.

### (Step B-2)

The present step is to prepare Compound (Ia) by carrying out the substitution reaction of Compound (V) which is publicly known or is obtained in (B-1), if necessary, in an inert solvent, subsequently or concurrently carrying out the deprotection reaction if necessary.

The present step is carried out in the same manner as in Step A-2.

### (Process C)

### (Step C-1)

The present step is to prepare Compound (VII) by reacting Compound (VI) which is publicly known or can be easily obtained from the publicly known compound with R^{2a}-Y in the presence or absence of a base catalyst in an inert solvent.

The present step is carried out in the same manner as in Step A-1.

The compound, thus obtained, can be further purified, if necessary, by a conventional method, for example, recrystallization and silica gel column chromatography.

In the case where the compound thus obtained is the desired Compound (Ib) and deprotection and conversion of a functional group are not required, Compound (Ib) can be obtained without conducting Step C-2 described below.

### (Step C-2)

The present step is to prepare Compound (Ib) by carrying out the substitution reaction of Compound (VII) which is publicly known or is obtained in (C-1), if necessary, in an inert solvent, subsequently or concurrently carrying out deprotection reaction if necessary.

The present step is carried out in the same manner as in Step A-2.

Moreover, the compound of the present invention can be obtained in accordance with known methods for example, a production method of metronidazole is disclosed by Jacob, et al. (US Patent No. 2,944,061) and a production method of tinidazole is disclosed by Butler, et al. (US Patent No. 3,376,311); and, for example, a production method of a derivative having a dihydropyridine ring is disclosed by Gorlitzer, et al. (Pharmazie (1999), 54(12), 889-892), a production method of a carbamate derivative is disclosed by Hay, et al. (Bioorg. Med. Chem. Lett. (1999), 9(15), 2237-2242), a production method of a derivative having an isoquinoline ring is disclosed by Parveen, et al. (Bioorg. Med. Chem. Lett. (1999), 9(15), 2031-2036), a production method of a derivative having a pyrrole ring is disclosed by Anadlu, et al. (Eur. J. Med. Chem. (1999), 34(3), 275-278), a production method of a derivative having a benzene ring substituted with a carboxyl group is disclosed by Everett, et al. (Bioorg. Med. Chem. Lett. (1999), 9(9), 1267-1272), a production method of a derivative having a halogen-substituted benzene ring and a derivative having a pyridine ring is disclosed by Shafiee, et al. (J. Heterocycl. Chem. (1998), 35(3), 607-610), a production method of a derivative having an arylcarbonyloxy group is disclosed by Bowden, et al. (Eur. J. Med. Chem. (1997), 32(12), 995-1000), a production method of a derivative having a dioxolane ring is disclosed by Baji, et al. (Eur. J. Chem. (1997), 32(7-8), 637-650), a production method of a derivative having a hydroxyaryl group is disclosed by Arrendondo, et al. (Bioorg. Med. Chem. Lett. (1996), 6(15), 1781-1784), a production method of a derivative having a phenylamide group is disclosed by Shafiee, et al. (J. Sci. Islamic Repub. Iran (1995), 6(1), 25-8), a production method of a derivative having an alkylthio group is disclosed by Rao, et al. (J. Chem. Soc. Perkin Trans. 1 (1994), (17), 2399-2402), a production method of a derivative having a hydroxyalkylaryl group is disclosed by Furlan, et al. (European Unexamined Patent Publication No. EP535528), a production method of a derivative having a β-lactam ring is disclosed by Bertola, et al. (European Unexamined Patent Publication No. EP490450), a production method of an aminoalkylphenylacyl derivative is disclosed by Bundgaargd, et al. (International Unexamined Patent Publication No. WO90/08128), a production method of a derivative having an aralkylcarbonyloxy group is disclosed by Rao, et al. (Indian J. Chem., Sect. B (1990), 29B(11), 1034-1040), a production method of an N-substituted aminomethylbenzoate derivative is disclosed by Jansen, et al. (Int. J. Pharm. (1990), 58(2), 143-153), a production method of a derivative having a pyridine ring is disclosed by Alcalde, et al. (Farmaco (1989), 44(11), 1095-107), a production method of metronidazole and secnidazole accompanying a nitro group transfer reaction is disclosed by Buforn, et al. (US Patent No. 492,591), a production method of dimetridazole using dimethyl sulfate is disclosed by Buforn, et al. (US Patent No. 4,925,952), a production method of metronidazole.secnidazole using alkylene sulfate is disclosed by Bonnamas, et al. (US Patent No. 4,925,949), a production method of a derivative having an alkylcarbonyloxy group is disclosed by Johansen, et al. (Int. J. Pharm. (1986), 32(2-3), 199-206), a production method of a derivative having a dextran ring is disclosed by Vermeersch, et al. (Bull. Soc. Chim. Belg. (1985), 94(8), 591-596), a production process of an L-cysteine derivative is disclosed by Reiner (European Unexamined Patent Publication No. 140395), a production method of an ester derivative of an amino acid residue is disclosed by Cho (European Unexamined Patent Publication No. 127274), a production method of a derivative having a hydroxyalkylaryl group is disclosed by Tessitore (European Unexamined Patent Publication No. 11657), a production method of a derivative having a hydroxyalkylaryl group is disclosed by Scalesciani (European Unexamined Patent Publication No. EP103100), a production method of an ester derivative of an amino acid residue is disclosed by Bundgaard, et al. (Int. J. Pharm. (1984), 18(1-2), 67-77), a production method of an ester derivative of a dimethylglycine residue is disclosed by Thorbek, et al. (European Unexamined Patent Publication No. 96870), a production method of a 2-nitroimidazole derivative having an aryloxy ring is disclosed by Hofheinz (European Unexamined Patent Publication No. EP81719), a production method of a platinum salt derivative is disclosed by Bales, et al. (J. Chem. Soc., Chem. Comm. (1983), (8), 432-433), a production method of a retinoic acid derivative is disclosed by Whitefield, et al. (UK Unexamined Patent Publication No. 2097783), a production method of a derivative having a hydroxyalkylaryl group is disclosed by Bononi (US Patent No. 4,463,012), a production method of a derivative having an alkylsulfonylphenyloxy group and a production method of a derivative having alkylthiophenyloxy group are disclosed by Winkelmann, et al. (UK Unexamined Patent Publication No.s 1541280 and 1590974), a production method of a derivative having an acetamide-substituted phenyloxy group is disclosed by Winkelmann, et al. (Arzneim. -Forsch. (1978), 28(5), 739-49), a production method of a derivative having a phenylcarbamate group and a production method of a derivative having a hydrazinecarboxyl group are disclosed by Cavalleri, et al. (J. Med. Chem. (1978), 21(8), 781-4), a production method of a derivative having a hydrazine group is disclosed by Winkelmann, et al. (Arzneim. -Forsch. (1977), 27(12), 2251-63), a production method of a 2-nitroimidazole derivative having a phenylcarbamate group is disclosed by Cavalleri, et al. (US Patent No. 4,113,953), a production method of a 2-nitroimidazole derivative having an aluren group is disclosed by Cavalleri, et al. (J. Med. Chem. (1977), 20(11), 1522-5), a production method of a derivative having a substituted phenoxy group is disclosed by Winkelmann, et al. (US Patent No. 4,031,232), a production method of a 2-nitroimidazole derivative having a hydroxy group is disclosed by Assandri, et al. (UK Unexamined Patent Publication No. 1480192), a production method of a derivative having an alkylcarbonyloxy group is disclosed by Hoffer (UK Unexamined Patent Publication No. 1453417), a production method of a derivative having a quinoline ring is disclosed by Kreider, et al. (US Patent No.s 3,910,925 and 3,828,056), a production method of dimetridazole using formic acid as a solvent is disclosed by Frank, et al. (UK Unexamined Patent Publication No. 1493496), a production method of metronidazole using alkylene oxide is disclosed by Frank, et al. (UK Unexamined Patent Publication No. 1481349), a production method of a derivative having a phenylamide group is disclosed by Heeres, et al. (US Patent No. 3,928,374), a production method of ornidazole using epichlorohydrin is disclosed by Hoffer, et al. (J. Med. Chem. (1974), 17(9), 1019-20), a production method of 1-methyl-5-isopropyl-2-nitroimidazole is disclosed by Martin, et al. (US Patent No. 3,828,064), a production method of a derivative having a carboxyimidamide group is disclosed by Rufer, et al. (US Patent No. 3,966,732), a production methods of a derivative having a carbamate group and a 2-nitromidazole derivative are disclosed by Challier, et al. (UK Unexamined Patent Publication No. 1352288), a production method of metronidazole using ethylene oxide and sulfuric acid is disclosed by Muhlbrod (UK Unexamined Patent Publication No. 1301225), a production method of a 2-nitromidazole derivative having a phenylhydrazone group or alkenyl group is disclosed by Cavalleri, et al. (J. Heterocycl. Chem. (1972), 9(5), 979-84), a production method of a derivative having a carboxyimidyl group is disclosed by Papaioannou (US Patent No. 3,694,452), a production method of a derivative having an alkenyl group is disclosed by Hoffer, et al. (US Patent No. 3,652,579), a production method of a 2-benzyl derivative is disclosed by Hoff, et al. (UK Unexamined Patent Publication No. 1336228), a production method of secnidazole by nitration using nitric acid and its following hydrolysis is disclosed by Jeanmart, et al. (UK Unexamined Patent Publication No. 1278757), a production method of secnidazole and 1-(2-methyl-5-nitroimidazol-1-yl)2-propanone is disclosed by Jeanmart, et al. (UK Unexamined Patent Publication No. 1278758), a production method of 1,5-dimethyl-2-nitroimidazole and 1,4,5-trimethyl-2-nitroimidazole is disclosed by Lancini, et al. (UK Unexamined Patent Publication No. 1114154), a production method of a derivative having a nitroso group is disclosed by Kulsa, et al. (US Patent No. 3,711,495), a production method of a derivative having a hydroxy group is disclosed by Chemerda, et al. (US Patent No. 3,584,007), a production method of a 2-nitroimidazole derivative substituted with an alkyl group is disclosed by Lancini, et al. (South African Unexamined Patent Publication No. 6905670), a production method of a derivative having a sulfonyl group (including tinidazole) is disclosed by Miller, et al. (J. Med. Chem. (1970), 13(5), 849-52), a production method of a 2-nitroimidazole derivative having a hydroxy group is disclosed by Lancini, et al. (UK Unexamined Patent Publication No. 1229170), a production method of a derivative having an alkyl halide group is disclosed by Kajfez, et al. (Farm. Glas. (1969), 25(2), 49-54), and a production method of 4-iodo-1,2-dimethyl-5-nitroimidazole is disclosed by Hoffer, et al. (J. Heterocycle Chem. (1966), 3(4), 454-8)).

Examples of administration forms of the external preparation for skin diseases of the present invention include ointment, cream, lotion, moisturized patch or moisture-free patch, shampoo, gel, rinse, face lotion, milky lotion, paste, shaving cream, foundation, cologne, pack, semi-solid, solid or liquid. These preparations can be produced in accordance with routine methods, for example, as described below using, as necessary, additives such as antioxidants (including carboxylic acids such as ascorbic acid and citric acid; and phenols such as tocopherol and dibutylhydroxytoluene), antiseptics (including carboxylic acids such as dehydroacetic acid, salicylic acid and disodium edetate; and phenols such as ethyl paraoxybenzoate, methyl paraoxybenzoate, isopropyl paraoxybenzoate and thymol), wetting agents (including glycols such as glycerin, propylene glycol, dipropylene glycol and 1,3-butylene glycol; organic salts such as hyaluronic acid; and amides such as urea), consistency agents (including polymer compounds such as polyethylene glycol; and celluloses such as carboxymethyl cellulose sodium and carboxypropyl cellulose), buffers (including organic acids such as citric acid, lactic acid and tartaric acid; inorganic acids such as hydrochloric acid and boric acid; salts such as sodium dihydrogen phosphate and sodium citrate; organic bases such as triethanolamine; and inorganic bases such as sodium hydroxide and potassium hydroxide), adsorbents (including water-containing aluminum silicates such as kaolin and bentonite; and inorganic bases such as magnesium hydroxide-aluminum hydroxide co-precipitate and aluminum hydroxide), bases (including organic substances such as white petrolatum, Tween 60, Tween 80, liquid paraffin, beeswax, petrolatum, castor oil, silicone oil, hydrogenated castor oil, natural rubber, coconut oil fatty acid diethanolamide, polyoxyethylene hydrogenated castor oil, natural rubber latex and 1,3-pentadiene copolymer resin; polymer compounds such as polybutene, synthetic rubber SBR, polyethylene glycol monostearate, polyoxyethylene glycol monostearate, polyoxyethylene cetostearyl ether, polyoxyethylene oleylcetyl ether, silicon, starch grafted acrylate 300, sodium polyacrylate, methacrylic acid-n-butyl acrylate copolymer and carboxyvinyl polymer; fatty acids such as stearic acid; alcohols such as cetanol and myristyl alcohol, and fatty acid esters such as octadodecyl myristate, isopropyl myristate and cetyl octanoate), solvents (including carbohydrates such as ethanol, isopropanol, 1,3-butylene glycol, n-octadecylalcohol, crotamiton and caprylic/capric acid triglyceride), stabilizers (including inorganic salts such as sodium metaphosphate, zinc oxide and titanium oxide; and organic salts such as sodium polyoxyethylene lauryl sulfate ether sulfate and sodium lauryl sulfate), adhesives (including polymer compounds such as sodium polyacrylate and dipropylene glycol), emulsifiers (including carbohydrates such as sorbitan monooleate, polyoxyethylene sorbitan monooleate, D-sorbitol, polyglycerin monolaurate and sodium polyoxyethylene lauryl ether sulfate), surfactants (including polymer compounds such as polyglycerin monolaurate and polyoxyethylene oleyl alcohol ether), squalane, diluent, Span 60, Span 80, gelatin, propylparaben, methylparaben, lauryldimethyl-aminoacetate betaine, coconut oil fatty acid diethanol amide, N-[alkyl(12,14)oxy-2-hydroxypropyl]-L-arginine hydrochloride, silicone oil, jojoba oil, and fragrance.

Any fragrances can be used provided they can be generally used in foods, cosmetics, pharmaceuticals and so forth. Examples of naturally-derived fragrances include those obtained from plants such as rose, lavender and orange, and those obtained from animals such as musk oil (musk) obtained from musk deer and castorium (castor oil) obtained from beavers. Examples of synthetic fragrances include limonene, β-caryophyllene, farnesol, citral, γ-undecalactone, indole and rilal.

Ointments are produced by, for example, heating and stirring an active ingredient and base, heating and dispersing, followed by cooling to the room temperature while stirring.

Creams are produced by, for example, first producing a base while heating and stirring, adding an active ingredient itself or a solution containing the active ingredient while heating and stirring, and cooling the resulting emulsion to the room temperature.

Lotions are produced by, for example, adding the active ingredient itself or a solution containing the active ingredient to an oily base or mixed base consisting of an oily base melted by heating and an aqueous base while stirring and heating, and then adding an aqueous base and cooling the resulting liquid to the room temperature.

Moisturized patches are produced by, for example, adding an additive to a mixed base consisting of an oily base melted by heating and an aqueous base while stirring, adding an active ingredient or a solution containing the active ingredient to the mixture while heating with stirring, rolling out the resulting paste onto a non-woven fabric and cutting to an appropriate size.

Moisture-free patches are produced by, for example, adding an active ingredient or a solution containing the active ingredient to a mixed base consisting of an oily base melted by heating while heating and stirring, adding this to a mixture of synthetic resin that has been melted by heating while stirring, rolling out the resulting paste onto a non-woven or woven fabric and cutting to an appropriate size.

Gels are produced by, for example, uniformly dissolving a gel base followed by adding a hydrophilic organic solvent, adding an active ingredient, heating, dissolving and dispersing. A solvent is then added thereto while heating. Next, after neutralizing while stirring, the mixture is cooled to the room temperature.

Shampoos are produced by, for example, heating purified water, adding an active ingredient, anionic surfactant, humectant and so forth, and cationic polymer as necessary, followed by uniformly dissolving and then cooling.

Pastes are produced by, for example, adding fats and oils to a wax, heating to melt, adding pigment, hydrocarbon and effective ingredient and so forth, and humectant as necessary, followed by mixing uniformly and cooling.

Rinses are produced by, for example, adding aqueous ingredients such as effective ingredient, humectant and cationic surfactant to purified water followed by melting with heating. Oily components such as higher alcohols and hydrocarbons are then added thereto after melting with heating followed by stirring to obtain a uniform mixture and then cooling.

Liquids are produced by, for example, adding and mixing an effective ingredient, humectant, lower alcohol and so forth to purified water, and then adding water-soluble polymer as necessary. Liquids can also be produced by adding these to mixture of oily components such as fatty acids, fats and oils and fatty acid esters as necessary followed by melting with heating.

Soap is produced by, for example, adding alkali to heated fats and oils. Alternatively, soap is produced by adding and stirring an added lower alcohol in fats and oils followed by the addition of alkali, purified water and humectant. Polysaccharides may also be added to this and mixed thoroughly followed by the addition of dye, fragrance and an effective ingredient followed by mixing uniformly, cooling and drying to obtain soap.

Milky lotions can be produced by, for example, adding an effective ingredient and humectant, etc. to purified water followed by heating to melt, adding this to oily components such as surfactant and higher alcohol that have been melted by heating, and then mixing uniformly and cooling.

Shaving creams can be produced by, for example, adding an active ingredient, humectant, alkali and so forth to purified water followed by heating and melting. This is then added to a mixture of necessary ingredients such as fatty acid, fatty acid esters, fats and oils and so forth that have been melted by heating followed by mixing uniformly and cooling.

Face lotions are produced by, for example, adding an active ingredient, thickener, humectant and so forth to purified water followed by the addition of a mixture of alcohol, surfactant and oily components such as fats and oils and mixing uniformly.

Foundations are produced by, for example, mixing pigments and coloring pigments of finely ground clay minerals, adding fatty acid, higher alcohols and other fats and oils and esters and mixing uniformly.

Colognes are produced by, for example, adding and mixing an effective ingredient, humectant, lower alcohol and so forth into purified water, adding water-soluble polymer as necessary and then adding fragrance after cooling. If necessary, colognes can also be produced by adding these to a mixture of oily ingredients such as fatty acids, fats and oils and fatty acid esters, etc. after melting by heating, and then adding fragrance after cooling.

The raw materials used in packs are completely different depending on the preparation form. If the pack is in the form of a jelly, it is produced by, for example, heating and melting an effective ingredient, humectant, alkali and so forth in purified water, adding thickener, water-soluble polymer and so forth, followed by stirring. Next, alcohols, surfactant and so forth are added and dissolved followed by cooling.

Further, in the production of the external preparation for atopic dermatitis of the present invention, other pharmaceutically effective ingredients may be contained therein in addition to those agents provided that they do not impair the effect of combining those agents which is dermatologically applicable. Examples of these pharmaceutically active ingredients include known refrigerants, keratolytics, cortical inhibitors, antiseborrheics, germicides, antipruritics as well as drugs that can be used for skin diseases, specific examples of which include menthol, salicylic acid, estradiol, glycyrrhizic acid, benzalkonium chloride, phenol and camphor; narcotics and antihypnotics such as ethylmorphine hydrochloride, oxycodone hydrochloride, cocaine hydrochloride, pethidine hydrochloride, methamphetamine hydrochloride, dl-methylephedrine hydrochloride, morphine hydrochloride, fentanyl citrate, levallorphan tartrate; local germicides such as povidone iodide and iodoform; enzyme preparations such as lysozyme hydrochloride, streptokinase, streptodornase trypsin and deoxyribonuclease; herbal medicines such as Lithospermi Radex extract and scopolia extract; hemorrhoid preparations such as non-viable E. coli, epidihydrocholesterin and tribenoside; and hemostyptics such as thrombin, cellulose oxide and sodium alginate.

The following provides a more detailed explanation of the present invention through its Examples and Test Examples.

### [Example]

### (Example 1) Ointment for external use

### Prescription:

The ointment is composed of metronidazole (2 g), Tween 80 (1 g), propylene glycol (28 g)and white petrolatum (69 g).

### Preparation method:

A mixture of Tween 80, propylene glycol and metronidazole was added to a white petrolatum, while heating and stirring. The mixture was dispersed with continuously stirring while heating. The dispersion was then allowed to cool slowly to about 25°C and charged in a suitable vessel.

### (Example 2) Ointment for external use

### Prescription:

The ointment is comprised of metronidazole (2 g), propylene glycol (5 g), polyoxyethylene glycol monostearate (4 g), liquid paraffin (10 g), white petrolatum (60 g) and distilled water (an amount making total 100 g).

### Preparation method:

Distilled water, propylene glycol and metronidazole were dispersed under stirring and heating and a temperature of the dispersion was adjusted to about 70°C. Polyoxyethylene glycol monostearate, liquid paraffin and white petrolatum were melted and adjusted to about 70°C and the molten mixture was slowly added to the dispersion. The mixture was allowed to cool slowly to about 25°C while continuously stirring, and charged in a suitable vessel.

### (Example 3) Cream for external use

### Prescription:

The cream is comprised of metronidazole (2 g), stearic acid (5 g), polyoxyethylene cetostearyl ether (12E.O.)(0.5 g), polyoxyethylene cetostearyl ether (20E.O.) (0.5 g), cetanol (5 g), cetyl octanoate (5 g), liquid paraffin (5 g), beeswax (1 g), glycerin (5 g), 1,3-butylene glycol (5 g), triethanolamine (5 g), hydrochloric acid (2.7 g) and distilled water (an amount making total 100 g).

### Preparation method:

Polyoxyethylene cetostearyl ether (20E.O.), polyoxyethylene cetostearyl ether (12E.O.), stearic acid, cetanol, cetyl octanoate, liquid paraffin and beeswax were melted at a temperature of about 70 to 75°C. Distilled water, glycerin, 1,3-butylene glycol and triethanolamine were dissolved and kept at a temperature of about 70°C, and slowly added thereto while stirring. Subsequently, distilled water, metronidazole and hydrochloric acid were dissolved and the solution was heated to about 70°C and slowly added thereto. The emulsion thus obtained was cooled to a temperature of about 25°C while continuously stirring. The cream thus obtained was charged in a suitable vessel.

### (Example 4) Cream for external use

### Prescription:

The cream is composed of metronidazole (1.8 g), stearic acid (2 g), glycol monostearate (12 g), polyoxyethylene glycol monostearate (3 g), polyoxyethylene cetostearyl ether (12E.O.) (1 g), polyoxyethylene cetostearyl ether (20E.O.) (1 g), cetanol (2 g), liquid paraffin (5 g), cetyl octanoate (5 g), ethyl paraoxybenzoate (0.3 g), silicone oil (1 g), beeswax (1.5 g), 1,3-butylene glycol (7 g), glycerin (5 g), sodium hydroxide (suitable amount), hydrochloric acid (suitable amount) and distilled water (an amount making total 100 g).

### Preparation method:

To a dissolved solution of distilled water, 1,3-butylene glycol and glycerin, metronidazole was added and the hydrochloric acid was added until metronidazole was dissolved completely. The solution was heated to about 70°C and pH was made to 6.9 by the addition of sodium hydroxide. As oil phase, stearic acid, glycol monostearate, polyoxyethylene glycol monostearate, polyoxyethylene cetostearyl ether (12E.O.), polyoxyethylene cetostearyl ether (20E.O.), cetanol, liquid paraffin, cetyl octanoate, silicone, ethyl paraoxybenzoate and beeswax were melted and adjusted to temperature of about 70 to 75°C. To this oil phase mixture, the foregoing solution was slowly added while stirring. The emulsion thus obtained was cooled to a temperature of about 25°C while continuously stirring. The cream thus obtained was charged in a suitable vessel.

### (Example 5) Cream for external use

### Prescription:

The cream is composed of metronidazole (1.8 g), n-octadecyl alcohol (5 g), stearic acid (5 g), triethanolamine (5 g), liquid paraffin (10 g), disodium edetate (0.25 g), glycerin (10 g), thymol (0.25 g), hydrochloric acid (suitable amount)and distilled water (an amount making total 100 g).

### Preparation method:

A mixture of n-octadecyl alcohol, stearic acid and liquid paraffin was melted by heating while stirring and kept at about 70°C, and metronidazole was then added thereto. While keeping a temperature at about 70°C and stirring, a dissolved mixture of distilled water, glycerin and triethanolamine was slowly added thereto. Subsequently, disodium edetate and thymol were added thereto. The emulsion thus obtained was adjusted to pH 6.8 by adding hydrochloric acid, followed by cooling to a temperature of about 25°C while continuously stirring and charged in a suitable vessel.

### (Example 6) Lotion for external use

### Prescription:

The lotion is composed of metronidazole (2 g), stearic acid (4 g), cetanol (1 g), polyoxyethylene cetostearyl ether (20E.O.)(1 g), triethanolamine (0.2 g), glycerin (5 g), isopropanol (10 g), and distilled water (an amount making total 100 g).

### Preparation method:

Cetanol, polyoxyethylene cetostearyl ether (20E.O.), stearic acid and metronidazole were melted by heating while stirring. A molten mixture of triethanolamine, distilled water and glycerin was added thereto. The mixture was then cooled to a temperature of 40°C and isopropanol was added thereto. The mixture was cooled rapidly to a temperature of about 25°C while continuously stirring. After cooling, the mixture was charged in a suitable vessel.

### (Example 7) Lotion for external use

### Prescription:

The lotion is composed of metronidazole (1.8 g), n-octadecyl alcohol (1 g), cetanol (1 g), polyoxyethylene cetostearyl ether (12E.O.)(1 g), 1,3-butylene glycol (10 g), Tween 80 (1 g), sodium carboxymethyl cellulose (1 g), isopropanol (10 g), distilled water (an amount making total 100 g).

### Preparation method:

A temperature of a dissolved-mixture comprising 1,3-butylene glycol and distilled water was adjusted a temperature to about 70°C while continuously stirring. N-octadecyl alcohol, cetanol and polyoxyethylene cetostearyl ether (12E.O.) were melted by heating and adjusted to about 70°C, then slowly added to the above mixture. Then, under stirring, a material obtained by mixing metronidazole, sodium carboxymethyl cellulose and Tween 80 under heating was added to the above mixture. After the resulting mixture was cooled to a temperature of about 40°C, isopropanol was slowly added thereto. The mixture was cooled to a temperature of about 25°C while stirring and charged in a suitable vessel.

### (Example 8) Moisture patch

### Prescription:

The patch is composed of metronidazole (2 g), kaolin (5 g), liquid paraffin (10 g), glycerin (15 g), sodium carboxymethyl cellulose (5 g), crotamiton (1.5 g), zinc oxide (2 g), Tween 80 (1 g), gelatin (5 g), sodium polyacrylate (5 g), distilled water (an amount making total 100 g).

### Preparation method:

Distilled water, sodium carboxymethyl cellulose and gelatin were melted by heating and added to a mixture in which zinc oxide, sodium polyacrylate and liquid paraffin were dispersed by stirring. Kaolin was added thereto while stirring. Subsequently, metronidazole, crotamiton, glycerin and Tween 80 were mixed under stirring and heating, adjusted a temperature to about 60°C and added to the above mixture under stirring and heating. Ointment thus obtained was applied with 1000 g/m² to an unwoven fabric and the fabric was cut into a size of 10 cm x 14 cm (280 mg of metronidazole was contained per 14 g of ointment).

### (Example 9) Moisture patch

### Prescription:

The patch is composed of metronidazole (2 g), sorbitan monooleate (0.5 g), polyoxyethylene sorbitan monooleate (0.5 g), castor oil (1 g), crotamiton (1 g), gelatin (1 g), kaolin (12 g), sodium metaphosphate (0.15 g), 1,3-butylene glycol (5 g), starch grafted acrylate 300 (1 g), sodium polyacrylate (5 g), a methacrylic acid-n-butyl acrylate copolymer (3 g), D-sorbitol solution (70%) (50 g), tartaric acid (1.5 g), titanium oxide (1 g), magnesium hydroxide-aluminum hydroxide co-precipitate (0.25 g), dibutylhydroxytoluene (0.2 g) and distilled water (an amount making total 100 g).

### Preparation method:

Suitable amounts of distilled water and a D-sorbitol solution were mixed and dissolved. While continuously stirring, titanium oxide was added, and then, suitable amounts of kaolin and a D-sorbitol solution were added thereto. To the mixture was added a sodium metaphosphate solution dissolved in distilled water, then a gelatin solution dissolved in distilled water was added, and a methacrylic acid-n-butyl acrylate copolymer was further added. To the above mixture were added a dissolved mixture of sodium polyacrylate, starch grafted acrylate 300, magnesium hydroxide-aluminum hydroxide co-precipitate, 1,3-butylene glycol and castor oil, sorbitan monooleate and polyoxyethylene sorbitan monooleate, and a heated mixture of metronidazole, crotamiton and dibutylhydroxytoluene was added thereto. A finally remaining mixture of D-sorbitol solution and tartaric acid was adjusted to a temperature of 60°C and added thereto while stirring. Ointment thus obtained was applied with 1000 g/m² to an unwoven fabric and the fabric was cut into a size of 10 cm x 14 cm (280 mg of metronidazole was contained per 14 g of ointment).

### (Example 10) Dry patch (plaster)

### Prescription:

The patch is composed of metronidazole (2 g), liquid paraffin (8 g), dibutylhydroxytoluene(0.2 g), crotamiton(1 g), polyoxyethylene glycol monostearate (2 g), polyoxyethylene cetostearyl ether (20E.O.) (1.8 g), methacrylic acid-n acrylate copolymer (5 g), myristyl alcohol (8 g), natural rubber (20 g), synthetic rubber SBR (37 g), and polybutene (15 g).

### Preparation method:

Metronidazole, dibutylhydroxytoluene and crotamiton were mixed under stirring and heating. Subsequently, polyoxyethylene glycol monostearate, polyoxyethylene cetostearyl ether (20E.O.) and myristyl alcohol were added to the above mixture and the resulting mixture was mixed under heating. The resulting mixture was continuously added to a molten state mixture of a natural rubber latex, a methacrylic acid-n-butyl acrylate copolymer and a synthetic rubber SBR while stirring. Liquid paraffin and polybutene were continuously added thereto while stirring. Ointment thus obtained was applied with 100 g/m² to an unwoven fabric or woven fabric. After drying, the fabric was cut into a size of 10 cm x 14 cm (28 mg of metronidazole was contained per 1.4 g of ointment).

### (Example 11) Ointment for external use

### Prescription:

The ointment is composed of tinidazole (2 g), propylene glycol (28 g), cetyl octanoate (5 g), and white petrolatum (65 g).

### Preparation method:

Propylene glycol was added to white petrolatum under heating and stirring. A mixed material of tinidazole and cetyl octanoate was added to the above mixture and the resulting mixture was heated while continuously stirring to disperse the material therein. Then, the dispersion was allowed to cool slowly to about 25°C and charged in a suitable vessel to obtain ointment for external use.

### (Example 12) Ointment for external use

### Prescription:

The ointment is composed of tinidazole (2 g), propylene glycol (10 g), polyoxyethylene glycol monostearate (5 g), liquid paraffin (20 g), white petrolatum (60 g), and distilled water (an amount making total 100 g).

### Preparation method:

Distilled water and propylene glycol were mixed and a temperature of the mixture was adjusted to 70°C, followed by stirring. To the above mixture were added polyoxyethylene glycol monostearate, tinidazole, liquid paraffin and white petrolatum mixed at a temperature of 70°C. The mixture was allowed to cool slowly to a temperature of about 25°C while continuously stirring, and then, charged in a suitable vessel to obtain ointment for external use.

### (Example 13) Cream for external use

### Prescription:

The cream is composed of tinidazole (1.8 g), stearic acid (5 g), polyoxyethylene cetostearyl ether (12E.O.)(0.5 g), polyoxyethylene cetostearyl ether (20E.O.)(0.5 g), cetanol (5 g), cetyl octanoate (5 g), liquid paraffin (5 g), beeswax (1 g), glycerin (5 g), 1,3-butylene glycol (5 g), triethanolamine (5 g), hydrochloric acid (2.7 g), distilled water (an amount making total 100 g).

### Preparation method:

As oil phase, stearic acid, polyoxyethylene cetostearyl ether (12E.O.), polyoxyethylene cetostearyl ether (20E.O.), cetanol, cetyl octanoate, liquid paraffin, and beeswax were melted and stirred at a temperature of about 70 to 75°C. To the above mixture were slowly added, while keeping a temperature at about 70°C, a solution of distilled water, glycerin, 1,3-butylene glycol and triethanolamine dissolved therein. Then, a dissolved solution of distilled water, tinidazole and hydrochloric acid was heated to about 70°C and slowly added to the above mixture. The formed emulsified liquid was continuously stirred and after cooling it at a temperature of about 25°C, then, charged in a suitable vessel to obtain cream.

### (Example 14) Cream for external use

### Prescription:

The cream is composed of tinidazole (1.8 g), stearic acid (3 g), glycol monostearate (4 g), polyoxyethylene glycol monostearate (1 g), polyoxyethylene cetostearyl ether (12E.O.)(0.5 g), polyoxyethylene cetostearyl ether (20E.O.)(0.5 g), cetanol (5 g), liquid paraffin (10 g), cetyl octanoate (5 g), ethyl paraoxybenzoate (0.3 g), silicone oil(1 g), beeswax (1.5 g), 1,3-butylene glycol (7 g), glycerin (5 g), sodium hydroxide (suitable amount), hydrochloric acid (suitable amount), and distilled water (an amount making total 100 g).

### Preparation method:

To a dissolved solution of distilled water, 1,3-butylene glycol and glycerin, tinidazole was added and further hydrochloric acid was added thereto until tinidazole was completely dissolved. This liquid was heated to a temperature of about 70°C and a pH thereof was made 6.9 with sodium hydroxide. This mixture was slowly added while stirring to a molten liquid in which stearic acid, glycol monostearate, polyoxyethylene glycol monostearate, polyoxyethylene cetostearyl ether (12E.O.), polyoxyethylene cetostearyl ether (20E.O.), cetanol, liquid paraffin, cetyl octanoate, ethyl paraoxybenzoate, silicone oil and beeswax were mixed and adjusted to a temperature of about 70 to 75°C. The formed emulsion was cooled to a temperature of about 25°C while continuously stirring, and then, charged in a suitable vessel to obtain cream.

### (Example 15) Cream for external use

### Prescription:

The cream is composed of tinidazole (2 g), n-octadecyl alcohol (5. g), stearic acid (5 g), triethanolamine (5 g), liquid paraffin (8 g), disodium edetate (0.2 g), glycerin (10 g), thymol (0.2 g), hydrochloric acid (suitable amount), distilled water (an amount making total 100 g).

### Preparation method:

A mixture of n-octadecyl alcohol, stearic acid and liquid paraffin was melted by heating while stirring and a temperature was adjusted to about 70°C, and tinidazole was then added thereto. To the resulting mixture was slowly added a dissolved material of distilled water, glycerin and triethanolamine adjusted a temperature to about 70°C while stirring. After adjusting a pH thereof to 6.8 by the addition of hydrochloric acid, disodium edetate and thymol were added thereto while continuously stirring. The mixture was cooled to a temperature of about 25°C and then charged in a suitable vessel to obtain cream for external use.

### (Example 16) Lotion for external use

### Prescription:

The lotion is composed of tinidazole (2 g), stearic acid (3 g), cetanol (1 g), polyoxyethylene cetostearyl ether (20E.O.)(0.5 g), triethanolamine (0.2 g), glycerin (5 g), isopropanol (10 g), and distilled water (an amount making total 100 g).

### Preparation method:

Cetanol, polyoxyethylene cetostearyl ether (20E.O.), stearic acid and tinidazole were melted by heating while stirring, and a molten mixture of triethanolamine, distilled water and glycerin was further added thereto. Next, the resulting mixture was cooled to a temperature of 40°C, isopropanol was added thereto and the mixture was rapidly cooled to a temperature of about 25°C while continuously stirring. After cooling, the mixture was charged in a suitable vessel to obtain lotion for external use.

### (Example 17) Lotion for external use

### Prescription:

The lotion is composed of tinidazole (1.8 g), isopropanol (10 g), n-octadecyl alcohol (10 g), cetanol (5 g), Tween 80 (2 g), 1,3-butylene glycol (10 g), sodium carboxymethyl cellulose (3 g), and distilled water (an amount making total 100 g).

### Preparation method:

n-Octadecyl alcohol and cetanol were melted by heating and slowly added to a heated mixture of distilled water, sodium carboxymethyl cellulose, Tween 80, 1,3-butylene glycol and tinidazole. Then, the resulting mixture was cooled to a temperature of about 40°C and isopropanol was added thereto, and the mixture was cooled to about 25°C while continuously stirring. The resulting material was charged in a suitable vessel to obtain lotion for external use.

### (Example 18) Moisture patch

### Prescription:

The patch is composed of tinidazole (2 g), kaolin (5 g), liquid paraffin (10 g), glycerin (15 g), sodium carboxymethyl cellulose (5 g), crotamiton (1.5 g), zinc oxide (2 g), Tween 80 (2 g), gelatin (5 g), sodium polyacrylate (5 g), and distilled water (an amount making total 100 g).

### Preparation method:

To distilled water were added a material in which sodium carboxymethyl cellulose and gelatin were melted by heating and added, and then, kaolin was added to disperse therein. This material was added under stirring to a material in which zinc oxide, sodium polyacrylate and liquid paraffin had been dispersed by stirring. Further, to the above mixture was added a material in which tinidazole, crotamiton, glycerin and Tween 80 had been stirred and heated, and a temperature of which had been adjusted to about 60°C under stirring and heating. Ointment thus obtained was applied with 1000g/m² to an unwoven fabric and the fabric was cut into a size of 10 cm x 14 cm (280 mg of tinidazole was contained per 14 g of ointment) to obtain a patch.

### (Example 19) Moisture patch

### Prescription:

The patch is composed of tinidazole (2 g), sorbitan monooleate (0.5 g), polyoxyethylene sorbitan monooleate (0.5 g), castor oil (1 g), crotamiton (1 g), gelatin (1 g), kaolin (12 g), sodium metaphosphate (0.15 g), 1,3-butylene glycol (5 g), starch grafted acrylate 300 (2 g), sodium polyacrylate (5 g), methacrylic acid-n-butyl acrylate copolymer (4 g), D-sorbitol solution (70%)(50 g), tartaric acid (1.7 g), titanium oxide (1 g), magnesium hydroxide-aluminum hydroxide co-precipitate (0.25 g), dibutylhydroxytoluene (0.2 g), and distilled water (an amount making total 100 g).

### Preparation method:

Suitable amounts of distilled water and D-sorbitol solution were mixed and dissolved. To this mixture was added titanium oxide, and then, suitable amounts of kaolin and a D-sorbitol solution were added to the mixture under stirring. To this mixture was added gelatin, and then, a methacrylic acid-n-butyl acrylate copolymer. To this mixture was further added a material obtained by mixing while stirring a mixed material in which sodium polyacrylate, starch grafted acrylate 300, magnesium hydroxide-aluminum hydroxide co-precipitate, 1,3-butylene glycol and castor oil were dissolved, a material in which tinidazole, crotamiton and dibutylhydroxytoluene were dispersed under heating, and a mixture of sorbitan monooleate and polyoxyethylene sorbitan monooleate. Then, to the resulting mixture was added a material in which sodium metaphosphate was dissolved in a small amount of distilled water, and finally, was added a mixture of a remaining D-sorbitol solution and tartaric acid adjusted to a temperature of about 60°C under stirring. Ointment thus obtained was applied with 1000 g/m² to an unwoven fabric and the fabric was cut into a size of 10 cm x 14 cm (280 mg of tinidazole was contained per 14 g of ointment) to obtain a patch.

### (Example 20) Dry patch (plaster)

### Prescription:

The patch is composed of tinidazole (2 g), liquid paraffin (8 g), dibutylhydroxytoluene (0.2 g), crotamiton (1 g), polyoxyethylene glycol monostearate (2 g), polyoxyethylene cetostearyl ether (20E.O.)(1.8 g), methacrylic acid-n-butyl acrylate copolymer (5 g), myristyl alcohol (8 g), natural rubber latex (as a solid material)(20 g), synthetic rubber SBR latex (as a solid material)(37 g), and polybutene (15 g).

### Preparation method:

Tinidazole, dibutylhydroxytoluene and crotamiton were dispersed by stirring under heating. Polyoxyethylene glycol monostearate, polyoxyethylene cetostearyl ether (20E.O.) and myristyl alcohol were added to the above mixture and the resulting mixture was mixed under heating. The resulting mixture was added to a molten state mixture of a methacrylic acid-n-butyl acrylate copolymer, a natural rubber latex and a synthetic rubber SBR latex while continuously stirring. To this mixture were also further added liquid paraffin and polybutene while continuously stirring. Ointment thus obtained was applied with 100 g/m² to an unwoven or woven fabric. After drying, the fabric was cut into a size of 10 cm x 14 cm (28 mg of tinidazole was contained per 1.4 g of ointment) to obtain plaster.

### (Example 21) Cream for external use

### Prescription:

The cream is composed of metronidazole (2 g), clotrimazole (0.1 g), clobetasol propionate (0.005 g), glycol monostearate (10 g), cetanol (7 g), liquid paraffin (9 g), white petrolatum (3.5 g), propylene glycol (6.5 g), sodium lauryl sulfate (1 g), and purified water (an amount making total 100 g).

### Preparation method:

Glycol monostearate, cetanol, liquid paraffin and white petrolatum were stirred under heating to about 85°C, and to the mixture was added a mixture of propylene glycol, sodium lauryl sulfate and purified water prepared by stirring under heating to about 85°C. Then, under stirring, metronidazole, clotrimazole and clobetasol propionate were added thereto. The resulting mixture was cooled to about 25°C while continuously stirring and the resulting cream was charged in a suitable vessel.

### (Example 22) Cream for external use

### Prescription:

The cream is composed of (a) metronidazole (2 g), lidocaine (0.05 g), prednisolone valerate acetate (0.005 g); (b) glycol monostearate (10 g), cetanol (7 g), liquid paraffin (9 g), white petrolatum (3.5 g); and (c) propylene glycol (6.5 g), sodium lauryl sulfate (1 g), and purified water (an amount making total 100 g).

### Preparation method:

Component(b) was stirred under heating at about 85°C, and to the mixture was added component(c) which had been stirred under heating to about 85°C, and component(a) was added to the above mixture while stirring. The mixture was cooled to about 25°C while continuously stirring, and the resulting cream was charged in a suitable vessel.

### (Example 23) Cream for external use

### Prescription:

The cream is composed of tinidazole (2 g), clotrimazole (0.1 g), clobetasol propionate (0.002 g), glycol monostearate (10 g), cetanol (7 g), liquid paraffin (9 g), white petrolatum (3.5 g), propylene glycol (6.5 g), sodium lauryl sulfate (1 g), and purified water (an amount making total 100 g).

### Preparation method:

Glycol monostearate, cetanol, liquid paraffin and white petrolatum were stirred under heating to about 85°C, and to the mixture was added a mixture of propylene glycol, sodium lauryl sulfate and purified water prepared by stirring under heating to about 85°C. Then, under stirring, to the mixture were added tinidazole, clotrimazole and clobetasol propionate. The mixture was cooled to about 25°C while continuously stirring, and the resulting cream was charged in a suitable vessel.

### (Example 24) Cream for external use

### Prescription:

The cream is composed of tinidazole (2 g), chloramphenicol (0.001 g), hydrocortisone acetate (0.001 g), glycol monostearate (8 g), cetanol (7 g), liquid paraffin (10 g), white petrolatum (3.5 g), propylene glycol (6.5 g), sodium lauryl sulfate (0.8 g), and purified water (an amount making total 100 g).

### Preparation method:

Glycol monostearate, cetanol, liquid paraffin and white petrolatum were stirred under heating to about 85°C, and to the mixture were added a mixture of propylene glycol, sodium lauryl sulfate and purified water prepared by stirring under heating to about 85°C. Then, under stirring, to the mixture were added tinidazole, chloramphenicol and hydrocortisone acetate. The mixture was cooled to about 25°C while continuously stirring, and the resulting cream was charged in a suitable vessel.

### (Example 25) Gel

### Prescription:

The gel is composed of (a) tinidazole (3 g), diphenhydramine hydrochloride (0.2 g), betamethasone (0.01 g), carpronium chloride (0.2 g); (b) polyoxyethylene oleyl alcohol ether (1 g); (c) polyoxyethylene glycol 1500 (6 g), polyoxyethylene glycol 400 (2 g), disodium EDTA (0.2 g); (d) dipropylene glycol (8 g); (e) aqueous phase: potassium hydroxide (0.1 g); (f) carboxyvinyl polymer (0.5 g), methyl cellulose (0.2 g), and purified water (an amount making total 100 g).

### Preparation method:

After (f) was homogeneously dissolved, (c) was added thereto and (a) was added thereto, and the mixture was heated, dissolved and dispersed. Subsequently, (b) was added to (d) and the mixture was melted by heating at about 60°C, and added to the above dispersion. The mixture was neutralized by the addition of (e) while stirring and cooled to a temperature of about 25°C. The resulting gel was charged in a suitable vessel.

### (Example 26) Cream for external use

### Prescription:

The cream is composed of metronidazole (2 g), glycol monostearate (10.4 g), cetanol (7.3 g), liquid paraffin (9 g), propylparaben (0.05 g), white petrolatum (3.5 g), propylene glycol (6.5 g), sodium lauryl sulfate (1 g), methylparaben (0.05 g), urea (2 g), and purified water (an amount making total 100 g).

### Preparation method:

Glycol monostearate, cetanol, liquid paraffin, propylparaben and white petrolatum were stirred while heating at about 85°C. A mixture of propylene glycol, sodium lauryl sulfate, methylparaben, urea and purified water were stirred while heating at about 85°C and added thereto. Metronidazole was added thereto while stirring. The mixture was cooled to about 25°C while continuously stirring and then charged in a suitable vessel.

### (Example 27) Shampoo

### Prescription:

The shampoo is composed of metronidazole (2 g), polyglycerin monolaurate (4 g), polyoxyethylene lauryl ether sodium sulfate (7 g), lauryl dimethylaminoacetate betaine (2.5 g), coconut fatty acid diethanol amide (4 g), polyethylene glycol (5 g), 1,3-butylene glycol (3 g), citric acid (suitable amount), and purified water (to make total amount 100 g).

### Preparation method:

Metronidazole is added to a mixture containing suitable amounts of polyethylene glycol and purified water and the mixture is melted by heating. In other vessel are weighed suitable amounts of polyglycerin monolaurate, polyoxyethylene lauryl ether sodium sulfate, lauryl dimethylacetate betaine, coconut fatty acid diethanol amide, polyethylene glycol, 1,3-butylene glycol and purified water, and the resulting mixture is heated to about 70°C while stirring and added to a mixture of metronidazole, polyethylene glycol and purified water. The mixture is adjusted to pH about 6.5 with citric acid, and is cooled until a temperature becomes about 25°C under stirring.

### (Example 28) Gel

### Prescription:

The gel is composed of metronidazole (1 g), polyethylene glycol (8 g), carboxyvinyl polymer (0.5 g), methyl cellulose (0.2 g), propylene glycol (5 g), glycerin (2 g), polyoxyethylene oleyl cetyl ether (1 g), isopropanol (5 g), sodium hydroxide (suitable amount), and purified water (an amount making total 100 g)

### Preparation method:

Polyethylene glycol was added to purified water and dissolved, and metronidazole was further added thereto and dissolved by heating. The solution was cooled to about 50°C, and to the solution was added a material in which polyoxyethylene cetyl ether was added to propylene glycol and glycerin and heated to about 50°C under stirring. While further continuously stirring, sodium hydroxide was added thereto and a pH thereof was adjusted to about 6.8. The mixture was further cooled to about 40°C, isopropanol was then added thereto, and after cooling the mixture to about 25°C, it was charged in a suitable vessel.

### (Example 29) Ointment

### Prescription:

The ointment is composed of (a) metronidazole (2 g), crotamiton (2 g); (b) stearic acid (2 g), glycol monostearate (12 g), polyoxyethylene glycol monostearate (3 g), polyoxyethylene cetostearyl ether (12E.O.)(1 g), polyoxyethylene cetostearyl ether (20E.O.)(1 g), cetanol (2 g), liquid paraffin (8 g); and (c) 1,3-butylene glycol (7 g), glycerin (5 g), and purified water (an amount making total 100 g)

### Preparation method:

Component(b) was dissolved and adjusted to about 75°C. To the solution was added under stirring a material in which component(c) had been dissolved and adjusted to about 75°C, followed by the addition of component(a). The mixture was cooled to a temperature of about 25°C while continuously stirring, and then, charged in a suitable vessel.

### (Example 30) Cream for external use

### Prescription:

The cream is composed of (a) metronidazole (2 g), lidocaine (0.05 g), prednisolone valerate acetate (0.005 g); (b) glycol monostearate (10 g), cetanol (7 g), liquid paraffin (9 g), white petrolatum (3.5 g); (c) propylene glycol (6.5 g), sodium lauryl sulfate (1 g), purified water (an amount making the total amount 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 31) Cream for external use

### Prescription:

The cream is composed of (a) metronidazole (2.5 g), ketoconazole (0.1 g); (b) stearic acid (5 g), stearyl alcohol (5 g), liquid paraffin (5 g), isopropyl myristate (1 g), Span 60 (1 g), thymol(0.2 g); and (c) Tween 60 (0.5 g), propylene glycol (5 g), triethanolamine (0.4 g), and distilled water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 32) Cream for external use

### Prescription:

The cream is composed of (a) metronidazole (3 g), pipemidic acid trihydrate (0.1 g), prednisolone (0.001 g); (b) glycol monostearate (5 g), polyoxyethylene (23) cetyl ether (2 g), cetanol (6 g), white petrolatum (5 g), liquid paraffin (5 g), caprylic/capric acid triglyceride (5 g), octyl dodecyl myristate (3 g), propyl parahydroxybenzoate (0.1 g); and (c) propylene glycol (5 g), methyl parahydroxybenzoate (0.1 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 33) Ointment for external use

### Prescription:

The ointment is composed of (a) metronidazole (2 g), crotamiton (1 g), fluorocinolone acetonide (0.001 g); (b) white petrolatum (45 g), cetanol (20 g), polyoxyethylene hydrogenated castor oil (5 g), Tween 80 (2 g), liquid paraffin (5 g), propyl parahydroxybenzoate(0.1 g); and (c) methyl parahydroxybenzoate (0.1 g), and distilled water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 34) Ointment for external use

### Prescription:

The ointment is composed of (a) metronidazole (2 g), diphenhydramine hydrochloride (0.2 g), lidocaine (0.1 g); (b) stearyl alcohol (7 g), cetanol (3 g), white petrolatum (30 g), glycol monostearate (10 g), span 80 (1.5 g), liquid paraffin (5 g); and (c) propylene glycol (5 g), Tween 80 (1 g), and distilled water (an amount making total 100 g)

### Preparation method:

Component(c) was dissolved and adjusted to about 85°C. To the solution was added under stirring a material in which component(b) had been dissolved and adjusted to about 85°C, followed by the addition of component(a). The mixture was cooled to a temperature of about 25°C while continuously stirring, and then, charged in a suitable vessel.

### (Example 35) Ointment for external use

### Prescription:

The ointment is composed of (a) metronidazole (2 g), gentamicin sulfate (0.005 g); (b) glycol monostearate (15 g), polyoxyethylene glycol monostearate (3 g), polyoxyethylene cetostearyl ether (2 g), cetanol (5 g), beeswax (5 g), white petrolatum (20 g), and (c) distilled water (an amount making the total amount 100 g)

### Preparation method:

Component(c) was dissolved and adjusted to about 85°C. To the solution was added a material in which component(b) had been dissolved and adjusted to about 85°C under stirring, followed by the addition of component(a). The mixture was cooled to a temperature of about 25°C while continuously stirring, and then, charged in a suitable vessel.

### (Example 36) Lotion

### Prescription:

The lotion is composed of (a) metronidazole (2 g), betamethasone valerate (0.005 g), bifonazole (0.05 g); (b) stearic acid (2 g), cetanol (1.5 g), petrolatum (4 g), squalane (5 g), caprylic/capric acid trigliceride (2 g), sorbitan monooleate (2 g), polyethylene glycol (5 g); (c) dipropylene glycol (5 g), triethanol amine (0.7 g), purified water (60 g); (d) isopropanol (10 g), and purified water (an amount making total 100 g)

### Preparation method:

Component(c) was dissolved and adjusted to about 75°C. To the solution was added under stirring a material in which component(b) had been dissolved and adjusted to about 75°C, followed by the addition of component(a). The mixture was rapidly cooled to a temperature of about 40°C while continuously stirring, then component (d) was added thereto, and the mixture was cooled to about 25°C under stirring. The resulting lotion was charged in a suitable sealed vessel.

### (Example 37) Patch

### Prescription:

The patch is composed of (a) metronidazole (3 g), crotamiton (1 g), prednisolone (0.05 g); (b) D-sorbitol (70%)(30 g), purified water (9 g), kaolin (13 g), titanium oxide (1 g); (c) gelatin (1 g), purified water (4 g); (d) sodium metaphosphate (0.1 g), purified water (1 g); (e) sodium polyacrylate (5 g), starch grafted acrylate 300 (1 g), propylene glycol (5 g), castor oil (1 g), magnesium hydroxide-aluminum hydroxide co-precipitate (0.25 g), sorbitan monooleate (0.5 g), polyoxyethylene sorbitan monooleate (0.5 g); (f) D-sorbitol (70%) (14 g), dibutylhydroxytoluene (0.2 g); (g) methacrylic acid and n-butyl acrylate copolymer (3 g); and (h) D-sorbitol (70%)(4.9 g), tartaric acid (1.5 g)

### Preparation method:

Component(b) was dissolved and adjusted to about 40°C. To the solution was added under stirring a material in which component (d) had been dissolved and adjusted to about 60°C, followed by the addition of component (c) and then component (g). To the resulting mixture was added a material in which components (a) and (e) had been well mixed, followed by the addition of component(f), and then, component (h) was added thereto little by little under stirring. 14 g of ointment thus obtained was applied evenly to an unwoven fabric having a size of 10 cm x 14 cm to obtain a patch.

### (Example 38) Patch (Plaster)

### Prescription:

The plaster is composed of (a) metronidazole(3 g), indometacin (1 g); (b) liquid paraffin (7 g), isopropyl myristate (3 g), polybutene (15 g), 1,3-pentadiene copolymer resin (26 g); (c) polyoxyethylene sorbitan monostearate (1.5 g), zinc oxide (3 g), titanium oxide (2 g), dibutylhydroxytoluene (0.2 g), crotamiton (1 g); (d) kaolin (6 g); (e) natural rubber latex (as a solid material)(15 g), synthetic rubber SBR (as a solid material)(17 g); and (f) glycerin (0.25 g), purified water (1 g), and sodium polyacrylate (0.05 g)

### Preparation method:

component(b) was mixed and melted at about 110°C and a temperature thereof was adjusted to about 90□C. To this mixture was added conponent(a), and after adjusting to about 70°C, a material in which component(c) and (d) had been mixed was added to the above mixture. Under further stirring, conpornent(f) was added and component(e) was also added thereto at about 70°C. 14 g of ointment thus obtained was applied evenly to an unwoven fabric in a size of 10 cm x 14 cm to obtain a patch.

### (Example 39) Gel

### Prescription:

The gel is composed of (a) metronidazole (3 g), diphenhydramine hydrochloride (0.5 g), betamethasone (0.01 g); (b) polyoxyethylene oleyl alcohol ether (1 g); (c) polyethylene glycol 1500 (6 g), polyoxyethylene glycol 400 (2 g), disodium EDTA (0.2 g); (d) dipropylene glycol (8 g); (e) potassium hydroxide (0.1 g); (f) carboxyvinyl polymer (0.5 g), methylcellulose (0.2 g), purified water (an amount making total 100 g)

### Preparation method:

After component(f) was homogeneously dissolved, component(c) was added to the solution, and then, component(a) was added to the same to dissolve or disperse therein under heating. To the resulting material was added a material in which component(b) had been added to component(d) and mixed to melt at about 60°C. Moreover, under stirring, component(e) was added thereto while stirring to neutralize the mixture, and the mixture was cooled to about 25°C. The resulting gel was charged in a suitable vessel.

### (Example 40) Cream for external use

### Prescription :

The cream is composed of (a) tinidazole (1 g), prednisolone valerate acetate (0.005 g); (b) glycol monostearate (8 g), cetanol (7 g), liquid paraffin (10 g), white petrolatum (3.5 g); (c) propylene glycol (6.5 g), sodium lauryl sulfate (1 g), purified water (an amount making total 100 g).

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 41) Cream for external use

### Prescription:

The cream is composed of (a) tinidazole (2.5 g), azelastine hydrochloride (0.02 g), prednisolone acetate (0.001 g); (b) glycol monostearate(5 g), polyoxyethylene(23) cetyl ether (2 g), cetanol(5 g), white petrolatum (3.5 g), liquid paraffin (5 g), isopropyl myristate (5 g), octyl dodecyl myristate (3 g), propyl parahydroxybenzoate (0.15 g); (c) propylene glycol (7 g), methyl parahydroxybenzoate (0.15 g), distilled water (an amount making the total amount 100 g).

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 42) Cream

### Prescription:

The cream is composed of (a) tinidazole (2.0 g), tolnaftate (0.05 g), (b) stearic acid (5 g), stearyl alcohol (5 g), liquid paraffin (5 g), isopropyl myristate (1 g), Span 60 (1.2 g), thymol(0.2 g); and (c) Tween 60 (0.7 g), propylene glycol (6 g), triethanolamine (0.4 g), and purified water (an amount making the total amount 100 g).

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 43) Cream

### Prescription:

The cream is composed of (a) tinidazole (2 g), aciclovir (0.2 g); (b) stearic acid (5 g), stearyl alcohol (5 g), liquid paraffin (5 g), isopropyl myristate (1 g), Span 60 (1.2 g), thymol (0.2 g); (c) Tween 60 (0.7 g), propylene glycol (6 g), triethanolamine (0.4 g), purified water (an amount making total 100 g).

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 44) Ointment for external use

### Prescription:

The ointment is composed of (a) tinidazole (2 g), diclofenac sodium (0.05 g), crotamiton (1 g), fluocinolone acetonide (0.001 g); (b) white petrolatum (45 g), cetanol (20 g), polyoxyethylene hydrogenated castor oil (5 g), Tween 80 (2 g), liquid paraffin (5 g), propyl parahydroxybenzoate (0.1 g); and (c) methyl parahydroxybenzoate (0.1 g), and purified water (an amount making the total amount 100 g).

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 45) Ointment for external use

### Prescription:

The ointment is composed of (a) tinidazole (2 g), extract of calves blood (1 g), diphenhydramine hydrochloride (0.2 g), lidocaine (0.1 g); (b) stearyl alcohol (7 g), cetanol (3 g), white petrolatum (30 g), glycol monostearate (10 g), span 80 (1.5 g), liquid paraffin (5 g); and (c) propylene glycol (5 g), Tween 80 (1 g), and distilled water (an amount making the total amount 100 g).

### Preparation method:

Component(c) was dissolved and adjusted to about 85°C. To the solution was added under stirring a material in which componentb) had been dissolved and adjusted to about 85°C, followed by the addition of component(a). The mixture was cooled to a temperature of about 25°C while continuously stirring, and then, charged in a suitable vessel.

### (Example 46) Ointment for external use

### Prescription:

The ointment is composed of (a) tinidazole (2 g), gentamicin sulfate (0.005 g), (b) glycol monostearate (15 g), polyoxyethylene glycol monostearate (3 g), polyoxyethylene cetostearyl ether (2 g), cetanol (5 g), beeswax (5 g), white petrolatum (20 g); (c) distilled water (an amount making the total amount 100 g).

### Preparation method:

Component(c) was adjusted to about 85°C. To the solution was added under stirring a material in which component(b) had been dissolved and adjusted to about 85°C, followed by the addition of component(a). The mixture was cooled to a temperature of about 25°C while continuously stirring, and then, charged in a suitable vessel.

### (Example 47) Lotion

### Prescription:

The lotion is composed of (a) tinidazole (2 g), nofloxacin (0.005 g), clotrimazole (0.05 g); (b) stearic acid (2 g), cetanol (1.5 g), white petrolatum (4 g), squalane (5 g), caprylic/caproic acid triglyceride (2 g), sorbitan monooleate (2 g), polyethylene glycol (5 g); (c) dipropylene glycol (5 g), triethanolamine (0.7 g), purified water (60 g); and (d) isopropanol (10 g), purified water (an amount making total 100 g).

### Preparation method:

Component(c) was dissolved and adjusted to about 70°C. To the solution was added under stirring a material in which component(b) had been dissolved and adjusted to about 70°C, followed by the addition of component(a). The mixture was rapidly cooled to a temperature of about 40°C while continuously stirring, then compoent(d) was added thereto, and the mixture was cooled to about 25°C under stirring. The resulting lotion was charged in a suitable sealed vessel.

### (Example 48) Patch

### Prescription:

The patch is composed of (a) tinidazole (3 g), crotamiton (1 g), prednisolone (0.05 g); (b) D-sorbitol (70%)(30 g), purified water (9 g), kaolin (13 g), titanium oxide (1 g); (c) gelatin (1 g), purified water (4 g); (d) sodium metaphosphate (0.1 g), purified water (1 g); (e) sodium polyacrylate (5 g), starch grafted acrylate 300 (1 g), propylene glycol (5 g), castor oil (1 g), magnesium hydroxide-aluminum hydroxide co-precipitate (0.25 g), sorbitan monooleate (0.5 g), sorbitan polyoxyethylene monooleate (0.5 g); (f) D-sorbitol (70%)(14 g), dibutylhydroxyltoluene (0.2 g); (g) methacrylic acid-n-butyl acrylate copolymer (3 g); and (h) D-sorbitol (70%)(4.9 g), tartaric acid (1.5 g).

### Preparation method:

A temperature of component(b) was adjusted to about 40°C, and a material in which a temperature of component(d) had been adjusted to about 60°C was added to component(b) under stirring. Then, comopnent(c) was added to the above mixture and component(g) was added thereto while stirring. To this mixture was added a material in which component(a) and component(e) had been well mixed, followed by the addition of component(f), and component(h) is added thereto while stirring. From the resulting ointment, 14 g was weighed and applied evenly to an unwoven fabric in a size of 10 cm x 14 cm to obtain a patch.

### (Example 49) Patch (plaster)

### Prescription:

The patch is composed of (a) tinidazole (3 g), indometacin (1 g); (b) liquid paraffin (7 g), isopropyl myristate (3 g), polybutene (15 g), 1,3-pentadiene copolymer resin (26 g); (c) polyoxyethylene sorbitan monostearate (1.5 g), zinc oxide (3 g), titanium oxide (2 g), dibutylhydroxyltoluene (0.2 g), crotamiton (1 g); (d) kaolin (6 g); (e) natural rubber latex (as a solid material) (15 g), synthetic rubber SBR (as a solid material) (17 g); (f) glycerin (0.25 g), purified water (1 g), sodium polyacrylate (0.05 g).

### Preparation method:

Component(b) was mixed and melted at a temperature of about 110°C, and then, adjusted to about 90°C, and component(a) was added thereto and the resulting mixture was adjusted to a temperature of about 70°C. To the mixture was added a material in which mixture of component(c) and (d) had been mixed. Further, component(f) was added thereto and (e) was added at a temperature of about 70°C. The resulting ointment was applied with 100 g/m² to an unwoven or woven fabric and the fabric was cut into a size of 10 cm x 14 cm.

### (Example 50) Cream for external use

### Prescription:

The cream is composed of (a) tinidazole (1 g), fluorouracil (0.02 g), prednisolone valerate acetate (0.005 g); (b) glycol monostearate (8 g), cetanol (7 g), liquid paraffin (10 g), white petrolatum (3.5 g); and (c) propylene glycol (6.5 g), sodium lauryl sulfate (1 g), and purified water (an amount making the total amount 100 g).

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 51) Cream for external use

### Prescription:

The cream is composed of metronidazole (2 g), glycol monostearate (10 g), cetanol (7 g), liquid paraffin (9 g), white petrolatum (3.5 g), propylene glycol (6.5 g), sodium lauryl sulfate (1 g), and purified water (an amount making the total amount 100 g)

### Preparation method:

Glycol monostearate, cetanol, liquid paraffin and white petrolatum were mixed by stirring under heating at about 85°C. To the above mixture was added a material in which propylene glycol, sodium lauryl sulfate and purified water had been mixed by stirring under heating at about 85°C, and metronidazole was added thereto while stirring. The resulting cream was charged in a suitable vessel.

### (Example 52) Cream for external use

### Prescription:

The cream is composed of (a) metronidazole (0.5 g); (b) glycol monostearate (10 g), cetanol (7 g), liquid paraffin (9 g), white petrolatum (3.5 g); and (c) propylene glycol (6.5 g), sodium lauryl sulfate (1 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 53) Cream for external use

### Prescription:

The cream is composed of (a) tinidazole (0.5 g); (b) glycol monostearate (10 g), cetanol (7 g), liquid paraffin (9 g), white petrolatum (3.5 g); (c) propylene glycol (6.5 g), sodium lauryl sulfate (1 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 54) Cream for external use

### Prescription:

The cream is composed of (a) metronidazole (1.5 g), ketoconazole (0.1 g); (b) glycol monostearate(5 g), polyoxyethylene (23) cetyl ether (2 g), stearic acid (0.5 g), cetanol (5 g), white petrolatum (3.5 g), liquid paraffin (5 g), isopropyl myristate (5 g), octyl dodecyl myristate (3 g), propyl parahydroxybenzoate (0.15 g); and (c) propylene glycol (7 g), methyl parahydroxybenzoate (0.15 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 55) Cream for external use

### Prescription:

The cream is composed of (a) tinidazole (1.5 g), ketoconazole (0.1 g); (b) glycol monostearate (5 g), polyoxyethylene (23) cetyl ether (2 g), stearic acid (0.5 g), cetanol (5 g), white petrolatum (3.5 g), liquid paraffin (5 g), isopropyl myristate (5 g), octyl dodecyl myristate (3 g), propyl parahydroxybenzoate (0.15 g); and (c) propylene glycol (7 g), methyl parahydroxybenzoate (0.15 g), and distilled water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 56) Cream for external use

### Prescription:

The cream is composed of (a) metronidazole (3 g), norfloxacin (0.2 g); (b) stearic acid (5 g), stearyl alcohol (5 g), liquid paraffin (5 g), isopropyl myristate (1 g), Span 60 (1.2 g), thymol (0.2 g); and (c) Tween 60 (0.7 g), propylene glycol (6 g), triethanolamine (0.4 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 57) Cream for external use

### Prescription:

The cream is composed of (a) tinidazole (3 g), norfloxacin (0.2 g); (b) stearic acid (5 g), stearyl alcohol (5 g), liquid paraffin (5 g), isopropyl myristate (1 g), Span 60 (1.2 g), thymol (0.2 g); and (c) Tween 60 (0.7 g), propylene glycol (6 g), triethanolamine (0.4 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 58) Ointment for external use

### Prescription:

The ointment is composed of (a) metronidazole (2 g), diclofenac sodium (0.1 g); (b) white petrolatum (45 g), cetanol(20 g), polyoxyethylene hydrogenated castor oil (5 g), Tween 80 (2 g), crotamiton (3 g), liquid paraffin (5 g), propyl parahydroxybenzoate (0.1 g); and (c) methyl parahydroxybenzoate (0.1 g), distilled water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 59) Ointment for external use

### Prescription:

The ointment is composed of (a) tinidazole (2 g), diclofenac sodium (0.1 g); (b) white petrolatum (45 g), cetanol (20 g), polyoxyethylene hydrogenated castor oil (5 g), Tween 80 (2 g), crotamiton (3 g), liquid paraffin (5 g), propyl parahydroxybenzoate (0.1 g); and (c) methyl parahydroxybenzoate (0.1 g), and distilled water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 60) Cream for external use

### Prescription:

The cream is composed of (a) metronidazole (10 g); (b) glycol monostearate (7 g), polyoxyethylene glycol monostearate (3 g), polyoxyethylene cetostearyl ether (2 g), polyoxyethylene hydrogenated castor oil (1 g), cetanol (5 g), beeswax (1 g), liquid paraffin (3 g); and (c) polyethylene glycol (5 g), 1,3-butylene glycol (4 g), and distilled water (an amount making total 100 g)

### Preparation method:

Component(c) was dissolved and adjusted to about 85°C. To the solution was added under stirring a material in which component(b) had been dissolved and adjusted to about 85°C, followed by the addition of component(a). The mixture was cooled to a temperature of about 25°C while continuously stirring, and then, charged in a suitable vessel.

### (Example 61) Cream for external use

### Prescription:

The cream is composed of (a) tinidazole (10 g); (b) glycol monostearate (7 g), polyoxyethylene glycol monostearate (3 g), polyoxyethylene cetostearyl ether (2 g), polyoxyethylene hydrogenated castor oil (1 g), cetanol (5 g), beeswax (1 g), liquid paraffin (3 g); and (c) polyethylene glycol (5 g), 1,3-butylene glycol (4 g), and distilled water (an amount making total 100 g)

### Preparation method:

Component(c) was dissolved and adjusted to about 85°C. To the solution was added under stirring a material in which component(b) had been dissolved and adjusted to about 85°C, followed by the addition of component(a). The mixture was cooled to a temperature of about 25°C while continuously stirring, and then, charged in a suitable vessel.

### (Example 62) Lotion

### Prescription:

The lotion is composed of (a) metronidazole (5 g); (b) stearic acid (2 g), cetanol (1.5 g), white petrolatum (4 g), squalane (5 g), caprylic/capric acid triglyceride (2 g), sorbitan monooleate (2 g), polyethylene glycol (5 g); and (c) dipropylene glycol (5 g), triethanol amine (0.7 g), purified water (60 g); (d) isopropanol (10 g), and purified water (an amount making total 100 g)

### Preparation method:

Component(c) was dissolved and adjusted to about 70°C. To the solution was added under stirring a material in which component(b) had been dissolved and adjusted to about 70°C, followed by the addition of component(a). The mixture was cooled to a temperature of about 40°C while continuously stirring, and then, component(d) was added thereto, and the mixture was cooled to about 25°C under stirring. The resulting lotion was charged in a suitable sealed vessel.

### (Example 63) Lotion

### Prescription:

The lotion is composed of (a) metronidazole (5 g), tranilast (0.4 g); (b) stearic acid (2 g), cetanol (1.5 g), white petrolatum (4 g), squalane (5 g), caprylic/capric acid triglyceride (2 g), sorbitan monooleate (2 g), polyethylene glycol (5 g); and (c) dipropylene glycol (5 g), triethanol amine (0.7 g), purified water (60 g); (d) isopropanol (10 g), and purified water (an amount making total 100 g)

### Preparation method:

Component(c) was dissolved and adjusted to about 70°C. To the solution was added under stirring a material in which component(b) had been dissolved and adjusted to about 70°C, followed by the addition of component(a). The mixture was cooled to a temperature of about 40°C while continuously stirring, and then, component(d) was added thereto, and the mixture was cooled to about 25°C under stirring. The resulting lotion was charged in a suitable sealed vessel.

### (Example 64) Lotion

### Prescription:

The lotion is composed of (a) tinidazole (3 g), clotrimazole (0.1 g), prednisolone acetate (0.005 g); (b) stearic acid (2 g), cetanol (1.5 g), white petrolatum (4 g), squalane (5 g), caprylic/capric acid triglyceride (2 g), sorbitan monooleate (2 g), polyethylene glycol (5 g); and (c) dipropylene glycol (5 g), triethanol amine (0.7 g), purified water (60 g); (d) isopropanol (10 g), and purified water (an amount making total 100 g)

### Preparation method:

Component(c) was dissolved and adjusted to about 70°C. To the solution was added under stirring a material in which component(b) had been dissolved and adjusted to about 70°C, followed by the addition of component(a). The mixture was cooled to a temperature of about 40°C while continuously stirring, and then, component(d) was added thereto, and the mixture was cooled to about 25°C under stirring. The resulting lotion was charged in a suitable sealed vessel.

### (Example 65) Cream for external use

### Prescription:

The cream is composed of (a) metronidazole (2 g); (b) glycol monostearate (10 g), cetanol (7 g), liquid paraffin (9 g), white petrolatum (3.5 g); and (c) urea (2 g), propylene glycol (6.5 g), sodium lauryl sulfate (1 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 66) Cream for external use

### Prescription:

The cream is composed of (a) tinidazole (2 g); (b) glycol monostearate (10 g), cetanol (7 g), liquid paraffin (9 g), white petrolatum (3.5 g); and (c) urea (2 g), propylene glycol (6.5 g), sodium lauryl sulfate (1 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 67) Cream for external use

### Prescription:

The cream is composed of (a) metronidazole (10 g); (b) glycol monostearate (10 g), cetanol (7 g), liquid paraffin (9 g), white petrolatum (2.5 g); and (c) urea (2 g), polyethylene glycol (7 g), Tween 80 (1 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 68) Cream for external use

### Prescription:

The cream is composed of (a) tinidazole (10 g); (b) glycol monostearate (10 g), cetanol (7 g), liquid paraffin (9 g), white petrolatum (2.5 g); and (c) urea (2 g), polyethylene glycol (7 g), Tween 80 (1 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 69) Ointment for external use

### Prescription:

The ointment is composed of (a) metronidazole (3 g); (b) white petrolatum (45 g) cetanol (20 g), polyoxyethylene hydrogenated castor oil (5 g), liquid paraffin (5 g), propyl parahydroxybenzoate (0.1 g); and (c) methyl parahydroxybenzoate (0.1 g), Tween 80 (2 g), polyethylene glycol (5 g), and distilled water (an amount making total 100 g)

### Preparation method:

Comonent(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 70) Ointment for external use

### Prescription:

The ointment is composed of (a) tinidazole (3 g); (b) white petrolatum (45 g), cetanol (20 g), polyoxyethylene hydrogenated castor oil (5 g), liquid paraffin (5 g), propyl parahydroxybenzoate (0.1 g); and (c) methyl parahydroxybenzoate (0.1 g), Tween 80 (2 g), polyethylene glycol (5 g), and distilled water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 71) Lotion

### Prescription:

The lotion is composed of (a) metronidazole (3 g); (b) stearic acid (2 g), cetanol (1.5 g), white petrolatum (4 g), squalane (5 g), caprylic/caproic acid triglyceride (2 g), sorbitan monooleate (2 g); (c) polyethylene glycol (5 g), dipropylene glycol (5 g), triethanol amine (0.2 g), purified water (60 g); and (d) isopropanol (10 g), and purified water (an amount making total 100 g)

### Preparation method:

Component(c) was dissolved and adjusted to about 70°C. To the solution was added under stirring a material in which component(b) had been dissolved and adjusted to about 70°C, followed by the addition of component(a). The mixture was cooled to a temperature of about 40°C while continuously stirring, and then, component(d) was added thereto, and the mixture was cooled to about 25°C under stirring. The resulting lotion was charged in a suitable sealed vessel.

### (Example 72) Lotion

### Prescription.

The lotion is composed of (a) tinidazole (3 g); (b) stearic acid (2 g), cetanol (1.5 g), white petrolatum (4 g), squalane (5 g), caprylic/caproic acid triglyceride (2 g), sorbitan monooleate (2 g); (c) polyethylene glycol (5 g), dipropylene glycol (5 g), triethanol amine (0.2 g), purified water (60 g); and (d) isopropanol (10 g), and purified water (an amount making the total amount 100 g)

### Preparation method:

Component(c) was dissolved and adjusted to about 70°C. To the solution was added under stirring a material in which component(b) had been dissolved and adjusted to about 70°C, followed by the addition of component(a). The mixture was cooled to a temperature of about 40°C while continuously stirring, and then, component(d) was added thereto, and the mixture was cooled to about 25°C under stirring. The resulting lotion was charged in a suitable sealed vessel.

### (Example 73) Cream for external use

### Prescription:

The cream is composed of (a) metronidazole (2 g), tranilast (0.1 g); (b) glycol monostearate (5 g), polyoxyethylene (23) cetyl ether (2 g), stearic acid (0.5 g), cetanol (5 g), white petrolatum (3.5 g), liquid paraffin (5 g), isopropyl myristate (5 g), octyl dodecyl myristate (3 g), propyl parahydroxybenzoate (0.15 g); and (c) propylene glycol (7 g), methyl parahydroxybenzoate (0.15 g), and distilled water (an amount making total 100 g)

### Preparation method:

Component(b) was dissolved and adjusted to about 75°C. To the solution was added under stirring a material in which component(c) had been dissolved and adjusted to about 75°C, followed by the addition of component(a). The mixture was cooled to a temperature of about 25°C while continuously stirring, and then, charged in a suitable vessel.

### (Example 74) Cream for external use

### Prescription:

The cream is composed of (a) tinidazole (2 g), tranilast (0.1 g); (b) glycol monostearate (5 g), polyoxyethylene (23) cetyl ether (2 g), stearic acid (0.5 g), cetanol (5 g), white petrolatum (3.5 g), liquid paraffin (5 g), isopropyl myristate (5 g), octyl dodecyl myristate (3 g), propyl parahydroxybenzoate (0.15 g); and (c) propylene glycol (7 g), methyl parahydroxybenzoate (0.15 g), and distilled water (an amount making total 100 g)

### Preparation method:

Component(b) was dissolved and adjusted to about 75°C. To the solution was added under stirring a material in which component(c) had been dissolved and adjusted to about 75°C, followed by the addition of component(a). The mixture was cooled to a temperature of about 25°C while continuously stirring, and then, charged in a suitable vessel.

### (Example 75) Cream for external use

### Prescription:

The cream is composed of (a) metronidazole (1.5 g); (b) stearic acid (5 g), cetanol (5 g), polyoxyethylene stearyl ether (3 g); and (c) glycerin (6 g), 1,3-butylene glycol (4 g), triethanol amine (0.3 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 76) Cream for external use

### Prescription:

The cream is composed of (a) metronidazole (3.0 g); (b) stearic acid (5 g), cetanol (5 g), polyoxyethylene stearyl ether (3 g); and (c) glycerin (6 g), 1,3-butylene glycol (4 g), triethanol amine (0.3 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 77) Cream for external use

### Prescription: -

The cream is composed of (a) tinidazole (1.5 g); (b) stearic acid (5 g), cetanol (5 g), polyoxyethylene stearyl ether (3 g); and (c) glycerin (6 g), 1,3-butylene glycol (4 g), triethanol amine (0.3 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 78) Cream for external use

### Prescription:

The cream si composed of (a) tinidazole (3.0 g); (b) stearic acid (5 g), cetanol (5 g), polyoxyethylene stearyl ether (3 g); and (c) glycerin (6 g), 1,3-butylene glycol (4 g), triethanol amine (0.3 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 79) Cream for external use

### Prescription:

The cream is composed of(a) metronidazole (1.0 g); (b) stearic acid (0.5 g), glycol monostearate (8 g), stearyl alcohol (5 g), liquid paraffin (8 g); and (c) propylene glycol (6 g), glycerin (4 g), sodium polyoxyethylene lauryl ether sulfate (1 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 80) Cream for external use

### Prescription:

The cream is composed of (a) metronidazole (2.5 g); (b) stearic acid (0.5 g), glycol monostearate (8 g), stearyl alcohol (5 g), liquid paraffin (8 g); and (c) propylene glycol (6 g), glycerin (4 g), sodium polyoxyethylene lauryl ether sulfate (1 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 81) Cream for external use

### Prescription:

The cream is composed of (a) tinidazole (1.0 g); (b) stearic acid (0.5 g), glycol monostearate (8 g), stearyl alcohol (5 g), liquid paraffin (8 g); and (c) propylene glycol (6 g), glycerin (4 g), sodium polyoxyethylene lauryl ether sulfate (1 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 82) Cream for external use

### Prescription:

The cream is composed of (a) tinidazole (2.5 g); (b) stearic acid (0.5 g), glycol monostearate (8 g), stearyl alcohol (5 g), liquid paraffin (8 g); and (c) propylene glycol (6 g), glycerin (4 g), sodium polyoxyethylene lauryl ether sulfate (1 g), and purified water (an amount making total 100 g)

### Preparation method:

Component(a) was dissolved in a suitable amount of purified water under heating. Then, to the mixture was added component (c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 83) Cream for external use

### Prescription:

The cream is composed of (a) metronidazole (1.0 g); (b) glycol monostearate (10.4 g), cetanol (7.3 g), liquid paraffin (9 g), white petrolatum (3.5 g), propylparaben (0.05 g); and (c) propylene glycol (6.5 g), methylparaben (0.05 g), sodium lauryl sulfate (1 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 84) Cream for external use

### Prescription:

The cream is composed of (a) metronidazole (1.8 g); (b) glycol monostearate (10.4 g), cetanol (7.3 g), liquid paraffin (9 g), white petrolatum (3.5 g), propylparaben (0.05 g); and (c) propylene glycol (6.5 g), methylparaben (0.05 g), sodium lauryl sulfate (1 g), the purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 85) Cream for external use

### Prescription:

The cream is composed of (a) tinidazole (1.0 g); (b) glycol monostearate (10.4 g), cetanol (7.3 g), liquid paraffin (9 g), white petrolatum (3.5 g), propylparaben (0.05 g); and (c) propylene glycol (6.5 g), methylparaben (0.05 g), sodium lauryl sulfate (1 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 86) Cream for external use

### Prescription:

The cream is composed of (a) tinidazole (2.0 g); (b) glycol monostearate (10.4 g), cetanol (7.3 g), liquid paraffin (9 g), white petrolatum (3.5 g), propylparaben (0.05 g); and (c) propylene glycol (6.5 g), methylparaben (0.05 g), sodium lauryl sulfate (1 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 87) Cream for external use

### Prescription:

The cream is composed of (a) tinidazole (1.0 g); (b) glycol monostearate (10.4 g), cetanol (7.3 g), liquid paraffin (9 g), white petrolatum (3.5 g), propylparaben (0.05 g); and (c) propylene glycol (6.5 g), methylparaben (0.05 g), sodium lauryl sulfate (1 g), and purified water (an amount making total 100 g)

### Preparation method:

Component(a) was dissolved in a suitable amount of purified water under heating. Then, to the mixture was added component(c) heated to about 85°C, and the resulting mixture was added to a solution of component(b) dissolved by heating at about 85°C whereby the mixture was emulsified under stirring. The emulsion was cooled to a temperature of about 25°C while continuously stirring, and then, charged in a suitable vessel.

### (Example 88) Cream for external use

### Prescription:

The cream is composed of (a) metronidazole (2.0 g); (b) glycol monostearate (10.4 g), cetanol (7.3 g), liquid paraffin (9 g), white petrolatum (3.5 g), propylparaben (0.05 g); and (c) propylene glycol (6.5 g), methylparaben (0.05 g), sodium lauryl sulfate (1 g), and purified water (an amount making total 100 g)

### Preparation method:

Component(a) was dissolved in a suitable amount of purified water under heating. Then, to the mixture was added component(c) heated to about 85°C, and the resulting mixture was added to a solution of component(b) dissolved by heating at about 85°C whereby the mixture was emulsified under stirring. The emulsion was cooled to a temperature of about 25°C while continuously stirring, and then, charged in a suitable vessel.

### (Example 89) Cream for external use

### Prescription:

The cream is composed of (a) metronidazole (1.8 g); (b) glycol monostearate (10.4 g), cetanol (7.3 g), liquid paraffin (9 g), white petrolatum (3.5 g), propylparaben (0.05 g); and (c) propylene glycol (6.5 g), methylparaben (0.05 g), sodium lauryl sulfate (1 g), and purified water (an amount making total 100 g)

### Preparation method:

Component(a) was dissolved in a suitable amount of purified water under heating. Then, to the mixture was added component(c) heated to about 85°C, and the resulting mixture was added to a solution of component(b) dissolved by heating at about 85°C whereby the mixture was emulsified under stirring. The emulsion was cooled to a temperature of about 25°C while continuously stirring, and then, charged in a suitable vessel.

### (Example 90) Cream for external use

### Prescription:

The cream is composed of (a) tinidazole (1.5 g); (b) glycol monostearate (10.4 g), cetanol (7.3 g), liquid paraffin (9 g), white petrolatum (3.5 g), propylparaben (0.05 g); and (c) propylene glycol (6.5 g), methylparaben (0.05 g), sodium lauryl sulfate (1 g), and purified water (an amount making total 100 g)

### Preparation method:

Component(a) was dissolved in a suitable amount of purified water under heating. Then, to the mixture was added component(c) heated to about 85°C, and the resulting mixture was added to a solution of component(b) dissolved by heating at about 85°C whereby the mixture was emulsified under stirring. The emulsion was cooled to a temperature of about 25°C while continuously stirring, and then, charged in a suitable vessel.

### (Example 91) Cream for external use

### Prescription:

The cream is composed of (a) tininidazole (2.0 g); (b) glycol monostearate (7 g), stearyl alcohol (7 g), liquid paraffin (5 g), polyoxyethylene cetostearyl ether (3 g); and (c) glycerin (5 g), 1,3-butylene glycol (7 g), sodium carboxymethyl cellulose (0.4 g), Tween 80 (1 g), and purified water (an amount making total 100 g)

### Preparation method:

Component(a) was dissolved in a suitable amount of purified water under heating. Then, to the mixture was added component(c) heated to about 85°C, and the resulting mixture was added to a solution of component(b) dissolved by heating at about 80°C whereby the mixture was emulsified under stirring. The emulsion was cooled to a temperature of about 25°C while continuously stirring, and then, charged in a suitable vessel.

### (Example 92) Cream for external use

### Prescription:

The cream is composed of (a) metronidazole (2.0 g); (b) glycol monostearate (7 g), stearyl alcohol (7 g), liquid paraffin (5 g), polyoxyethylene cetostearyl ether (3 g); and (c) glycerin (5 g), 1,3-butylene glycol (7 g), sodium carboxymethyl cellulose (0.4 g), Tween 80 (1 g), and purified water (an amount making total 100 g)

### Preparation method:

Component(a) was dissolved in a suitable amount of purified water under heating. Then, to the mixture was added component(c) heated to about 85°C, and the resulting mixture was added to a solution of component(b) dissolved by heating at about 80°C whereby the mixture was emulsified under stirring. The emulsion was cooled to a temperature of about 25°C while continuously stirring, and then, charged in a suitable vessel.

### (Example 93) Cream for external use

### Prescription:

The cream is composed of (a) metronidazole (5.0 g); (b) glycol monostearate(7 g), stearyl alcohol (4 g), white petrolatum (3.5 g), isopropyl myristate (3 g), Span 60 (1 g), Tween 60 (0.5 g); and (c) propylene glycol (7 g), glycerin (2 g), Tween 80 (0.1 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 94) Cream for external use

### Prescription:

The cream is composed of (a) tinidazole (3.0 g); (b) glycol monostearate (7 g), stearyl alcohol (4 g), white petrolatum (3.5 g), isopropyl myristate (3 g), Span 60 (1 g), Tween 60 (0.5 g); and (c) propylene glycol (7 g), glycerin (2 g), Tween 80 (0.1 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 75°C whereby the mixture was emulsified under stirring. The emulsion was cooled to a temperature of about 30°C while continuously stirring, and then, charged in a suitable vessel.

### (Example 95) Cream for external use

### Prescription:

The cream is composed of (a) tinidazole (1.5 g); (b) glycol monostearate (7.28 g), sorbitan monostearate (3.12 g), cetanol (7.3 g), white petrolatum (3.5 g), liquid paraffin (9 g), propylparaben (0.05 g); and (c) propylene glycol (6.5 g), sodium lauryl sulfate (1 g), methylparaben (0.05 g), and purified water (an amount making the total amount 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 85°C whereby the mixture was emulsified under stirring. The emulsion was cooled to a temperature of about 30°C while continuously stirring, and then, charged in a suitable vessel.

### (Example 96) Cream for external use

### Prescription:

The cream is composed of (a) tinidazole (2.0 g); (b) glycol monostearate (7.28 g), sorbitan monostearate (3.12 g), cetanol (7.3 g), white petrolatum (3.5 g), liquid paraffin (9 g), propylparaben (0.05 g); and (c) propylene glycol (6.5 g), sodium lauryl sulfate (1 g), methylparaben (0.05 g), and purified water (an amount making total 100 g)

### Preparation method:

Component(a) was dissolved in a suitable amount of purified water under heating. Then, to the mixture was added component(c) heated to about 80°C, and the resulting mixture was added to a solution of component(b) dissolved by heating at about 85°C whereby the mixture was emulsified under stirring. The emulsion was cooled to a temperature of about 30°C while continuously stirring, and then, charged in a suitable vessel.

### (Example 97) Cream for external use

### Prescription:

The cream is composed of (a) metronidazole (2.0 g); (b) glycerol monostearate (6 g), stearyl alcohol (5 g), cetanol (6 g), isopropyl myristate (1 g), Span 60 (1.5 g), Tween 60 (1 g); and (c) sodium carboxymethyl cellulose (0.2 g), propylene glycol (4 g), and purified water (an amount making the total amount 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 98) Cream for external use

### Prescription:

The cream is composed of (a) metronidazole (1.0 g); (b) glycol monostearate (7 g), stearyl alcohol (4 g), white petrolatum (3 g), isopropyl myristate (3 g), Span 60 (1 g), Tween 60 (0.5 g);and (c) propylene glycol (7 g), glycerin (2 g), Tween 80 (0.1 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 99) Cream for external use

### Prescription:

The cream is composed of (a) tinidazole (1.0 g); (b) glycol monostearate (7 g), stearyl alcohol (4 g), white petrolatum (3 g), isopropyl myristate (3 g), Span 60 (1 g), Tween 60 (0.5 g); and (c) propylene glycol (7 g), glycerin (2 g), Tween 80 (0.1 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 100) Cream for external use

### Prescription:

The cream is composed of (a) metronidazole (2.0 g), nofloxacin (0.05 g); (b) glyceryl monostearate (2 g), stearyl alcohol (5 g), white petrolatum (3 g), isopropyl myristate (3 g), Span 60 (1 g), Tween 60 (0.5 g); and (c) propylene glycol (7 g), glycerin (2 g), Tween 80 (0.1 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 101) Cream for external use

### Prescription:

The cream is composed of (a) tinidazole (2.0 g), tranilast (0.2 g); (b) glycol monostearate (4 g), cetanol (4 g), stearyl alcohol (3 g), polyoxyethylene cetyl alcohol (2 g), isopropyl myristate (3 g), Span 60 (1 g), Tween 60 (0.5 g); and (c) propylene glycol (5 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 102) Cream for external use

### Prescription:

The cream is composed of (a) tinidazole (2.0 g), ketoprofen (0.5 g); (b) glycol monostearate (4 g), cetanol (4 g), stearyl alcohol (3 g), polyoxyethylene cetyl alcohol (2 g), isopropyl myristate (3 g), Span 60 (1 g), Tween 60 (0.5 g); and (c) propylene glycol (5 g), and purified water (an amount making total 100 g)

### Preparation method:

Component(b) was dissolved under heating and adjusted to about 75°C. To the solution was added under stirring a material in which Component(c) had been dissolved and adjusted to about 75°C, followed by the addition of component(a) and stirring. The mixture was cooled to a temperature of about 25°C while continuously stirring, and then, charged in a suitable vessel.

### (Example 103) Cream for external use

### Prescription:

The cream is composed (a) metronidazole (2.5 g), procaine hydrochloride (0.2 g); (b) glycerol monostearate (2 g), stearyl alcohol (5 g), white petrolatum (3 g), isopropyl myristate (3 g), Span 60 (1 g), Tween 60 (0.5 g); and (c) propylene glycol (7 g), glycerin (2 g), Tween 80 (0.1 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 104) Cream for external use

### Prescription:

The cream is composed of (a) tinidazole (3.0 g), tamoxifen citrate (0.05 g); (b) glycol monostearate (4 g), cetanol (4 g), stearyl alcohol (3 g), polyoxyethylene cetyl alcohol (2 g), isopropyl myristate (3 g), Span 60 (1 g), Tween 60 (0.5 g); and (c) propylene glycol (5 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 105) Cream for external use

### Prescription:

The cream is composed of (a) tinidazole (2.0 g), carpronium chloride (0.5 g); (b) stearic acid (0.5 g), glycol monostearate (12 g), stearyl alcohol (7 g), white petrolatum (2 g), liquid paraffin (5 g); and (c) polyethylene glycol (5 g), 1,3-butylene glycol (5 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 106) Cream for external use

### Prescription:

The cream is composed of (a) tinidazole (2.0 g), extract of calves blood (0.5 g); (b) stearic acid (0.5 g), glycol monostearate (12 g), stearyl alcohol (7 g), white petrolatum (2 g), liquid paraffin (5 g); and (c) polyethylene glycol (5 g), 1,3-butylene glycol (5 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 107) Cream for external use

### Prescription:

The cream is composed of (a) metronidazole (0.5 g); (b) stearic acid (0.5 g), glycol monostearate (10 g), cetanol (5 g), white petrolatum (3 g); and (c) propylene glycol (7 g), sodium lauryl sulfate (1 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 108) Cream for external use

### Prescription:

The cream is composed of (a) tinidazole (0.5 g); (b) stearic acid (0.5 g), glycol monostearate (10 g), cetanol (5 g), white petrolatum (3 g); and (c) propylene glycol (7 g), sodium lauryl sulfate (1 g), and purified water (an amount making total 100 g)

### Preparation method:

Component(b) was dissolved under heating and adjusted to about 75°C. To the solution was added under stirring a material in which Component(c) had been dissolved and adjusted to about 75°C, followed by the addition of component(a) and stirring. The mixture was cooled to a temperature of about 25°C while continuously stirring, and then, charged in a suitable vessel.

### (Example 109) Cream for external use

### Prescription:

The cream is composed of (a) metronidazole (5 g); (b) stearic acid (0.5 g), glycol monostearate (10 g), cetanol (5 g), white petrolatum (3 g); and (c) propylene glycol (7 g), sodium lauryl sulfate (1 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 110) Cream for external use

### Prescription:

The cream is composed of (a) tinidazole (5 g); (b) stearic acid (0.5 g), glycol monostearate (10 g), cetanol (5 g), white petrolatum (3 g); and (c) propylene glycol (7 g), sodium lauryl sulfate (1 g), and purified water (an amount making total 100 g)

### Preparation method:

Component(b) was dissolved under heating and adjusted to about 75°C. To the solution was added under stirring a material in which component(c) had been dissolved and adjusted to about 75°C, followed by the addition of component(a) and stirring. The mixture was cooled to a temperature of about 25°C while continuously stirring, and then, charged in a suitable vessel.

### (Example 111) Ointment

### Prescription:

The ointment is composed of (a) metronidazole (2 g); (b) stearic acid (2 g), glycol monostearate (12 g), polyoxyethylene glycol monostearate (3 g), polyoxyethylene cetyl/stearyl ether (12E.O.)(1 g), polyoxyethylene cetyl/stearyl ether (20E.O.)(1 g), cetanol (2 g), liquid paraffin (8 g); and (c) 1,3-butylene glycol (7 g), glycerin (5 g), and purified water (an amount making total 100 g)

### Preparation method:

Component(b) was dissolved and adjusted to about 75°C. To the solution was added under stirring a material in which Component(c) had been dissolved and adjusted to about 75°C, followed by the addition of component(a). The mixture was cooled to a temperature of about 25°C while continuously stirring, and then, charged in a suitable vessel.

### (Example 112) External preparation

### Prescription:

The preparation is composed of (a) metronidazole (2 g), ketoconazole (0.2 g); (b) glyceryl monostearate (7.5 g), sorbitan monostearate (3 g), stearyl alcohol (7 g), liquid paraffin (8 g), white petrolatum (5 g), span 80 (1 g); and (c) propylene glycol (5 g), 1,3-butylene glycol (3 g), Tween 80 (1 g), and purified water (an amount making total 100 g)

### Preparation method:

Component(b) was dissolved and adjusted to about 75°C. To the solution was added under stirring a material in which component(c) had been dissolved and adjusted to about 75°C, followed by the addition of component(a). The mixture was cooled to a temperature of about 25°C while continuously stirring, and then, charged in a suitable vessel.

### (Example 113) External preparation

### Prescription:

The preparation is composed of (a) metronidazole (2 g); (b) glyceryl monostearate (7.5 g), sorbitan monostearate (3 g), stearyl alcohol (7 g), liquid paraffin (8 g), white petrolatum (5 g), span 80 (1 g); and (c) propylene glycol (5 g), 1,3-butylene glycol (3 g), Tween 80 (1 g), and purified water (an amount making total 100 g)

### Preparation method:

Compornent(b) was dissolved and adjusted to about 75°C. To the solution was added under stirring a material in which component(c) had been dissolved and adjusted to about 75°C, followed by the addition of component(a). The mixture was cooled to a temperature of about 25°C while continuously stirring, and then, charged in a suitable vessel.

### (Example 114) External preparation

### Prescription:

The preparation is composed of (a) tinidazole (2 g), isoconazole nitrate (0.2 g); (b) glycol monostearate (7.5 g), sorbitan monostearate (3 g), stearyl alcohol (7 g), liquid paraffin (8 g), white petrolatum (5 g), span 80 (1 g); and (c) propylene glycol (5 g), 1,3-butylene glycol (3 g), Tween 80 (1 g), and purified water (an amount making total 100 g)

### Preparation method:

Component(b) was dissolved and adjusted to about 75°C. To the solution was added under stirring a material in which component(c) had been dissolved and adjusted to about 75°C, followed by the addition of component(a). The mixture was cooled to a temperature of about 25°C while continuously stirring, and then, charged in a suitable vessel.

### (Example 115) External preparation

### Prescription:

The preparation is composed of (a) tinidazole (2 g); (b) glyceryl monostearate (7.5 g), sorbitan monostearate (3 g), stearyl alcohol (7 g), liquid paraffin (8 g), white petrolatum (5 g), span 80 (1 g); and (c) propylene glycol (5 g), 1,3-butylene glycol (3 g), Tween 80 (1 g), purified water (an amount making total 100 g)

### Preparation method:

Component(b) was dissolved and adjusted to about 75°C. To the solution was added under stirring a material in which component(c) had been dissolved and adjusted to about 75°C, followed by the addition of component(a). The mixture was cooled to a temperature of about 25°C while continuously stirring, and then, charged in a suitable vessel.

### (Example 116) Shampoo

### Prescription:

The shampoo is composed of tinidazole (1.5 g), polyglyceryl monolaurate (4 g), sodium polyoxyethylene lauryl ether sulfate (7 g), lauryl-dimethylaminoacetate betaine (2.5 g), coconut fatty acid diethanol amide (4 g), polyethylene glycol (5 g), 1,3-butylene glycol (3 g), citric acid (a suitable amount), and purified water (an amount making total 100 g)

### Preparation method:

Metronidazole was added to a mixture of suitable amounts of polyethylene glycol and purified water, and the mixture was melted by heating. In other vessel were weighed suitable amounts of polyglyceryl monolaurate, sodium polyoxyethylene lauryl ether sulfate, lauryl dimethylacetate betaine, coconut fatty acid diethanol amide, polyethylene glycol, 1,3-butylene glycol and purified water, and the mixture was heated to about 70°C under stirring and added to a mixture of tinidazole, polyethylene glycol and purified water. A pH of the mixture was adjusted to about 6.5 with citric acid. The resulting mixture was cooled until a temperature thereof became about 25°C under stirring.

### (Example 117) Rinse

### Prescription:

The rinse is composed of (a) metronidazole (2 g); (b) isopropyl myristate (1 g), butyl myristate (1 g), silicone oil (2 g), liquid paraffin (1 g), a hydrochloric acid solution of N-[alkyl(12,14)oxy-2-hydroxypropyl]-L-alginic acid (2 g); and (c) lactic acid (0.05 g), polyethylene glycol (6 g), and purified water (an amount making total 100 g)

### Preparation method:

Component(b) was heated to about 80°C, and to the mixture was added a material in which component(a) had been added to component(c) while stirring to melt the material under heating and adjusted to about 80°C. The mixture was cooled to about 25°C under stirring and charged in a suitable vessel.

### (Example 118) Rinse

### Prescription:

The rinse is composed of (a) tinidazole (2 g); (b) isopropyl myristate (1 g), butyl myristate (1 g), silicone oil (2 g), liquid paraffin (1 g), a hydrochloric acid solution of N-[alkyl(12,14)oxy-2-hydroxypropyl]-L-alginic acid (2 g); and (c) lactic acid (0.05 g), polyethylene glycol (6 g), and purified water (an amount making total 100 g)

### Preparation method:

Component(b) was heated to about 80°C, and to the mixture was added a material in which component(a) had been added to component(c) while stirring to melt the material under heating and adjusted to about 80°C. The mixture was cooled to about 25°C under stirring and charged in a suitable vessel.

### (Example 119) Soap

### Prescription:

The soap is composed of metronidazole (3 g), monoglycerol laurate (75 g), sodium monoglyceryl fatty acid sulfate (7 g), stearyl alcohol (8 g), silicone oil (1 g), glycerin (3 g), polyethylene glycol (5 g), sodium carboxymethyl cellulose (0.4 g), purified water (an amount making total 100 g), and perfume (a suitable amount)

### Preparation method:

Compositions except perfume were melted by heating under stirring. Cooling was initiated and perfume was added before the melt was solidified. The solid material was dried in a dark place with a sufficient time to obtain soap.

### (Example 120) Soap

### Prescription:

The soap is composed of (a) tinidazole (2 g); (b) monoglycerol laurate (75 g), sodium monoglyceryl fatty acid sulfate (7 g), stearyl alcohol (8 g), silicone oil (1 g), glycerin (3 g), polyethylene glycol (5 g), sodium carboxymethyl cellulose (0.4 g), purified water (an amount making total 100 g), and perfume (a suitable amount)

### Preparation method:

Compositions except perfume were melted by heating under stirring. Cooling was initiated and perfume was added before the melt was solidified. The solid material was dried in a dark place with a sufficient time to obtain soap.

### (Example 121) Face lotion

### Prescription:

The lotion is composed of (a) metronidazole (1 g); (b) propylene glycol (3 g), polyethylene glycol (5 g), sodium carboxymethyl cellulose (0.4 g);(c) polyoxethylen oleyl cetyl ether (1 g), jojoba oil (0.5 g); (d) perfume (a suitable amount), ethanol (8 g); and (e) purified water (an amount making total 100 g)

### Preparation method:

Component(b) was added to (e) and the mixture was melted by heating. To the above mixture was added component(a) and the resulting mixture was melted and cooled to room temperature. Further, to the above mixture was added a material in which component(c) had been dissolved and dispersed in component(d) and the resulting mixture was stirred and homogenized.

### (Example 122) Face lotion

### Prescription:

The lotion is composed of (a) tinidazole (0.5 g); (b) propylene glycol (3 g), polyethylene glycol (5 g), sodium carboxymethyl cellulose (0.4 g); (c) polyoxethylene oleyl cetyl ether (1 g), jojoba oil (0.5 g); (d) perfume (a suitable amount), ethanol (8 g); and (e) purified water (an amount making total 100 g)

### Preparation method:

Component(b) was added to component(e) and the mixture was melted by heating. To the above mixture was added component(a) and the resulting mixture was melted and cooled to room temperature. Further, to the above mixture was added a material in which component(c) had been dissolved and dispersed in component(d) and the resulting mixture was stirred and homogenized.

### (Example 123) Gel

### Prescription:

The gel is composed of tinidazole (1 g), polyethylene glycol (8 g), carboxyvinyl polymer (0.5 g), methyl cellulose (0.2 g), propylene glycol (5 g), glycerin (2 g), polyoxyethylene oleyl cetyl ether (1 g), isopropanol (5 g), sodium hydroxide (a suitable amount), an purified water (an amount making total 100 g) Preparation method:

Polyethylene glycol was added to purified water and melted, and after tinidazole was added to the mixture, the mixture was dissolved under heating. The solution was cooled to about 50°C, and to the solution were added under stirring a material in which polyoxyethylene cetyl ether had been added to propylene glycol and glycerin heated to about 50°C. Further, under continuous stirring, sodium hydroxide was added to the above mixture and a pH thereof was adjusted to about 6.8. After cooling the resulting mixture to about 40°C, isopropanol was added thereto, and the resulting mixture was cooled to about 25°C, and charged in a suitable vessel.

### (Example 124) Cream

### Prescription:

The cream is composed of (a) secnidazole (2 g); (b) glycol monostearate (10 g), polyoxyethylene glycol monostearate (3 g), polyoxyethylene cetyl/stearyl ether (2 g), cetanol(4 g), beeswax (1 g), octyl dodecyl myristate (7 g), isopropyl myristate (2 g); (c) polyethylene glycol (3 g), carboxyvinyl polymer (0.2 g), purified water (an amount making total 100 g); and (d) an aqueous sodium hydroxide solution (a suitable amount)

### Preparation method:

Component(b) was heated to about 75°C, then, to component(b) was added component(c) heated to about 75°C under stirring, followed by the addition of component(a) under stirring. Thereafter, a pH of the mixture was adjusted to about 6.8 with component(d). Thereafter, the resulting mixture was cooled to a temperature of about 25°C, and the resulting cream was charged in a suitable vessel.

### (Example 125) Cream

### Prescription:

The cream is composed of (a) Panidazole (2 g); (b) glycol monostearate (10 g), polyoxyethylene glycol monostearate (3 g), polyoxyethylene cetyl/stearyl ether (2 g), cetanol (4 g), beeswax (1 g), octyldodecyl myristate (7 g), isopropyl myristate (2 g); (c) polyethylene glycol (3 g), carboxyvinyl polymer (0.2 g), purified water (an amount making total 100 g); and (d) an aqueous sodium hydroxide solution (a suitable amount)

### Preparation method:

Component(b) was heated to about 75°C, then, to component(b) was added component(c) heated to about 75°C under stirring, followed by the addition of component(a) under stirring. Thereafter, a pH of the mixture was adjusted to about 6.8 with (d). Thereafter, the resulting mixture was cooled to a temperature of about 25°C, and the resulting cream was charged in a suitable vessel.

### (Example 126) Cream

### Prescription:

The cream is composed of (a) dimetridazole (2 g); (b) glycol monostearate (10 g), polyoxyethylene glycol monostearate (3 g), polyoxyethylene cetyl/stearyl ether (2 g), cetanol (4 g), beeswax (1 g), octyl dodecyl myristate (7 g), isopropyl myristate (2 g); (c) polyethylene glycol (3 g), carboxyvinyl polymer (0.2 g), purified water (an amount making total 100 g); (d) an aqueous sodium hydroxide solution (a suitable amount)

### Preparation method:

Component(b) was heated to about 75°C, then, to component(b) was added component(c) heated to about 75°C under stirring, followed by the addition of component(a) under stirring. Thereafter, a pH of the mixture was adjusted to about 6.8 with component(d). Thereafter, the resulting mixture was cooled to a temperature of about 25°C, and the resulting cream was charged in a suitable vessel.

### (Example 127) Cream

### Prescription:

The cream is composed of (a) ronidazole(2 g); (b) glycol monostearate (10 g), polyoxyethylene glycol monostearate (3 g), polyoxyethylene cetostearyl ether (2 g), cetanol (4 g), beeswax (1 g), octyl dodecyl myristate (7 g), isopropyl myristate (2 g); (c) polyethylene glycol (3 g), carboxyvinyl polymer (0.2 g), purified water (an amount making total 100 g); and (d) an aqueous sodium hydroxide solution (a suitable amount)

### Preparation method:

(b) was heated to about 75°C, then, to (b) was added (c) heated to about 75°C under stirring, followed by the addition of (a) under stirring. Thereafter, a pH of the mixture was adjusted to about 6.8 with (d). Thereafter, the resulting mixture was cooled to a temperature of about 25°C, and the resulting cream was charged in a suitable vessel.

### (Example 128) Cream

### Prescription:

The cream is composed of (a) ipronidazole (2 g); (b) glycol monostearate (10 g), polyoxyethylene glycol monostearate (3 g), polyoxyethylene cetyl/stearyl ether (2 g), cetanol (4 g), beeswax (1 g), octyl dodecyl myristate (7 g), isopropyl myristate (2 g); (c) polyethylene glycol (3 g), carboxyvinyl polymer (0.2 g), purified water (an amount making the total amount 100 g); (d) an aqueous sodium hydroxide solution (a suitable amount)

### Preparation method:

Component(b) was heated to about 75°C, then, to component(b) was added component(c) heated to about 75°C under stirring, followed by the addition of (a) under stirring. Thereafter, a pH of the mixture was adjusted to about 6.8 with component(d). Thereafter, the resulting mixture was cooled to a temperature of about 25°C, and the resulting cream was charged in a suitable vessel.

### (Example 129) Cream

### Prescription:

The cream is composed of (a) ornidazole (2 g); (b) glycol monostearate (10 g), polyoxyethylene glycol monostearate (3 g), polyoxyethylene cetyl/stearyl ether (2 g), cetanol (4 g), beeswax (1 g), octyl dodecyl myristate (7 g), isopropyl myristate (2 g); and and (c) polyethylene glycol (3 g), carboxyvinyl polymer (0.2 g), purified water (an amount making total 100 g); and (d) an aqueous sodium hydroxide solution (a suitable amount)

### Preparation method:

Component(b) was heated to about 75°C, then, to component(b) was added component(c) heated to about 75°C under stirring, followed by the addition of component(a) under stirring. Thereafter, a pH of the mixture was adjusted to about 6.8 with component(d). Thereafter, the resulting mixture was cooled to a temperature of about 25°C, and the resulting cream was charged in a suitable vessel.

### (Example 130) Cream for external use

### Prescription:

The cream is composed of (a) metronidazole (5 g); (b) glycol monostearate (10 g), cetanol (7 g), liquid praffin (9 g), white petrolatum (2.5 g); and (c) urea (2 g), polyethylene glycol (7 g), Tween 80 (1 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### (Example 131) Cream for external use

### Prescription:

The cream is composed of (a) tinidazole (5 g); (b) glycol monostearate (10 g), cetanol (7 g), liquid praffin (9 g), white petrolatum (2.5 g); and (c) urea (2 g), polyethylene glycol (7 g), Tween 80 (1 g), and purified water (an amount making total 100 g)

### Preparation method:

Component(b) was dissolved and adjusted to about 85°C. To the solution was added under stirring a material in which component(c) had been dissolved and adjusted to about 85°C, followed by the addition of component(a). The mixture was cooled to a temperature of about 25°C while continuously stirring, and then, charged in a suitable vessel.

### [Test Examples]

### (Test Example 1) Treatment of Atopic Dermatitis

The therapeutic effect of the ointment produced in the above Example 1 was examined by applying to patients with atopic dermatitis.

The ointment was applied to the target patients indicated below.

Target patient A: 1-year-old male infant suffering from atopic dermatitis

Target patient B: 2-year-old male infant suffering from atopic dermatitis

Target patient C: 40-year-old female suffering from atopic dermatitis

Target patient D: 60-year-old female suffering from atopic dermatitis

Target patient E: 27-year-old male suffering from atopic dermatitis

For target patients A and B, the ointment for external use produced in Example 1 was applied twice a day for 4 consecutive weeks to the face appearing prominent atopic dermatitis, and the status of inflammation was observed.

In addition, for target patients C, D and E, the ointment for external use produced in the Example 1 was applied twice a day for 4 consecutive weeks to affected areas of prominent atopic dermatitis extending from the lower leg to the ankle, and the status of inflammation was observed.

Therapeutic effects were evaluated by scoring rash, eczema and other dermatitis symptoms at the start of treatment along with the status of healing over 3 days, 1 week, 2 weeks, 3 weeks and 4 weeks later. In addition, the presence of itchiness of the skin surface and skin condition were evaluated after 4 weeks.

Furthermore, evaluation scores were determined in the manner indicated below.
5: Prominent rash, eczema and other dermatitis symptoms, extreme itchiness, subconscious scratching of the skin surface and the presence of resulting scratches.
4: Prominent rash, eczema and other dermatitis symptoms, itchiness but not to the extend of grade 5.
3: Rash, eczema and other dermatitis symptoms able to be confirmed and only bothersome itchiness.
2: Rash, eczema and other dermatitis symptoms only able to be confirmed slightly, and not that much different from normal skin.
1: Absence of rash, eczema and other dermatitis symptoms, no itchiness and appearance of normal skin.

Those results are shown in Table 2 below.

**[Table 2]**

| Patient | Start of treatment | After 3 days | After 1 week | After 2 weeks | After 3 weeks | After 4 weeks | Overall evaluation | |
|---|---|---|---|---|---|---|---|---|
| | | | | | | | Itchiness | Skin surface |
| A | 5 | 5 | 3 | 1 | 1 | 1 | none | Normal |
| B | 5 | 4 | 3 | 2 | 1 | 1 | none | Normal |
| C | 5 | 5 | 4 | 1 | 1 | 1 | none | Normal |
| D | 5 | 5 | 4 | 3 | 2 | 1 | none | Normal |
| E | 3 | 3 | 1 | 1 | 1 | 1 | none | Normal |

As is clear from the above results, the external preparation of the present invention was observed to demonstrate improvement of dermatitis symptoms in 3-7 days after the start of application during treatment of atopic dermatitis, and the skin was no different from completely normal skin 3-4 weeks after the start of application. Furthermore, there was no irritation of the skin by the preparation during application. In addition, there were also no adverse side effects such as rebound observed, which are observed with steroid-based external preparations, even after administration was discontinued.

### (Test Example 2) Treatment of Atopic Dermatitis

The therapeutic effect of the cream produced in the above Example 4 was examined by applying to patients with atopic dermatitis. The cream was applied to the target patients indicated below.
Target patient F: 2-year-old male infant suffering from atopic dermatitis
Target patient G: 8-year-old male infant suffering from atopic dermatitis
Target patient H: 50-year-old female suffering from atopic dermatitis
Target patient I: 40-year-old female suffering from atopic dermatitis
Target patient J: 27-year-old male suffering from atopic dermatitis

For target patients F, G, H, I and J, the cream for external use produced in the Example 4 was applied twice a day for 4 consecutive weeks to the face that exhibited prominent atopic dermatitis, and the status of inflammation was observed.

Therapeutic effects were evaluated by scoring rash, eczema and other dermatitis symptoms at the start of treatment along with the status of healing over 3 days, 1 week, 2 weeks, 3 weeks and 4 weeks later in the same manner as the Test Example 1. In addition, the presence of itchiness of the skin surface and skin condition were evaluated after 4 weeks. Furthermore, the evaluation scores in the above Test Example 1 were used for evaluation.

Those results are shown in Table 3 below.

**[Table 3]**

| Patient | Start of treatment | After 3 days | After 1 week | After 2 weeks | After 3 weeks | After 4 | Overall evaluation | |
|---|---|---|---|---|---|---|---|---|
| | | | | | | | Itchiness | Skin surface |
| F | 5 | 4 | 2 | 1 | 1 | 1 | none | Normal |
| G | 5 | 4 | 3 | 2 | 1 | 1 | none | Normal |
| H | 5 | 5 | 3 | 1 | 1 | 1 | none | Normal |
| I | 3 | 2 | 2 | 1 | 1 | 1 | none | Normal |
| J | 3 | 2 | 1 | 1 | 1 | 1 | none | Normal |

As is clear from the above results, the external preparation of the present invention was observed to demonstrate improvement of dermatitis symptoms in 3-7 days after the start of application during treatment of atopic dermatitis, and the skin was no different from complete normal skin 3-4 weeks after the start of application. Furthermore, there was no irritation of the skin by the preparation during application. In addition, there were also no adverse side effects such as rebound observed, which are observed with steroid-based external preparations, even after administration was discontinued.

### (Test Example 3) Treatment of Atopic Dermatitis

The therapeutic effect of the ointment produced in the above Example 11 was examined by applying to patients with atopic dermatitis. The cream was applied to the target patients indicated below.
Target patient K: 1-year-old male infant suffering from atopic dermatitis
Target patient L: 2-year-old male infant suffering from atopic dermatitis
Target patient M: 35-year-old female suffering from atopic dermatitis
Target patient N: 54-year-old female suffering from atopic dermatitis
Target patient O: 27-year-old male suffering from atopic dermatitis

For target patients K and L, the ointment for external use produced in the Example 11 was applied twice a day for 4 consecutive weeks to the face that exhibited prominent atopic dermatitis, and the status of inflammation was observed.

In addition, for target patients M, N and O, the ointment for external use produced in the Example 11 was applied twice a day for 4 consecutive weeks to affected areas of prominent atopic dermatitis extending from the lower leg to the ankle, and the status of inflammation was observed.

Therapeutic effects were evaluated by scoring rash, eczema and other dermatitis symptoms at the start of treatment along with the status of healing over 3 days, 1 week, 2 weeks, 3 weeks and 4 weeks later in the same manner as the above Test Example 1. In addition, the presence of itchiness of the skin surface and skin condition were evaluated after 4 weeks. Furthermore, the evaluation scores in the above Test Example 1 were used for evaluation.

Those results are shown in Table 4 below.

**[Table 4]**

| Patient | Start of treatment | After 3 days | After 1 week | After 2 weeks | After 3 weeks | After 4 weeks | Overall evaluation | |
|---|---|---|---|---|---|---|---|---|
| | | | | | | | Itchiness | Skin surface |
| K | 5 | 5 | 3 | 1 | 1 | 1 | none | Normal |
| L | 5 | 4 | 3 | 2 | 1 | 1 | none | Normal |
| M | 5 | 5 | 4 | 1 | 1 | 1 | none | Normal |
| N | 5 | 5 | 4 | 3 | 2 | 1 | none | Normal |
| O | 4 | 3 | 1 | 1 | 1 | 1 | none | Normal |

As is clear from the above results, the external preparation of the present invention was observed to demonstrate improvement of dermatitis symptoms in 3-7 days after the start of application during treatment of atopic dermatitis, and the skin was no different from complete normal skin 3-4 weeks after the start of application. Furthermore, there was no irritation of the skin by the preparation during application. In addition, there were also no adverse side effects such as rebound observed, which are observed with steroid-based external preparations, even after administration was discontinued.

### (Test Example 4) Treatment of Atopic Dermatitis

The therapeutic effect of the cream produced in the above Example 14 was examined by applying to patients with atopic dermatitis.

The cream was applied to the target patients indicated below.
Target patient P: 2-year-old male infant suffering from atopic dermatitis
Target patient Q: 6-year-old male infant suffering from atopic dermatitis
Target patient R: 53-year-old female suffering from atopic dermatitis
Target patient S: 58-year-old female suffering from atopic dermatitis
Target patient T: 35-year-old male suffering from atopic dermatitis
For target patients P, Q, R, S and T, the cream for external use produced in the Example 14 was applied twice a day for 4 consecutive weeks to the face that exhibited prominent atopic dermatitis, and the status of inflammation was observed.

Therapeutic effects were evaluated by scoring rash, eczema and other dermatitis symptoms at the start of treatment along with the status of healing over 3 days, 1 week, 2 weeks, 3 weeks and 4 weeks later in the same manner as the Test Example 1. In addition, the presence of itchiness of the skin surface and skin condition were evaluated after 4 weeks. Furthermore, the evaluation scores in the above Test Example 1 were used for evaluation.

Those results are shown in Table 5 below.

**[Table 5]**

| Patient | Start of treatment | After 3 days days | After 1 week 1 | After 2 weeks | After 3 weeks | After 4 weeks | Overall evaluation | |
|---|---|---|---|---|---|---|---|---|
| | | | | | | | Itchiness | Skin surface |
| P | 5 | 4 | 3 | 1 | 1 | 1 | none | Normal |
| Q | 5 | 4 | 3 | 3 | 1 | 1 | none | Normal |
| R | 5 | 5 | 3 | 2 | 1 | 1 | none | Normal |
| S | 5 | 4 | 2 | 1 | 1 | 1 | none | Normal |
| T | 5 | 4 | 2 | 1 | 1 | 1 | none | Normal |

As is clear from the above results, the external preparation of the present invention was observed to demonstrate improvement of dermatitis symptoms in 3-7 days after the start of application during treatment of atopic dermatitis, and the skin was no different from complete normal skin 3-4 weeks after the start of application. Furthermore, there was no irritation of the skin by the preparation during application. In addition, there were also no adverse side effects such as rebound observed, which are observed with steroid-based external preparations, even after administration was discontinued.

### (Text Example 5) Treatment of Atopic Dermatitis

The therapeutic effect of the cream produced in the above Example 21 was examined by applying to patients with atopic dermatitis.

The cream was applied to the target patients indicated below.
Target patient U: 40-year-old female suffering from atopic dermatitis
Target patient V: 38-year-old female suffering from atopic dermatitis
Target patient W: 55-year-old female suffering from atopic dermatitis

### Method:

For target patients V and W, the cream for external use produced in Example 21 was applied twice a day for 4 consecutive weeks to the face that exhibited prominent atopic dermatitis, and the status of inflammation was observed.

For target patient U, a cream for external use containing only metronidazole was applied twice a day for 4 consecutive weeks to the face that exhibited prominent atopic dermatitis, and the status of inflammation was observed.

Therapeutic effects were evaluated by scoring rash, eczema and other dermatitis symptoms at the start of treatment along with the status of healing over 3 days, 1 week, 2 weeks, 3 weeks and 4 weeks later in the same manner as the Test Example 1. In addition, the presence of itchiness of the skin surface and skin condition were evaluated after 4 weeks.

Furthermore, evaluation scores were determined in the manner indicated below.

### Skin Condition:

5: Prominent rash, eczema and other dermatitis symptoms while also suffering from pain.
4: Prominent rash, eczema and other dermatitis symptoms, but not to the extent of grade 5.
3: Rash, eczema and other dermatitis symptoms able to be confirmed, but not to the extent of grade 4.
2: Rash, eczema and other dermatitis symptoms only able to be confirmed slightly, and not that much different from normal skin.
1: Absence of rash, eczema and other dermatitis symptoms, and appearance of normal skin.

### Skin Itchiness:

3: Prominent itchiness, skin is scratched subconsciously.
2: Occasional itchiness, and scratching able to be controlled.
1: No itchiness whatsoever.

Those results are summarized in Table 6 below.

In Table 6, S1 refers to the score for skin condition, while S2 refers to the score for itchiness.

**[Table 6]**

| Patient | Start of treatment | 3 days after | 1 week after | 2 weeks after | 3 weeks after | 4 weeks after | Overall evaluation |
|---|---|---|---|---|---|---|---|
| | S1:S2 | S1:S2 | S1:S2 | S1:S2 | S1:S2 | S1:S2 | S1:S2. |
| U | 5:3 | 5:3 | 3:3 | 2:2 | 2:2 | 1:1 | 1:1 |
| V | 5:3 | 4:2 | 3:1 | 2:1 | 2:1 | 1:1 | 1:1 |
| W | 5:3 | 4:1 | 3:1 | 1:1 | 1:1 | - | 1:1 |

As indicated above, although patients U, V and W exhibited skin conditions no different from those of healthy individuals after 4 weeks, patients V and W, who applied the cream for external use of Example 21, which is a compound preparation, no longer exhibited itchiness sooner than patient U, who applied an external preparation containing metronidazole alone. In addition, improvement of the skin was also faster in patients V and W. Furthermore, since symptoms of atopic dermatitis were no longer able to be confirmed for patient W in week 3, application was discontinued in week 3 at that patient's request.

### (Text Example 6) Treatment of Atopic Dermatitis

The therapeutic effect of the creams produced in the above Examples 22 and 51 were examined by applying to patients with atopic dermatitis.

### Test Method:

The cream for external use produced in the Example 22 was applied twice a day for 4 consecutive weeks to the right arm of target patient U of Test Example 5 having atopic dermatitis, and the status of inflammation was observed. In addition, the cream for external use produced in Example 51 was applied twice a day for 4 consecutive weeks to the left arm of target patient U of the Test Example 5 having atopic dermatitis, and the status of inflammation was observed.

Therapeutic effects were evaluated by scoring rash, eczema and other dermatitis symptoms at the start of treatment along with the status of healing over 3 days, 1 week, 2 weeks, 3 weeks and 4 weeks later. In addition, the presence of itchiness of the skin surface and skin condition were evaluated after 4 weeks.

Furthermore, the same evaluation scores as used in Test Example 5 were used for evaluation.

Those results are summarized in Table 7.

In Table 7, S1 refers to the score for skin condition, while S2 refers to the score for itchiness.

**[Table 7]**

| Target | Start of treatment days | After 3 | After 1 week | After 2 weeks | After 3 weeks | After 4 weeks | Overall evaluation |
|---|---|---|---|---|---|---|---|
| | S1:S2 | S1:S2 | S1:S2 | S1:S2 | S1:S2 | S1:S2 | S1:S2 |
| Right arm | 4:3 | 3:1 | 2:1 | 2:1 | 1:1 | 1:1 | 1:1 |
| Left arm | 4:3 | 4:3 | 3:2 | 2:2 | 2:1 | 1:1 | 1:1 |

As is indicated above, the skin conditions of the left and right arms exhibiting the same symptoms in the same patient U improved after 4 weeks. Itchiness disappeared more quickly and the skin improved more rapidly on the right arm, to which was applied the cream for external use produced in Example 22 in the form of a compound preparation, than on the left arm, to which was applied the cream for external use of Example 51 in the form of an external preparation of metronidazole alone.

### (Test Example 7) Treatment of Atopic Dermatitis

The therapeutic effect of the cream produced in the above Example 23 was examined by applying to patients with atopic dermatitis.

The cream was applied to the target patients indicated below.
Target patient X: 30-year-old female suffering from atopic dermatitis
Target patient Y: 28-year-old female suffering from atopic dermatitis
Target patient Z: 26-year-old female suffering from atopic dermatitis
Target patient a: 50-year-old female suffering from atopic dermatitis on the head

### Method:

For target patient X, the cream for external use containing only tinidazole was applied twice a day for 4 consecutive weeks to the face that exhibited prominent atopic dermatitis, and the status of inflammation was observed.

For target patients Y and Z, a cream for external use produced in the Example 23 was applied twice a day for 4 consecutive weeks to the face that exhibited prominent atopic dermatitis, and the status of inflammation was observed.

For target patient a, the gel produced in the Example 25 was applied 2-3 times a day until symptoms improved, and those effects were observed.

Therapeutic effects were evaluated by scoring rash, eczema and other dermatitis symptoms at the start of treatment along with the status of healing over 3 days, 1 week, 2 weeks, 3 weeks and 4 weeks later in the same manner as the Test Example 1. In addition, the presence of itchiness of the skin surface and skin condition were evaluated after 4 weeks.

Furthermore, the same evaluation scores as used in the Test Example 5 were used for evaluation.

Those results are summarized in Table 8.

In Table 8, S1 refers to the score for skin condition, while S2 refers to the score for itchiness.

**[Table 8]**

| Patient | Start of treatment | After 3 days | After 1 week | After 2 weeks | After 3 weeks | After weeks | 4 Overall evaluation |
|---|---|---|---|---|---|---|---|
| | S1 :S2 | S1:S2 | S1:S2 | S1:S2 | S1:S2 | S1:S2 | S1:S2 |
| X | 5:3 | 5:3 | 4:2 | 3:2 | 2:1 | 2:1 | 2:1 |
| Y | 5:3 | 4:1 | 3:1 | 2:1 | 1:1 | 1:1 | 1:1 |
| Z | 4:3 | 3:1 | 2:1 | 1:1 | -:- | -:- | 1:1 |
| a | 4:3 | 3:1 | 3:1 | 2:1 | 2:1 | 1:1 | 1:1 |

As is indicated above, although target patients X, Y, Z and a exhibited skin conditions not different from those of healthy individuals after 4 weeks, itchiness disappeared more quickly and the skin improved more rapidly in patients Y, Z and a, who applied compound preparations in form of the cream for external use of the Example 23 or the gel of the Example 25, than patient Y, who applied an external preparation containing tinidazole alone. Furthermore, since symptoms of atopic dermatitis were unable to be confirmed for patient Z in week 2, application was discontinued in week 2 at that patient's request.

### (Test Example 8) Treatment of Atopic Dermatitis

The therapeutic effect of an external preparation of the present invention was examined by applying to an actual patient with atopic dermatitis. The external preparation was applied to the target patients indicated below.
Target patient b: 40-year-old male suffering from atopic dermatitis

### Method:

The cream for external use of the Example 11 was applied twice a day for 4 consecutive weeks to the left arm of target patient b having atopic dermatitis, and the status of inflammation was observed.

In addition, the cream for external use of the Example 24 was applied twice a day for 4 consecutive weeks to the right arm of the same target patient b having atopic dermatitis, and the status of inflammation was observed.

Therapeutic effects were evaluated by scoring rash, eczema and other dermatitis symptoms at the start of treatment along with the status of healing over 3 days, 1 week, 2 weeks, 3 weeks and 4 weeks later. In addition, the presence of itchiness of the skin surface and skin condition were evaluated after 4 weeks.

Furthermore, the same evaluation scores as used in Test Example 5 were used for evaluation.

Those results are summarized in Table 9.

In Table 9, S1 refers to the score for skin condition, while S2 refers to the score for itchiness.

**[Table 9]**

| Target | Start of treatment | After 3 days | After 1 week | After 2 weeks | After 3 weeks | After 4 weeks | Overall evaluation |
|---|---|---|---|---|---|---|---|
| | S1:S2 | S1:S2 | S1:S2 | S1:S2 | S1:S2 | S1:S2 | S1:S2 |
| Right arm | 5:3 | 5:1 | 4:1 | 2:1 | 2:1 | 2:1 | 1:1 |
| Left arm | 5:3 | 5:3 | 4:2 | 4:2 | 3:1 | 3:1 | 3:1 |

As is indicated above, the skin conditions of the left and right arms exhibiting the same symptoms in the same patient b improved after 4 weeks. Itchiness disappeared more quickly and the skin improved more rapidly on the right arm, to which was applied the cream for external use of Example 24 in the form of a compound preparation, than on the left arm, to which was applied the cream for external use of Example 11 in the form of an external preparation of tinidazole alone.

The following test examples 9-33 do not form part of the invention.

### (Test Example 9)

The therapeutic effects of external preparations produced in the examples were examined by applying to actual patients with eczema/rash and seborrheic dermatitis.

The external preparations were applied to the target patients indicated below.
Target patient c: 60-year-old female suffering from cosmetic rash.
Target patient d: 34-year-old male suffering from seborrheic dermatitis.
Target patient e: 45-year-old male suffering from insect bite (mite).
Target patient f: 57-year-old male suffering from tinea.
Target patient g: 30-year-old female suffering from acne.
Target patient h: 28-year-old male suffering from suppurative dermatitis on the head.
Target patient i: 25-year-old male suffering from dermatitis herpitiformis (blisters) on the neck.
Target patient j: 45-year-old female suffering from candidiasis between the fingers.
Target patient k: 63-year-old male suffering from dry eczema on the back.
Target patient l: 28-year-old male suffering from suppurative dermatitis on the head.
Target patient m: 63-year-old male suffering from boils and eczema on the neck.
Target patient n: 33-year-old male suffering from early herpes on the forehead.
Target patient o: 23-year-old female suffering from dry eczema on the lower limbs.

### Method:

The cream for external use produced in the Example 22 was applied twice a day for 4 consecutive weeks to target patients c and d, and its effect was observed.

The ointment for external use produced in the Example 33 was applied twice a day until symptoms improved to target patient e, and its effect was observed.

The cream for external use produced in the Example 31 was applied twice a day for 4 consecutive weeks to target patient f, and its effect was observed.

The cream for external use produced in the Example 30 was applied twice a day until symptoms improved to target patient g, and its effect was observed.

The gel produced in the Example 39 was applied twice or three times a day until symptoms improved to target patient h, and its effect was observed.

The cream for external use produced in the Example 32 was applied twice or three times a day until symptoms improved to target patient i, and its effect was observed.

The ointment for external use produced in the Example 35 was applied twice a day until symptoms improved to target patient j, and its effect was observed.

The lotion for external use produced in the Example 36 was applied twice or three times a day for 4 consecutive weeks to target patient k, and its effect was observed.

The patch produced in the Example 37 was applied twice or three times a day for 3 consecutive weeks to target patient I, and its effect was observed.

The plaster produced in Example 38 was applied twice or three times a day for 3 consecutive weeks to target patient m, and its effect was observed.

The cream for external use produced in the Example 21 was applied twice or forth a day until symptoms improved to target patient n, and its effect was observed.

The ointment for external use produced in the Example 34 was applied twice or three times a day until symptoms improved to target patient o, and its effect was observed.

Therapeutic effects were evaluated by scoring rash, eczema and other dermatitis symptoms at the start of treatment along with the status of healing over 3 days, 1 week, 2 weeks, 3 weeks and 4 weeks later. In addition, the presence of itchiness of the skin surface and skin condition were evaluated after 4 weeks.

Furthermore, in addition to using the same evaluation scores as used in Test Example 5 for evaluation, pain was also evaluated using the scores indicated below. Pain Status:
3: Stinging pain
2: Pain not noticed unless affected area touched
1: No pain even if affected area touched

Those results are summarized in Table 10 below.

In Table 10, S1 refers to the score for skin condition, S2 refers to the score for itchiness, and S3 refers to the score for pain.

**[Table 10]**

| Patient | Evaluation | Start of treatment | After 3 days | After 1 week | After 2 weeks | After 3 weeks | After 4 weeks | Overall evaluation |
|---|---|---|---|---|---|---|---|---|
| c | S1:S2 | 5:3 | 5:2 | 4:1 | 3:1 | 2:1 | 2:1 | 2:1 |
| d | S1:S2 | 4:3 | 3:2 | 2:1 | 2:1 | 1:1 | 1:1 | 1:1 |
| e | S1:S2 | 3:3 | 2:1 | 1:1 | -:- | -:- | -:- | 1:1 |
| f | S1:S2 | 5:3 | 4:2 | 3:2 | 3:1 | 2:2 | 2:1 | 2:2 |
| g | S1:S2 | 4:3 | 3:1 | 2:1 | 2:1 | 1:1 | 1:1 | 1:1 |
| h | S1:S3 | 4:3 | 2:1 | 2:1 | 1:1 | -:- | -:- | 1:1 |
| i | S1:S2 | 4:3 | 2:1 | 1:1 | 1:1 | -:- | -:- | 1:1 |
| j | S1:S3 | 3:3 | 2:1 | 1:1 | -:- | -:- | -:- | 1:1 |
| k | S1:S3 | 4:2 | 3:2 | 1:1 | 1:1 | 1:1 | -:- | 1:1 |
| l | S1:S2 | 4:3 | 4:3 | 3:1 | 2:1 | 2:1 | -:- | 2:1 |
| m | S1:S3 | 3:3 | 3:3 | 2:2 | 2:1 | 2:1 | -:- | 2:1 |
| n | S1:S2 | 3:3 | 1:1 | -:- | -:- | -:- | -:- | 1:1 |
| o | S1:S2 | 4:3 | 3:1 | 2:1 | 1:1 | 1:1 | -:- | 1:1 |

As is indicated above, during treatment of various skin diseases, application of the external preparations of the present invention resulted in improvement in symptoms being observed 3-7 days after the start of treatment, and the skin was no different from normal skin after 3-4 weeks. Although the skin of patient c became keloid due to the adverse side effects of using steroids for about six months, dermatitis had been relieved. Although patient f was not completely healed after 4 weeks due to having suffered from tinea over the long period of roughly 40 years, improvement in skin condition was remarkable. Furthermore, there was no irritation of the skin by the preparation during application. In addition, there were also no adverse side effects such as rebound observed, which are observed with steroid-based external preparations, even after administration was discontinued.

### (Test Example 10)

The therapeutic effects of external preparations produced in the examples were examined by applying to actual patients with eczema/rash and seborrheic dermatitis.
The external preparations were applied to the target patients indicated below.
Target patient p: 10-year-old boy suffering from psoriasis vulgaris on the instep of the foot.
Target patient q: 10-year-old boy suffering from psoriasis vulgaris on the lower limb.
Target patient r: 45-year-old male suffering from suppurative dermatitis caused by an insect bite.
Target patient s: 50-year-old female suffering from erythroderma on the face.
Target patient t: 20-year-old female suffering from acne.
Target patient u: 23-year-old female suffering from eczema on the upper arm
Target patient v: 50-year-old female suffering from atopic dermatitis on the head.
Target patient w: 63-year-old female suffering from tinea on the toes of the feet.
Target patient x: 65-year-old male suffering from a tumor on the neck (having both odor and pain).

### Method :

The cream for external use produced in the Example 44 was applied twice a day for 4 consecutive weeks to target patients p and q, and its effect was observed.

The ointment for external use produced in the Example 46 was applied twice a day until symptoms improved to target patient r, and its effect was observed.

The cream for external use produced in the Example 41 was applied twice a day for 4 consecutive weeks to target patient s, and its effect was observed.

The cream for external use produced in the Example 40 was applied twice a day until symptoms improved to target patient t, and its effect was observed.

The cream for external use produced in the Example 42 was applied twice or three times a day until symptoms improved to target patient u, and its effect was observed.

The gel produced in the Example 25 was applied twice or three times a day until symptoms improved to target patient v, and its effect was observed.

The lotion produced in the Example 47 was applied twice or three times a day until symptoms improved to target patient w, and its effect was observed.

The cream for external use produced in the Example 50 was applied twice or three times a day for 4 consecutive weeks to target patient x, and its effect was observed.

Therapeutic effects were evaluated by scoring rash, eczema and other dermatitis symptoms at the start of treatment along with the status of healing over 3 days, 1 week, 2 weeks, 3 weeks and 4 weeks later. In addition, the presence of itchiness of the skin surface and skin condition were evaluated after 4 weeks.

Furthermore, the same evaluation scores as used in the Test Example 10 were used for evaluation, pain was also evaluated using the scores indicated below.

Those results are summarized in Table 11 below.

In Table 11, S1 refers to the score for skin condition, S2 refers to the score for itchiness, and S3 refers to the score for pain.

**[Table 11]**

| Patient | Evaluation | Start of treatment | After 3 days | After 1 week | After 2 weeks | After 3 weeks | After 4 weeks | Overall evaluation |
|---|---|---|---|---|---|---|---|---|
| p | S1:S2 | 4:3 | 3:2 | 2:1 | 2:1 | 1:1 | 1:1 | 1:1 |
| q | S1:S2 | 4:3 | 2:1 | 2:1 | 2:1 | 1:1 | 1:1 | 1:1 |
| r | S1:S3 | 4:3 | 2:1 | 2:1 | 1:1 | 1:1 | -:- | 1:1 |
| s | S1:S2 | 5:2 | 4:1 | 3:1 | 2:1 | 2:1 | 2:1 | 2:1 |
| t | S1:S2 | 3:3 | 1:1 | 1:1 | -:- | -:- | -:- | 1:1 |
| u | S1:S2 | 4:3 | 2:1 | 2:1 | 1:1 | -:- | -:- | 1:1 |
| v | S1:S2 | 4:3 | 3:1 | 3:1 | 2:1 | 2:1 1 | 1:1 | 1:1 |
| w | S1:S2 | 4:3 | 2:1 | 2:1 | 2:1 | 1:1 | 1:1 | 1:1 |
| x | S1:S3 | 4:3 | 4:3 | 3:2 | 3:2 | 2:1 | 2:1 | 2:1 |

As is indicated above, during treatment of various skin diseases, application of the creams for external use of the present invention resulted in improvement in symptoms being observed 3-7 days after the start of treatment, and the skin was no different from normal skin after 3-4 weeks. In addition, although patient v noticed partial hair loss on the head, the hair began to grow somewhat starting in week 3.

Furthermore, there was no irritation of the skin by the preparation during application. In addition, there were also no adverse side effects such as rebound observed, which are observed with steroid-based external preparations, even after administration was discontinued.

### (Test Example 11)

The therapeutic effects of external preparations of the present invention were examined by applying to actual patients with dermatitis and hircus.

The external preparations were applied to the target patients indicated below.
Target patient y (right): Right arm of a 33-year-old male suffering from hircus
Target patient y (left): Left arm of a 33-year-old male suffering from hircus

### Method:

Target patient y (right): Cream for external use of the Example 52
Target patient y (left): Cream for external use of the Example 53

The above creams for external use were each applied twice a day to site affected by hircus, and progress was observed.

Therapeutic effects were evaluated by scoring odor and other symptoms at the start of treatment along with the status of healing over 3 days, 1 week, 2 weeks and 3 weeks later.

Furthermore, evaluation scores were determined in the manner indicated below.

### Skin Soiling:

4: Soiled
3: Somewhat soiled
2: Almost clean
1: Clean

### Odor:

4: Strong odor
3: Slight odor
2: Hardly any odor
1: No odor

Those results are summarized in Table 12 below.

In Table 12, S4 refers to the score for skin soiling, and S5 refers to the score for odor.

**[Table 12]**

| Target | Start of treatment | After 3 days | After 1 week | After 2 weeks | After 3 weeks |
|---|---|---|---|---|---|
| | S4:S5 | S4:S5 | S4:S5 | S4:S5 | S4:S5 |
| Right arm | 4:4 | 4:4 | 3:3 | 3:1 | 1:1 |
| Left arm | 4:4 | 4:4 | 3:3 | 3:1 | 1:1 |

As indicated above, hircus on the right and left arms of target patient y was alleviated after 7-14 days, and was completely healed after 14-21 days.

Furthermore, there was no irritation or abnormalities of the skin of target patient y during application.

### (Test Example 12) Treatment of Hircus

The therapeutic effects of external preparations of the present invention were examined by applying to actual patients with dermatitis and hircus.

The external preparations were applied to the target patients indicated below.
Target patient z: 33-year-old male suffering from hircus
Target patient Aa: 33-year-old male suffering from hircus

### Method:

Target patient z: Cream for external use of the Example 22
Target patient Aa: Cream for external use of the Example24

The above creams for external use were each applied twice a day to site affected by hircus, and progress was observed.

Therapeutic effects were evaluated by scoring odor and other symptoms at the start of treatment along with the status of healing over 3 days, 1 week, 2 weeks and 3 weeks later.

Furthermore, the same evaluation scores used in the Test Examples 10 and 12 were used for evaluation.

Those results are summarized in Table 13 below.

In Table 13, S1 refers to the score for skin condition, and S5 refers to the score for odor.

**[Table 13]**

| Target patient | Evaluation | Start of treatment | After 3 days | After 1 week | After 2 weeks | After 3 weeks | After 4 weeks | Overall evaluation |
|---|---|---|---|---|---|---|---|---|
| z | S1:S5 | 4:3 | 2:1 | 1:1 | 1:1 | -:- | -:- | 1:1 |
| Aa | S1:S5 | 3:3 | 2:1 | 1:1 | -:- | -:- | -:- | 1:1 |

As indicated above, the external preparations of the present invention improved the skin condition and odor associated with hircus in a short period of time.

### (Test Example 13) Treatment of Hircus

The therapeutic effects of external preparations of the present invention were examined by applying to actual patients with dermatitis and hircus.
Target patient Ab: Right arm of 27-year-old male suffering from hircus
Target patient Ac: Left arm of 27-year-old male suffering from hircus
Target patient Ad: Right arm of 44-year-old male suffering from hircus
Target patient Ae: Left arm of 44-year-old male suffering from hircus
Target patient Af: Right arm of 23-year-old female suffering from hircus
Target patient Ag: Left arm of 23-year-old female suffering from circus

### Method :

Target patient Ab: Cream for external use of the Example 54
Target patient Ac: Cream for external use of the Example 55
Target patient Ad: Cream for external use of the Example 56
Target patient Ae: Cream for external use of the Example 57
Target patient Af: Cream for external use of the Example 58
Target patient Ag: Cream for external use of the Eample 59

The above creams for external use were each applied twice a day to site affected by hircus, and progress was observed.

Therapeutic effects were evaluated by scoring odor and other symptoms at the start of treatment along with the status of healing in the course of time.

Furthermore, the same evaluation scores used in the Test Example 11 were used for evaluation.

Those results are summarized in Table 14 below.

In Table 14, S4 refers to the score for skin soiling, and S5 refers to the score for odor.

**[Table 14]**

| Target patient | Evaluation | Start of treatment | After 1 day | After 3 days | After 5 days | After 7 days | After 10 days |
|---|---|---|---|---|---|---|---|
| Ab | S4:S5 | 4:4 | 4:4 | 3:2 | 2:1 | 2:1 | 1:1 |
| Ac | S4:S5 | 4:4 | 4:3 | 3:2 | 3:1 | 2:1 | 1:1 |
| Ad | S4:S5 | 4:4 | 4:3 | 3:2 | 3:1 | 2:1 | 1:1 |
| Ae | S4:S5 | 4:3 | 4:3 | 3:1 | 3:1 | 1:1 | 1:1 |
| Af | S4:S5 | 4:4 | 3:3 | 2:3 | 2:1 | 2:1 | 1:1 |
| Ag | S4: S5 | 4:4 | 4:3 | 3:3 | 3:1 | 2:1 | 2:1 |

As indicated above, odor associated with hircus was alleviated after 3-5 days for all of the target patients, and both hircus and skin condition improved after 7-10 days.

Furthermore, there was no irritation or abnormalities of the skin of any of the subjects during application.

Similar results were obtained against hircus for the external preparations of the Example 25 and the Examples 60-64, with odor improving after about 3-5 days, and both odor and skin condition improving after 7-10 days. In addition, lotions and gels were easy to use.

Furthermore, since it was possible that hircus may have been alleviated due to the effects of the base alone, a placebo was applied twice a day for about 2 consecutive weeks. This had no effect, however.

### (Test Example 14) Treatment of Odor

The therapeutic effects of external preparations of the present invention were examined by applying to actual patients with foot odor.

The external preparations were applied to the target patients indicated below.

Target patient Ah: Right foot of 24-year-old male having foot odor starting below the ankle (at the location from where foot odor is usually thought to emit).

Target patient Ai: Left foot of 24-year-old male having foot odor starting below the ankle (at the location from where foot odor is usually thought to emit). Method:
Target patient Ah: Lotion for external use of the Example 63
Target patient Ai: Placebo lotion for external use from which the active ingredient of the Example 63 had been removed.

When the lotion for external use containing the active ingredient was applied to the right foot, foot odor disappeared in about 4-5 hours. However, when the placebo lotion was applied to the left foot, foot odor persisted even after about 4-5 hours.

### (Test Example 15)

The therapeutic effects of external preparations of the present invention were examined by applying to actual patients with dermatitis and psoriasis.

The external preparations were applied to the target patients indicated below.
Target patient Aj: Right foot of 43-year-old male suffering from psoriasis
Target patient Ak: Left foot of 43-year-old male suffering from psoriasis
Target patient Al: Right foot of 40-year-old male suffering from psoriasis
Target patient Am: Left foot of 40-year-old male suffering from psoriasis
Target patient An: Right foot of 38-year-old female suffering from psoriasis
Target patient Ao: Left foot of 38-year-old female suffering from psoriasis
Target patient Ap: Right foot of 49-year-old female suffering from psoriasis
Target patient Aq: Left foot of 49-year-old female suffering from psoriasis Method :
Target patient Aj: Cream for external use of the Example 52
Target patient Ak: Cream for external use of the Example 53
Target patient Al: Cream for external use of the Example 65
Target patient Am: Cream for external use of the Example 66
Target patient An: Cream for external use of the Example 130
Target patient Ao: Cream for external use of the Example 131
Target patient Ap: Cream for external use of the Example 67
Target patient Aq: Cream for external use of the Example 68

The above creams for external use were each applied three times a day to the site affected by psoriasis, and progress was observed.

Furthermore, evaluation scores were determined in the manner indicated below.

### Evaluation:

5: Worse than at the start of treatment
4: No change from the start of treatment
3: Some improvement
2: Remarkable improvement
1: No different from normal skin

Those results are summarized in Table 15 below.

**[Table 15]**

| Target patient | After 3 days | After 7 days | After 21 days | After 1 month | After 2 months | After 3 months |
|---|---|---|---|---|---|---|
| Aj | 4 | 3 | 3 | 2 | 2 | 1 |
| Ak | 4 | 3 | 3 | 2 | 2 | 1 |
| Al | 4 | 2 | 2 | 2 | 1 | (1) |
| Am | 4 | 2 | 2 | 2 | 1 | (1) |
| An | 4 | 2 | 2 | 2 | 1 | 1 |
| Ao | 4 | 2 | 2 | 1 | 1 | 1 |
| Ap | 4 | 2 | 2 | 1 | 1 | (1) |
| Aq | 4 | 2 | 2 | 1 | 1 | (1) |

As indicated above, symptoms of psoriasis exhibited significant change after 7-21 days in all of the target patients, and psoriasis was completely healed after 1-2 months.
Although application was discontinued after 2 months for target patients Al, Am, Ap and Aq, there was no recurrence as of 1 month later. In addition, there were no adverse side effects and so forth observed in any of the target patients.

### (Test Example 16) Treatment of Psoriasis

Target patient Ar: Right foot of 38-year-old male suffering from psoriasis
Target patient As: Left foot of 38-year-old male suffering from psoriasis

### Method:

Target patient Ar: Cream for external use of the Example 65
Target patient As: Commercially available Bonalfa ointment (Teijin Co.,Ltd.) (ingredient: tacalcitol)

The above creams for external use were each applied twice a day to the site affected by psoriasis, and their respective therapeutic effects were observed in the course of time.

Furthermore, the same evaluation scores used in the Test Example 15 were used for evaluation.

Those results are summarized in Table 16 below.

**[Table 16]**

| Target patient | After 3 days | After 7 days | After 21 days | After 1 month | After 2 months | After 3 months |
|---|---|---|---|---|---|---|
| Ar | 4 | 2 | 2 | 1 | - | - |
| As | 4 | 4 | 3 | 2 | - | - |

As indicated above, skin condition clearly improved for target patient As, namely with application of the metronidazole external preparation. There were no adverse side effects observed for either preparation.

### (Test Example 17)

Target patient At: Head of 33-year-old male suffering from psoriasis
Target patient Au: Right elbow of 38-year-old male suffering from psoriasis
Target patient Av: Left elbow of 38-year-old male suffering from psoriasis

### Method:

Target patient At: Lotion of the Example 72
Target patient Au: Composite preparation for external use of the Example 73
Target patient Av: Cream for external use of the Example 65

The above creams for external use were each applied twice a day to the site affected by psoriasis, and their respective therapeutic effects were observed in the course of time.

Furthermore, the same evaluation scores used in Test Example 15 were used for evaluation.

Those results are summarized in Table 17 below.

**[Table 17]**

| Target patient | After 3 days | After 7 days | After 21 days | After 1 month | After 2 months | After 3 months |
|---|---|---|---|---|---|---|
| At | 4 | 2 | 1 | 1 | - | - |
| Au | 4 | 2 | 2 | 1 | 1 | - |
| Av | 4 | 3 | 2 | 2 | 1 | - |

As indicated above, remarkable improvement was observed after 7 to 21 days. With respect to a comparison of target patients Au and Av, a compound metronidazole preparation (2%) was applied to the right foot, and a preparation containing metronidazole alone (2%) was applied to the left foot. A comparison revealed the compound preparation to be more effective. In addition, there were no adverse side effects observed for any of the preparations.

### (Test Example 18) Treatment of Psoriasis

Testing was performed on target patients Aw, Ax, Ay, Az, Ba and Bb in the same manner as the Test Example 15.

### Method:

Target patients Aw and Ax: Ointment for external use of the Example 69
Target patients Ay and Az: Ointment for external use of the Example 70
Target patient Ba: Lotion of the Example 72
Target patient Bb: Composite preparation of the Example 74

The external preparations of the present invention demonstrated remarkable effects in all of these target patients as well, and four of the target patients were completely healed within 1 month, while the remaining two target patients were completely healed within 1-3 months.

### (Test Example 19) Treatment of Psoriasis

Target patient Bc: Right foot of 70-year-old patient suffering from psoriasis
Target patient Bd: Left foot of 70-year-old patient suffering from psoriasis Method:
Target patient Bc: cream for external use of the Example 73
Target patient Bd: cream for external use prepared in the same manner as the Example 73 but without adding metronidazole (preparation containing Tranilast only)

The above creams for external use were each applied twice a day to the site affected by psoriasis, and their respective therapeutic effects were observed in the course of time.

Furthermore, the same evaluation scores used in the Test Example 15 were used for evaluation.

Those results are summarized in Table 18 below.

**[Table 18]**

| Target patient | After 3 days | After 7 days | After 21 days | After 1 month | After 2 months | After 3 months |
|---|---|---|---|---|---|---|
| Bc | 4 | 2 | 2 | 2 | 1 | 1 |
| Bd | 4 | 4 | 4 | 4 | 3 | 4 |

As indicated above, there were no effects observed at all in the case of applying Tranilast at 0.1%.

### (Test Example 20) Use for Scars and Blotches

### Target patients:

Target patient Be: Right arm of 40-year-old male having a scar
Target patient Bf: Right arm of 40-year-old male having a scar
Target patient Bg: Face of 38-year-old female having blotches
Target patient Bh: Face of 60-year-old male having blotches
Target patient Bi: Right foot of 27-year-old patient having a scar
Target patient Bj: Left foot of 27-year-old patient having a scar

### Method :

Be: External preparation of the Example 75
Bf: External preparation of the Example 76
Bg: External preparation of the Example 78
Bh: External preparation of the Example 77
Bi: External preparation of the Example 77
Bj: External preparation prepared in the same manner as the Example 75 but without adding metronidazole

The above external preparations were each applied three times a day (twice a day for target patients Bi and Bj), and the progress was observed.

Furthermore, evaluation scores were determined in the manner indicated below.

### Evaluation:

3: Status prior to administration or no change
2: Improvement over previous condition
1: Clear improvement as compared with grade 2

Those results are summarized in Table 19 below.

**[Table 19]**

| As indicated by the above results, skin condition improved, and there are no particular adverse side effects observed. In addition, the skin had more luster and was smoother than before application. There were no particular | | | | | |
|---|---|---|---|---|---|
| Target patient | Start of treatment | After 2 weeks | After 3 weeks | After 1 month | After 2 months |
| Be | 3 | 3 | 3 | 2 | 2 |
| Bf | 3 | 3 | 2 | 2 | 1 |
| Bg | 3 | 3 | 2 | 1 | 1 |
| Bh | 3 | 3 | 2 | 2 | 1 |
| Bi | 3 | 3 | 2 | 1 | 1 |
| Bj | 3 | 3 | 3 | 3 | 3 |

changes in target patient Bj to which only a base was applied.

### (Test Example 21)

### Target patients:

Target patient Bk: Right hand of 34-year-old male on which the skin has been damaged by a burn
Target patient Bl: Right finger of 33-year-old male on which there is a wound from a cut
Target patient Bm: Right hand of a 34-year-old male on which the skin has been damaged following the removal of a wart
Target patient Bn: Right foot of 12-year-old boy having a scrape wound
Target patient Bo: Left foot of 12-year-old boy having a scrape wound
Target patient Bp: Face of 5-year-old infant on which the skin has been damaged by a scratch
Target patient Bq: Right arm of 5-year-old infant on which the skin has been damaged by a scratch
Target patient Br: Left arm of 5-year-old infant on which the skin has been damaged by a scratch

### Method:

Bk: External preparation of the Example 79
Bl: External preparation of the Example 81
Bm: External preparation of the Example 82
Bn: External preparation of the Example 79
Bo: External preparation of the Example 81
Bp: External preparation of the Example 80
Bq: External preparation of the Example 82
Br: External preparation prepared in the same manner as the Example 79 but without adding metronidazole

The above external preparations were each applied three times a day (twice a day for target patients Bq and Br), and the progress was observed.

Furthermore, evaluation scores were determined in the manner indicated below.

### Evaluation:

3: Status prior to administration or no change
2: Improvement over previous condition
1: Clear improvement as compared with grade 2

Those results are summarized in Table 20 below.

In Table 20, S6 refers to the score for skin condition, S7 refers to the score for pain, and S8 refers to the score for irritation.

**[Table 20]**

| Target patient | Evaluation | Start of treatment | After 1 day | After 3 days | After 1 week | After 2 weeks |
|---|---|---|---|---|---|---|
| Bk | S6:S7 | 3:3 | 3:2 | 1:1 | 1:1 | -:- |
| BI | S6:S7 | 3:3 | 3:2 | 1:1 | 1:1 | -:- |
| Bm | S6:S7 | 3:3 | 3:2 | 2:2 | 1:2 | 1:1 |
| Bn | S6:S7 | 3:3 | 3:3 | 2:1 | 1:1 | -:- |
| Bo | S6:S7 | 3:3 | 3:2 | 2:1 | 1:1 | -:- |
| Bp | S6:S8 | 3:3 | 3:2 | 1:1 | 1:1 | 1:1 |
| Bq | S6:S8 | 3:3 | 2:2 | 2:1 | 1:1 | 1:1 |
| Br | S6:S8 | 3:3 | 3:3 | 3:3 | 3:3 | -:- |

As indicated by the above results, skin condition improved, and there was no occurrence of adverse side effects. These preparations were particularly superior with respect to improving or eliminating pain quite rapidly. In addition, the skin also had more luster and was smoother than before application. Since there were no particular changes observed in target patient Br in which only a base was applied, application of base only was discontinued after 1 week at the request of the target patient and parents, and when the cream of the Example 82 was applied instead, the skin was nearly completely healed in 1-2 weeks.

### (Test Example 22)

### Target patients:

Target patient Bs: Right foot of 22-year-old female suffering from skin disease caused by weed ill
Target patient Bs: Right hand of 22-year-old female suffering from skin disease caused by weed ill
Target patient Bu: Two parts of face of 27-year-old male suffering from an insect bite
Target patient Bv: Right hand of 27-year-old male suffering from an insect bite
Target patient Bw: Right hand of 24-year-old female suffering from contact dermatitis
Target patient Bx: Right foot of 47-year-old male suffering from contact dermatitis
Target patient By: 28-year-old female suffering from dermatitis caused by detergent rash

### Method:

Bs: External preparation of the Example 83
Bt: External preparation of the Example 85
Bu: External preparation of the Example 84
Bv: External preparation of the Example 86
Bw: External preparation of the Example 86
Bx: External preparation of the Example 86
By: External preparation of the Example 85

The above external preparations were each applied two to three times a day (twice a day for target patients Bu and Bv, whenever the hands were washed for target patients Bw and By), and the progress was observed.

Furthermore, evaluation scores were determined in the manner indicated below.

### Evaluation:

3: Status prior to administration or no change
2: Improvement over previous condition
1: Clear improvement as compared with grade 2

Those results are summarized in Table 21 and Table 22 below.

**[Table 21]**

| Target patient | Start of treatment | After 1 hour | After 3 hours | After 6 hours | After 1 day | After 3 days |
|---|---|---|---|---|---|---|
| Bs | 3 | 3 | 2 | 1 | 1 | - |
| Bt | 3 | 3 | 2 | 1 | 1 | - |
| Bu | 3 | 3 | 2 | 2 | 1 | 1 |
| Bv | 3 | 3 | 2 | 1 | 1 | 1 |

**[Table 22]**

| Target | Start of treatment | After 3 days | After 7 days | After 2 wees | After 1 month | After 3 months |
|---|---|---|---|---|---|---|
| Bw | 3 | 3 | 2 | 2 | 1 | 1 |
| Bx | 3 | 3 | 3 | 2 | 2 | 1 |
| By | 3 | 2 | 1 | 1 | - | - |

As indicated by the above results, skin condition improved, and there was no occurrence of adverse side effects. These preparations were proved to eliminate itching discomfort and pain in a short period of time in particular. Although scars from the insect bites remained for target patients Bu and Bv, the scars disappeared about 1 week after the start of application. Although improvement similarly took some time for target patients Bw and Bx as well, itching was improved or eliminated in about 3-7 days after application.

### (Test Example 23)

### Target patients:

Target patient Bz: Back of a 78-year-old male suffering from dry pruritis
Target patient Ca: Back of 71-year-old male suffering from eczema through to a drug-induced side effect (anti-hypertension drug)
Target patient Cb: Back of 83-year-old male suffering from eczema
Target patient Cc: Both of arm of 83-year-old male suffering from eczema
Target patient Cd: Back of 68-year-old female suffering from dry pruritis
Target patient Ce: Face of 30-year-old female suffering from eczema caused by a cosmetics-induced side effect
Target patient Cf: Face of 40-year-old female suffering from eczema caused by a cosmetics-induced side effect

### Method:

The external preparation of the Example 87 to target patients Bz, Ca, Cb, Cc and Ce, and the external preparation of the Example 88 to target patients Cd and Cf were each applied twice a day, and the progress was observed.

Furthermore, evaluation scores were determined in the manner indicated below.

### Evaluation:

3: Status prior to administration or no change
2: Improvement over previous condition
1: Clear improvement as compared with grade 2

Those results are summarized in Table 23 below.

**[Table 23]**

| Target patient | Start of treatment | After 3 days | After 1 week | After 2 weeks | After 1 month |
|---|---|---|---|---|---|
| Bz | 3 | 1 | 1 | 1 | - |
| Ca | 3 | 2 | 2 | 1 | 1 |
| Cb | 3 | 2 | 1 | 1 | - |
| Cc | 3 | 2 | 1 | 1 | - |
| Cd | 3 | 1 | 1 | 1 | 1 |
| Ce | 3 | 3 | 2 | 2 | 1 |
| Cf | 3 | 3 | 2 | 2 | 1 |

As indicated by the above results, skin condition improved, and there was no occurrence of adverse side effects. Itching stopped in a few days, and skin condition improved each day. Although target patients Ce and Cf required about 1 month for skin condition to heal completely due to the particularly serious cosmetic rash exhibited by these target patients, itching disappeared after about 3 days.

### (Test Example 24)

### Target patients:

Target patient Cg: Right hand of 26-year-old male suffering from chapped skin
Target patient Ch: Left hand of 26-year-old male suffering from chapped skin
Target patient Ci: Right foot of 26-year-old male suffering from chilblain
Target patient Cj: Left foot of 26-year-old male suffering from chilblain

### Method:

Cg: External preparation of the Example 89
Ch: External preparation produced in the same manner as the Example 89 but without using metronidazole
Ci: External preparation of the Example 90
Cj: External preparation produced in the same manner as the Example 89 but without using metronidazole

The above external preparations were each applied twice a day for target patients Ci and Cj, whenever the hands were washed for target patients Cg and Ch, and the progress was observed.

Furthermore, evaluation scores were determined in the manner indicated below.

### Evaluation:

3: Status prior to administration or no change
2: Improvement over previous condition
1: Clear improvement as compared with grade 2

Those results are summarized in Table 24 below.

**[Table 24]**

| Target patient | Start of treatment | After 3 days | After 1 week | After 2 weeks | After 1 month |
|---|---|---|---|---|---|
| Cg | 3 | 3 | 2 | 2 | 2 |
| Ch | 3 | 3 | 3 | 3 | 3 |
| Ci | 3 | 2 | 2 | 2 | 1 |
| Cj | 3 | 3 | 3 | 3 | 3 |

As indicated by the above results, skin condition improved, and there was no occurrence of adverse side effects. Although skin condition was not improved by the preparations that did not contain the active ingredient, in those preparations that did contain the active ingredient, clear improvement was observed. In particular, itching and discomfort were improved in about 1 week.

### (Test Example 25)

### Target patients:

Target patient Ck: Back of a 74-year-old male suffering from dry erythroderma
Target patient Cl: Arm of a 74-year-old male suffering from dry erythroderma
Target patient Cm: Back of an 80-year-old male suffering from pustular psoriasis erythroderma

### Method:

The external preparation of the Example 91 was applied to target patients Ck and Cm, and the external preparation of the Example 92 was applied to target patient Cl twice a day, and the progress was observed.

Furthermore, evaluation scores were determined in the manner indicated below.

### Evaluation:

5: Skin condition worse than before administration
4: Skin condition before administration or no change
3: Some improvement as compared with before administration
2: Greater improvement as compared with grade 3
1: Clear improvement as compared with grade 2

Those results are summarized in Table 25 below.

**[Table 25]**

| Target patient | Start of treatment | After 3 days | After 1 week | After 2 weeks | After 1 month | After 2 months |
|---|---|---|---|---|---|---|
| Ck | 4 | 4 | 3 | 3 | 2 | 2 |
| Cl | 4 | 4 | 3 | 3 | 2 | 2 |
| Cm | 4 | 4 | 3 | 3 | 2 | 2 |

As indicated by the above results, skin condition improved, and there was no occurrence of adverse side effects. Itching improved in about 1 week. More time was required due to the nature of these skin conditions in being difficult to heal completely.

### (Test Example 26)

### Target patients:

Target patient Cn: Right foot of 55-year-old male suffering from tinea
Target patient Co: Left foot of 55-year-old male suffering from tinea
Target patient Cp: Right hand of 46-year-old female suffering from nail tinea
Target patient Cq: Right hand of 38-year-old female suffering from nail tinea

### Method:

The external preparation of the Example 93 was applied to target patient Cn twice a day, the external preparation of the Example 94 was applied to target patient Co twice a day, the external preparation of the Example 94 was applied to subject Cp three times a day, and the external preparation of the Example 93 was applied to target patient Cq three times a day, and the progress was observed.

Furthermore, evaluation scores were determined in the manner indicated below.

### Evaluation:

5: Skin condition worse than before administration
4: Skin condition before administration or no change
3: Some improvement as compared with before administration
2: Greater improvement as compared with grade 3
1: Clear improvement as compared with grade 2

Those results are summarized in Table 26 below.

**[Table 26]**

| Target patient | Start of treatment | After 1 week | After 2 weeks | After 3 weeks | After 1 month | After 2 months |
|---|---|---|---|---|---|---|
| Cn | 4 | 4 | 3 | 3 | 2 | 2 |
| Co | 4 | 3 | 3 | 2 | 2 | 2 |
| Cp | 4 | 3 | 2 | 2 | 1 | 1 |
| Cq | 4 | 3 | 2 | 2 | 2 | 2 |

As indicated by the above results, skin condition improved, and there was no occurrence of adverse side effects.

### (Test Example 27)

### Target patients:

Target patient Cr: Left foot of 49-year-old male suffering from suppurative skin disease
Target patient Cs: Area around the mouth of 61-year-old male suffering from herpes
Target patient Ct: Forehead of 33-year-old male suffering from herpes
Target patient Cu: Left arm of 64-year-old female suffering from suppurative skin disease
Target patient Cv: Right hand of 56-year-old subject suffering from candidiasis
Target patient Cw: Both hands of 38-year-old female suffering from periunguitis
Target patient Cx: Back of 33-year-old male suffering from dermal pruritis Method:

The external preparation of the Example 96 was applied to target patient Cr three times a day, the external preparation of the Example 95 was applied to target patient Cs three times a day, the external preparation of Example 95 was applied to target patient Ct twice a day, the external preparation of the Example 96 was applied to target patient Cu three times a day, the external preparation of the Example 96 was applied to target patient Cv three to four times a day, the external preparation of Example 96 was applied to target patient Cw whenever the hands were washed, and the external preparation of Example 95 was applied to target patient Cx twice a day, and the progress was observed.

Furthermore, evaluation scores were determined in the manner indicated below.

### Evaluation:

4: Condition before administration or no change
3: Some improvement as compared with before administration
2: Definite improvement
1: No different from normal skin

Those results are summarized in Tables 27 and 28 below.

**[Table 27]**

| Target patient | Start of treatment | After 1 day | After 3 days | After 1 week | After 2 weeks | After 1 month |
|---|---|---|---|---|---|---|
| Cs | 4 | 2 | 1 | 1 | - | - |
| Ct | 4 | 1 | 1 | 1 | - | - |
| Cx | 4 | 2 | 1 | 1 | - | - |

**[Table 28]**

| Target patient | Start of treatment | After 1 week | After 2 weeks | After 3 weeks | After 1 month | After 2 months |
|---|---|---|---|---|---|---|
| Cr | 4 | 3 | 3 | 2 | 2 | 2 |
| Cu | 4 | 3 | 3 | 3 | 2 | 2 |
| Cv | 4 | 3 | 3 | 3 | 2 | 2 |
| Cw | 4 | 3 | 3 | 2 | 2 | 1 |

As indicated by the above results, skin condition improved, and there was no occurrence of adverse side effects. In all target patients, itchiness, pain and discomfort disappeared prior to improvement of skin condition.

### (Test Example 28)

### Target patients:

Target patient Cx: Face of 62-year-old female on which blotches are present on normal skin.

Target patient Cy: Face of 38-year-old male having a scar (in the form of a knob) caused by the adverse side effects of steroids.

Target patient Cz: Foot of 5-year-old boy having a scar caused by the adverse side effects of steroids

### Method:

The cream for external use of the Example 97 was applied to the target patients twice a day.

Furthermore, evaluation scores were determined in the manner indicated below.

### Evaluation:

5: Blotches and scars, etc. can be clearly distinguished from other parts of the skin.
4: Blotches and scars, etc. can be clearly distinguished from other parts of the skin but not to the degree of grade 5.
3: Blotches and scars, etc. can be distinguished from other parts of the skin.
2: Blotches and scars, etc. are slightly visible, but are essentially no different from other parts of the skin.
1: No difference whatsoever from other parts of the skin

Those results are summarized in Table 29 below.

**[Table 29]**

| Target patient | Start of treatment | After 1 week | After 2 weeks | After 3 weeks | After 4 weeks | After 2 months | After 3 months |
|---|---|---|---|---|---|---|---|
| Cx | 4 | 4 | 4 | 3 | 3 | 2 | 2 |
| Cy | 5 | 5 | 5 | 5 | 5 | 3 | 2 |
| Cz | 3 | 3 | 2 | 1 | 1 | - | - |

As indicated above, the external preparation of the present invention clearly demonstrated remarkable improvement with respect to blotches as well as scars caused by the adverse side effects of steroids.

### (Test Example 29)

### Target patients:

Target patient Da: Face of 63-year-old female with normal skin having a dark complexion.

Target patient Db: Face of 65-year-old female with normal skin having a dark complexion.

### Method:

The external preparation of Example 98 was applied twice a day to target patient Da, while the preparation of Example 99 was applied twice a day to target patient Db.

Furthermore, evaluation scores were determined in the manner indicated below.

### Evaluation:

3: Present skin condition of the face that would generally be considered to be dark.
2: Somewhat lighter complexion not to the extent of grade 3.
1: Clearly lighter complexion as compared with grade 3.

Those results are summarized in Table 30 below.

**[Table 30]**

| Target | Start of treatment | After 2 weeks | After 1 month | After 2 months | After 3 months |
|---|---|---|---|---|---|
| Da | 3 | 3 | 2 | 1 | 1 |
| Db | 3 | 3 | 2 | 1 | 1 |

As indicated above, the external preparations of the present invention clearly resulted in remarkable improvement of pigment deposition.

### (Test Example 30)

### Target patients:

Target patient Dc: Right foot of 54-year-old male suffering from folliculitis
Target patient Dd: Back of 75-year-old male suffering from drug rash
Target patient De: 60-year-old male suffering from a laceration caused by contusion
Target patient Df: Left hand of 32-year-old male having pain caused by a scrape wound
Target patient Dg: Left shoulder of 44-year-old male suffering from suppurative skin disease
Target patient Dh: 38-year-old male whose eyebrows have become thin due to the adverse side effects of external steroid preparations
Target patient Di: Both arms of 38-year-old male on which scars remain due to the adverse side effects of external steroid preparations

### Method:

The external preparation of the Example 100 was applied twice a day to target patient Dc, the external preparation of the Example 101 was applied twice a day to target patient Dd, the external preparation of the Example 102 was applied three times a day to target patient De, the external preparation of the Example 103 was applied twice a day to target patient Df, the external preparation of the Example 104 was applied twice a day to target patient Dg, the external preparation of the Example 105 was applied 3-4 times a day to target patient Dh, and the external preparation of the Example 106 was applied twice a day to target patient Di, and the progress was observed.

Furthermore, the evaluation scores for target patients Dc through Di were determined in the manner indicated below.

### Evaluation:

5: Skin symptoms are extremely worse
4: Skin symptoms are extremely worse but not to the degree of grade 5
3: Skin symptoms are moderate
2: Skin symptoms are hardly able to be detected
1: Normal skin

Those results are summarized in Table 31 below.

**[Table 31]**

| Target patient | Start of treatment | After 3 days | After 1 week | After 2 weeks | After 3 weeks | After 4 weeks |
|---|---|---|---|---|---|---|
| Dc | 4 | 3 | 1 | 1 | - | - |
| Dd | 3 | 1 | 1 | 1 | 1 | 1 |
| De | 3 | 2 | 1 | 1 | - | - |
| Df | 3 | 1 | - | - | - | - |
| Dg | 4 | 3 | 2 | 2 | - | - |
| Di | 5 | 5 | 4 | 4 | 3 | 3 |

Furthermore, evaluation scores for target patient Dh were determined in the manner indicated below.

### Evaluation:

5: Eyebrows and vellus hair completely absent
4: Eyebrows absent but vellus hair growing
3: Slight eyebrow growth
2: Eyebrows have grown back but still visually conspicuous
1: Eyebrows identical to those of normal persons

Those results are summarized in Table 32 below

**[Table 32]**

| Target patient | Start of treatment | After 3 days | After 1 week | After 2 weeks | After 3 weeks | After 4 weeks |
|---|---|---|---|---|---|---|
| Dh | 5 | 5 | 4 | 3 | 3 | 2 |

As is clear from the above, the external preparations of the present invention exhibited ameliorative effects in each of the target patients.

Furthermore, target patient Dd suffered from hypertension and exhibited eczema and itching caused by the side effects of administration of an anti-hypertension drug. Since the drug rash and itching reappeared when use of the external cream preparation was discontinued, this target patient used the preparation for a long period of time.

In addition, target patients Dh and Di refer to the same individual. Although skin condition was evaluated as grade 3 after 4 weeks, it was upgraded to grade 2 one month later. Although his eyebrows were also evaluated as grade 2 after 3 months as compared with the start of application, the number of hairs was increasing a little at a time.

### (Test Example 31) Effects on Skin Moistness and Smoothness

### Target patients:

Target patient Dj: Right side of the face of 62-year-old female with normal skin.

Target patient Dk: Left side of the face of 62-year-old female with normal skin.

Target patient Dl: Right side of the face of 69-year-old female with normal skin.

Target patient Dm: Left side of the face of 69-year-old female with normal skin.

Target patient Dn: Left arm of 62-year-old female with normal skin.

Target patient Do: Right arm of 62-year-old female with normal skin.

### Method:

The external preparation of the Example 107 to target patient Dj, an external preparation produced in the same manner as the Example 107 without using metronidazole to target patient Dk, the external preparation of the Example 108 to target patient Dl, and an external preparation produced in the same manner as the Example 108 without using tinidazole to target patient Dm, were respectively applied twice a day, while the external preparation produced in the Example 109 to target patient Dn and the external preparation produced in the Example 110 to target patient Do were respectively applied three times a day. All of the external preparations were applied for 2 consecutive months, and the progress of the target patients was observed. Moreover, progress was also observed after application was discontinued 2 months after the start of application.

Furthermore, the evaluation scores were determined in the manner indicated below.

### Evaluation (evaluation of skin moistness and smoothness when waking up on the day after application):

5: Worse skin condition than before use
4: Skin condition before use or no change
3: Some improvement as compared with before use
2: Definite improvement as compared with before use
1: Extremely good skin condition

Those results are summarized in Tables 33 and 34 below.

**[Table 33] (Progress During Use)**

| Target patient | Before ol treatment | After 3 days | After 1 week | After 2 weeks | After 3 weeks | After 4 weeks | After 2 months |
|---|---|---|---|---|---|---|---|
| Dj | 4 | 4 | 3 | 2 | 2 | 1 | 1 |
| Dk | 4 | 4 | 4 | 4 | 4 | 4 | 3 |
| Dl | 4 | 4 | 3 | 2 | 2 | 1 | 1 |
| Dm | 4 | 4 | 4 | 4 | 4 | 4 | 4 |
| Dn | 4 | 3 | 2 | 2 | 2 | 1 | 1 |
| Do | 4 | 3 | 2 | 2 | 1 | 1 | 1 |

**[Table 34] (Progress After Discontinuation of Application)**

| Target patient | When discontinued | After 3 days | After 1 week | After 2 weeks | After 1 month |
|---|---|---|---|---|---|
| Dj | 1 | 1 | 1 | 2 | 2 |
| Dk | 3 | 4 | 4 | 4 | 4 |
| Dl | 1 | 1 | 1 | 1 | 2 |
| Dm | 4 | 4 | 4 | 4 | 4 |
| Dn | 1 | 1 | 1 | 2 | 2 |
| Do | 1 | 1 | 1 | 1 | 2 |

As indicated by the above results, satisfactory skin condition continued for 7-14 days after discontinuing application. There were no adverse side effects and the preparations were highly safe, while preparations not containing the active ingredient were not effective.

### (Test Example 32)

Target patient Dp: Left foot of 7-year-old boy having a scratch wound suffering from atopic dermatitis.

Target patient Dq: Right foot of 7-year-old boy having a scratch wound suffering from atopic dermatitis.

Target patient Dr: Left foot of 39-year-old male having a scratch wound suffering from atopic dermatitis.

Target patient Ds: Right foot of 39-year-old male having a scratch wound suffering from atopic dermatitis.

### Method :

Dp: Preparation of Example 112 applied twice a day.

Dq: Preparation of Example 113 applied twice a day.

Dr: Preparation of Example 114 applied three times a day

Ds: Preparation of Example 115 applied three times a day

Furthermore, the evaluation scores were determined in the manner indicated below.
5: Prominent rash, eczema and other dermatitis symptoms, extreme itchiness, subconscious scratching of the skin.
4: Prominent rash, eczema and other dermatitis symptoms along with considerable itchiness.
3: Rash, eczema and other dermatitis symptoms able to be confirmed and some itchiness.
2: Rash, eczema and other dermatitis symptoms only able to be confirmed slightly and not much different from normal skin, with some itchiness but to the extent that scratching can be controlled.
1: Absence of rash, eczema and other dermatitis symptoms, appearance of normal skin and no itchiness.

Those results are shown in Table 35 below.

**[Table 35]**

| Target patient | Start of treatment | After 3 days | After 7 days | After 2 weeks | After 3 weeks | After 1 months |
|---|---|---|---|---|---|---|
| Dp | 4 | 4 | 3 | 2 | 1 | 1 |
| Dq | 4 | 4 | 3 | 2 | 2 | 2 |
| Dr | 5 | 5 | 4 | 3 | 3 | 2 |
| Ds | 5 | 5 | 4 | 3 | 3 | 2 |

Although ketoconazole and isoconazole nitrate can normally not be administered to affected areas where wounds are present, by producing compound preparations with nitroimidazole derivative in the external preparations of the present invention, these preparations were able to be used at affected areas where wounds were present. Although there was no change in skin condition in target patients Cp and Cq after about 3 days, itchiness had nearly completely disappeared, and scratching stopped after about 5 days even when sleeping.

Target patients Cr and Cs had a history of atopic dermatitis going back roughly 20 years, and skin condition had worsened considerably due to the adverse side effects of steroid drugs. Itchiness disappeared nearly completely after about 5 days, and there was no scratching after about 7 days.

### (Test Example 33)

### Target patients:

Target patient Dt: Left side of the back (from the waist to the shoulder) of 72-year-old male suffering from dermal pruritis
Target patient Du: Left side of the back (same location as above) of 69-year-old female suffering from dermal pruritis
Target patient Dv: Right side of the back (same location as above) of 72-year-old male suffering from dermal pruritis
Target patient Dw: Right side of the back (same location as above) of 69-year-old female suffering from dermal pruritis

Furthermore, the evaluation scores were determined in the manner indicated below.

### Method:

An external preparation produced in the same manner as Example 29 without using metronidazole to target patient Dt, an external preparation produced in the same manner as the Example 29 by adding 10 g of crotamiton without using metronidazole for target patient Du, the external preparation of the Example 111 for target patient Dv, and the external preparation of the Example 29 for target patient Dw were respectively applied twice a day.

### Evaluation:

5: Itchiness worse than at the start of application
4: Same as the start of application or extremely itchy
3: Some alleviation of itchiness
2: Occasional itchiness but hardly noticeable
1: No itchiness whatsoever

Those results are summarized in Table 36 below.

**[Table 36]**

| Target patient | Start of treatment | After 3 days | After 1 week | After 2 weeks | After 3 weeks | After 1 month | Overall evaluation |
|---|---|---|---|---|---|---|---|
| Dt | 4 | 4 | 3 | 2 | 2 | 1 | Hardly any change Some im- |
| Du | 4 | 4 | 3 | 2 | 2 | 2 | provement Remarkable |
| Dv | 5 | 5 | 4 | 3 | 3 | 2 | improvement after 3 days to 1 week Remarkable |
| Dw after | 5 | 5 | 4 | 3 | 3 | 2 | improvement 2-3 days |

As indicated above, in comparison with the preparation containing only crotamiton, the external preparations of the present invention demonstrated remarkable improvement of dermal pruritis. However, when crotamiton was contained, effects appeared earlier.

### (Test Example 34) Stability Test

The ointment for external use produced in the above the Example 1, the cream for external use produced in the Example 4, the ointment for external use produced in the Example 11, and the cream for external use produced in the Example 14 were stored at room temperature and 40°C followed by observation of changes in their appearance, pH, content and viscosity after 6 months. Those results are shown in Table 37 below.

In Table 37, "NC" means no change as compared with before storing.

**[Table 37]**

| Example | Appearance room temperature | 40°C | pH room temperature | 40°C | Content room temperature | 40°C | Viscosity room temperature | 40°C |
|---|---|---|---|---|---|---|---|---|
| 1 | NC | NC | NC | NC | NC | NC | NC | NC |
| 4 | NC | NC | NC | NC | NC | NC | NC | NC |
| 11 | NC | NC | NC | NC | NC | NC | NC | NC |
| 14 | NC | NC | NC | NC | NC | NC | NC | NC |

The external preparations of the present invention exhibited no changes in appearance or pH, and did not exhibit any significant changes in content or viscosity.

Thus, the external preparations provided by the present invention were clearly determined to be pharmacologically stable.

## Claims

1. Use of nitroimidazole derivative represented by the following formula (I), a pharmaceutically acceptable salt thereof, an ester thereof or other derivatives thereof as an active ingredient for the production of an external preparation used for therapeutic or prophylactic treatment or amelioration of atopic dermatitis: 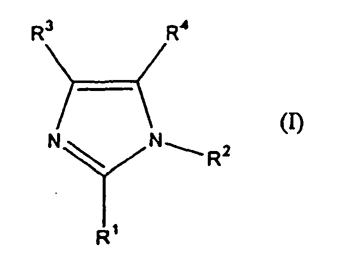 wherein R¹, R³ and R⁴ may be the same or different and each independently represents a hydrogen atom, a nitro group, a lower alkyl group having 1 to 6 carbon atoms, a lower alkyl group having 1 to 6 carbon atoms substituted by 1 or more substituents which may be the same or different selected from Substituent group α and Substituent group β, a lower alkenyl group having 2 to 6 carbon atoms, or a lower alkenyl group having 2 to 6 carbon atoms substituted by 1 or more substituents which may be the same or different selected from the Substituent group α and the Substituent group β; and R² represents a hydrogen atom, a lower alkyl group having 1 to 6 carbon atoms, a lower alkyl group having 1 to 6 carbon atoms substituted by 1 or more substituents which may be the same or different selected from the Substituent group α and the Substituent group β, a lower alkenyl groups having 2 to 6 carbon atoms or a lower alkenyl group having 2 to 6 carbon atoms substituted by 1 or more substituents, which may be the same or different selected from the Substituent group α and the Substituent group β, provided that any one of R¹, R³ and R⁴ is a nitro group,
wherein.
The Substituent group α comprises a lower alkyloxy group having 1 to 6 carbon atoms, a lower alkyloxy group having 1 to 6 carbon atoms substituted by 1 or more substituents which may be the same or different selected from the Substituent group β, a lower alkylcarbonyloxy group having 2 to 7 carbon atoms, a lower alkylcarbonyloxy group having 2 to 7 carbon atoms substituted by 1 or more substituents which may be the same or different selected from the Substituent group β, a lower alkylsulfonyl group having 1 to 6 carbon atoms, a lower alkylsulfonyl group having 1 to 6 carbon atoms substituted by 1 or more substituents which may be the same or different selected from the Substituent group β, a cycloalkyl group, a cycloalkyl group substituted by 1 or more substituents which may be the same or different selected from the Substituent group β, a heteroaryl group, a heteroaryl group substituted by 1 or more substituents which may be the same or different selected from the Substituent group β, an aryl group and an aryl group substituted by 1 or more substituents which may be the same or different selected from the Substituent group β; and
the Substituent group β comprises
a hydroxy group, a mercapto group, a halogen atom, an amino group, a lower alkylamino group having 1 to 6 carbon atoms, a lower alkyloxy group having 1 to 6 carbon atoms, a lower alkenyl group having 2 to 6 carbon atoms, a cyano group, a carboxy group, a carbamoyloxy group, a carboxyamide group, a thiocarboxyamide group and a morpholino group.

2. The use of Claim 1, wherein R⁴ is a nitro group.

3. The use of Claim 2, wherein R¹ and R² are the same or different and represent a lower alkyl group, a lower alkyl group substituted by 1 or more substituents selected from the Substituent group α and the Substituent group β, a lower alkenyl group, or a lower alkenyl group substituted by 1 or more substituents which may be the same or different selected from the Substituent group α and the Substituent group β, and R³ is a hydrogen atom.

4. The use of Claim 3, wherein the Substituent group α is a lower alkyloxy group and the Substituent group β is a hydroxy group, an amino group, a halogen atom, a cycloalkyl group, a heteroaryl group or an aryl group.

5. The use of Claim 4, wherein the Substituent group β is a hydroxy group, an amino group, a halogen atom or a heteroaryl group.

6. The use of Claim 5, wherein R¹ is a lower alkyl group.

7. The use of any one of Claims 1 and 5, wherein R² is a lower alkyl group substituted by a hydroxy group.

8. The use of Claim 1, which comprises 2-(2-methyl-5-nitroimidazole-1-yl)ethanol (general name: metronidazole), a pharmaceutically acceptable salt thereof, an ester thereof or other derivatives thereof as an active ingredient.

9. The use of Claim 3, wherein the Substituent group α is a lower alkylsulfonyl group or a lower alkylsulfonyl group substituted by substituents which may be the same or different selected from the Substituent group β and the Substituent group β is a hydroxy group, a halogen atom, an amino group, a lower alkylamino group, a lower alkyloxy group, a lower alkenyl group, a cyano group, a carboxy group, a cycloalkyl group or an aryl group.

10. The use of Claim 9, wherein R¹ is a lower alkyl group or lower alkyl group substituted by substituents which may be the same or different selected from the Substituent group β.

11. The use of Claim 9, wherein R² is a lower alkylsulfonyl group or a lower alkylsulfonyl group substituted by substituents which may be the same or different selected from the Substituent group β.

12. The use of Claim 1, wherein the preparation comprises 1-(2-ethylsulfonylethyl)-2-methyl-5-nitroimidazole (general name: tinidazole) or a pharmaceutically acceptable salt thereof as an active ingredient.

13. The use of Claim 1, wherein the preparation comprises one compound of the nitroimidazole derivatives as defined in any one of Claims 1 to 12 and one medicine selected from the group consisting of an antimycotic agent, antibacterial agent, sulfa, immunosuppressant, antiinflammatory agent, antibiotic, antiviral agent, metabolic antagonist, antihistamine, tissue repair promoter, vitamin, antiallergic, local anesthetic, hair agent and steroid being administered simultaneously or separately with an interval to skin of patient.

14. The use of Claim 13, wherein the antimycotic agent, the antibacterial agent, the sulfa, the immunosuppressant, the antiinflammatory agent, the antibiotic, the antiviral agent, the metabolic antagonist, the antihistamine, the tissue repair promoter, the vitamin, the antiallergic, the local anesthetic, the hair agent or the steroids is used with a concentration at which the agent itself does not demonstrate any pharmacological effect.

15. The use of any one of Claims 1 to 14, wherein the preparation further comprises crotamiton.

16. The use of any one of any of the preceding claims, wherein the skin disease is atopic dermatitis.

17. The use of any of the preceding claims, wherein the skin disease is infant atopic dermatitis.

18. The use of any one of Claims 1 to 17, wherein a concentration of the nitroimidazole derivative is 0.1 to 20% by weight based on the amount of the preparation.

## Patentansprüche

1. Verwendung eines Nitroimidazolderivates dargestellt durch die folgende Formel (I), eines pharmazeutisch akzeptablen Salzes davon, eines Esters davon oder anderen Derivaten davon als ein aktiver Bestandteil zur Herstellung einer Zubereitung zur äußerlichen Anwendung verwendet zur therapeutischen oder prophylaktischen Behandlung oder zur Verbesserung atopischer Dermatitis: 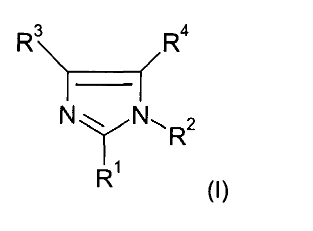 wobei R¹, R³ und R⁴ gleich oder unterschiedlich sein können und unabhängig voneinander ein Wasserstoffatom, eine Nitro-Gruppe, eine Niederalkyl-Gruppe mit 1 bis 6 Kohlenstoffatomen, eine Niederalkyl-Gruppe mit 1 bis 6 Kohlenstoffatomen substituiert mit ein oder mehreren Substituenten, welche gleich oder unterschiedlich sein können ausgewählt aus Substituentengruppe α und Substituentengruppe β, eine Niederalkenyl-Gruppe mit 2 bis 6 Kohlenstoffatomen, oder eine Niederalkenyl-Gruppe mit 2 bis 6 Kohlenstoffatomen substituiert mit ein oder mehreren Substituenten, welche gleich oder unterschiedlich sein können ausgewählt aus der Substituentengruppe α und der Substituentengruppe β, darstellen; und R² ein Wasserstoffatom, eine Niederalkyl-Gruppe mit 1 bis 6 Kohlenstoffatomen, eine Niederalkyl-Gruppe mit 1 bis 6 Kohlenstoffatomen substituiert mit ein oder mehreren Substituenten, welche gleich oder unterschiedlich sein können ausgewählt aus der Substituentengruppe α und der Substituentengruppe β, eine Niederalkenyl-Gruppe mit 2 bis 6 Kohlenstoffatomen oder eine Niederalkenyl-Gruppe mit 2 bis 6 Kohlenstoffatomen substituiert mit ein oder mehreren Substituenten, welche gleich oder unterschiedlich sein können ausgewählt aus der Substituentengruppe α und der Substituentengruppe β, darstellt, vorausgesetzt, dass jede der R¹, R³ und R⁴ Gruppen eine Nitro-Gruppe ist, wobei
die Substituentengruppe α eine Niederalkoxy-Gruppe mit 1 bis 6 Kohlenstoffatomen, eine Niederalkoxy-Gruppe mit 1 bis 6 Kohlenstoffatomen substituiert mit ein oder mehreren Substituenten, welche gleich oder unterschiedlich sein können ausgewählt von der Substituentengruppe β, eine Niederalkylcarbonyloxy-Gruppe mit 2 bis 7 Kohlenstoffatomen, eine Niederalkylcarbonyloxy-Gruppe mit 2 bis 7 Kohlenstoffatomen substituiert mit ein oder mehreren Substituenten, welche gleich oder unterschiedlich sein können ausgewählt von der Substituentengruppe β, eine Niederalkylsulfonyl-Gruppe mit 1 bis 6 Kohlenstoffatomen, eine Niederalkylsulfonyl-Gruppe mit 1 bis 6 Kohlenstoffatomen substituiert mit ein oder mehreren Substituenten, welche gleich oder unterschiedlich sein können ausgewählt von der Substituentengruppe β, eine Cycloalkyl-Gruppe, eine Cycloalkyl-Gruppe substituiert mit ein oder mehreren Substituenten, welche gleich oder unterschiedlich sein können ausgewählt aus der Substituentengruppe β, eine Heteroaryl-Gruppe, eine Heteroaryl-Gruppe substituiert mit ein oder mehreren Substituenten, welche gleich oder unterschiedlich sein können ausgewählt aus der Substituentengruppe β, eine Aryl-Gruppe und eine Aryl-Gruppe substituiert mit ein oder mehreren Substituenten, welche gleich oder unterschiedlich sein können ausgewählt aus der Substituentengruppe β umfasst und
die Substituentengruppe β eine Hydroxy-Gruppe, eine Mercapto-Gruppe, ein Halogenatom, eine Amino-Gruppe, eine Niederalkylamino-Gruppe mit 1 bis 6 Kohlenstoffatomen, eine Niederalkyloxy-Gruppe mit 1 bis 6 Kohlenstoffatomen, eine Niederalkenyl-Gruppe mit 2 bis 6 Kohlenstoffatomen, eine Cyano-Gruppe, eine Carboxy-Gruppe, eine Carbamoyloxy-Gruppe, eine Carboxyamid-Gruppe, eine Thiocarboxyamid-Gruppe und eine MorpholinoGruppe umfasst.

2. Verwendung gemäß Anspruch 1, wobei R⁴ eine Nitro-Gruppe ist.

3. Verwendung gemäß Anspruch 2, wobei R¹ und R² gleich oder unterschiedlich sind und eine Niederalkyl-Gruppe, eine Niederalkyl-Gruppe substituiert mit ein oder mehreren Substituenten ausgewählt von der Substituentengruppe α und der Substituentengruppe β, eine Niederalkenyl-Gruppe oder eine Niederalkenyl-Gruppe substituiert mit ein oder mehreren Substituenten, welche gleich oder unterschiedlich sein können ausgewählt aus der Substituentengruppe α und der Substituentengruppe β, darstellen, und R³ ein Wasserstoffatom ist.

4. Verwendung gemäß Anspruch 3, wobei die Substituentengruppe α eine Niederalkyloxy-Gruppe und die Substituentengruppe β eine Hydroxy-Gruppe, eine Amino-Gruppe, ein Halogenatom, eine Cycloalkyl-Gruppe, eine Heteroaryl-Gruppe oder eine Aryl-Gruppe ist.

5. Verwendung gemäß Anspruch 4, wobei die Substituentengruppe β eine Hydroxy-Gruppe, eine Amino-Gruppe, ein Halogenatom oder eine Heteroaryl-Gruppe ist.

6. Verwendung gemäß Anspruch 5, wobei R¹ eine Niederalkyl-Gruppe ist.

7. Verwendung gemäß einem der Ansprüche 1 bis 5, wobei R² eine Niederalkyl-Gruppe substituiert mit einer Hydroxy-Gruppe ist.

8. Verwendung gemäß Anspruch 1, welche 2-(2-Methyl-5-nitroimidazol-1-yl)ethanol (allgemeiner Name: Metronidazol), ein pharmazeutisch akzeptables Salz davon, einen Ester davon oder andere Derivate davon als einen aktiven Bestandteil umfasst.

9. Verwendung gemäß Anspruch 3, wobei die Substituentengruppe α eine Niederalkylsulfonyl-Gruppe oder eine Niederalkylsulfonyl-Gruppe substituiert mit Substituenten, welche gleich oder unterschiedlich sein können ausgewählt aus der Substituentengruppe β ist und die Substituentengruppe β eine Hydroxy-Gruppe, ein Halogenatom, eine Amino-Gruppe, eine Niederalkylamino-Gruppe, eine Niederalkyloxy-Gruppe, eine Niederalkenyl-Gruppe, eine Cyano-Gruppe, eine Carboxy-Gruppe, eine Cycloalkyl-Gruppe oder eine Aryl-Gruppe ist.

10. Verwendung gemäß Anspruch 9, wobei R¹ eine Niederalkyl-Gruppe oder eine Niederalkyl-Gruppe substituiert mit Substituenten, welche gleich oder unterschiedlich sein können ausgewählt aus der Substituentengruppe β.

11. Verwendung gemäß Anspruch 9, wobei R² eine Niederalkylsulfonyl-Gruppe oder eine Niederalkylsulfonyl-Gruppe substituiert mit Substituenten, welche gleich oder unterschiedlich sein können ausgewählt von der Substituentengruppe β.

12. Verwendung gemäß Anspruch 1, wobei die Zubereitung 1-(2-Ethylsulfonylethyl)-2-methyl-5-nitroimidazol (allgemeiner Name: Tinidazol) oder ein pharmazeutisch akzeptables Salz davon als ein aktiven Bestandteil umfasst.

13. Verwendung gemäß Anspruch 1, wobei die Zubereitung eine Verbindung der Nitroimidazolderivate, wie in einem der Ansprüche 1 bis 12 definiert, und ein Medikament ausgewählt aus der Gruppe bestehend aus einem antimykotischen Agens, antibakteriellen Agens, Sulfonamid, Immunsuppressivum, antiinflammatorischen Agens, Antibiotikum, antiviralen Agens, methabolischen Antagonisten, Antihistaminikum, Gewebereparaturpromotor, Vitamin, Antiallergikum, Lokalanästhetikum, Haar-Agens und Steroid umfasst, wobei die Zubereitung gleichzeitig oder getrennt in einem Intervall auf die Haut des Patienten gegeben wird.

14. Verwendung gemäß Anspruch 13, wobei das antimykotische Agens, das antibakterielle Agens, das Sulfonamid, das Immunsuppressivum, das antiinflammatorische Agens, das Antibiotikum, das antivirale Agens, der methabolische Antagonist, das Antihistaminikum, der Gewebereparaturpromotor, das Vitamin, das Antiallergikum, das Localanästthetikum, das Haar-Agens oder die Steroide in einer Konzentration verwendet werden, in der das Agens selbst keinen pharmakologischen Effekt zeigt.

15. Verwendung gemäß einem der Ansprüche 1 bis 14, wobei die Zubereitung zusätzlich Crotamiton umfasst.

16. Verwendung gemäß einem der vorstehenden Ansprüche, wobei die Hauterkrankung atopische Dermatitis ist.

17. Verwendung gemäß einem der vorstehenden Ansprüche, wobei die Hauterkrankung infantile atopische Dermatitis ist.

18. Verwendung gemäß einem der Ansprüche 1 bis 17, wobei eine Konzentration des Nitroimidazolderivates 0,1 bis 20 Gew.-% bezogen auf die Menge der Zubereitung ist.

## Revendications

1. Utilisation d'un dérivé de nitroimidazole représenté par la formule (I) suivante, d'un de ses sels pharmaceutiquement acceptables, d'un de ses esters ou d'autres dérivés de celui-ci comme ingrédient actif pour la production d'une préparation externe utilisée pour le traitement thérapeutique ou prophylactique ou l'amélioration de la dermatite atopique : 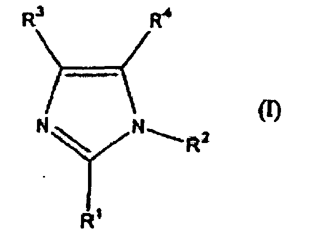 formule dans laquelle R¹, R³ et R⁴ peuvent être identiques ou différents et représentent chacun indépendamment un atome d'hydrogène, un groupe nitro, un groupe alkyle inférieur ayant 1 à 6 atomes de carbone, un groupe alkyle inférieur ayant 1 à 6 atomes de carbone substitué avec un ou plusieurs substituants qui peuvent être identiques ou différents, choisis dans le groupe de substituants α et le groupe de substituants β, un groupe alcényle inférieur ayant 2 à 6 atomes de carbone ou un groupe alcényle inférieur ayant 2 à 6 atomes de carbone substitué avec un ou plusieurs substituants qui peuvent être identiques ou différents, choisis dans le groupe de substituants α, et le groupe de substituants β ; et R² représente un atome d'hydrogène, un groupe alkyle inférieur ayant 1 à 6 atomes de carbone, un groupe alkyle inférieur ayant 1 à 6 atomes de carbone substitué avec un ou plusieurs substituants qui peuvent être identiques ou différents, choisis dans le groupe de substituants α et le groupe de substituants β, un groupe alcényle inférieur ayant 2 à 6 atomes de carbone ou un groupe alcényle inférieur ayant 2 à 6 atomes de carbone substitué avec un ou plusieurs substituants qui peuvent être identiques ou différents, choisis dans le groupe de substituants α et le groupe de substituants β, sous réserve que n'importe lequel des groupes R¹, R³ et R⁴ représente un groupe nitro,
où
le groupe de substituants α comprend :
un groupe alkyloxy inférieur ayant 1 à 6 atomes de carbone, un groupe alkyloxy inférieur ayant 1 à 6 atomes de carbone substitué avec un ou plusieurs substituants qui peuvent être identiques ou différents, choisis dans le groupe de substituants β, un groupe alkylcarbonyloxy inférieur ayant 2 à 7 atomes de carbone, un groupe alkylcarbonyloxy inférieur ayant 2 à 7 atomes de carbone substitué avec un ou plusieurs substituants qui peuvent être identiques ou différents, choisis dans le groupe de substituants β, un groupe alkylsulfonyle inférieur ayant 1 à 6 atomes de carbone, un groupe alkylsulfonyle inférieur ayant 1 à 6 atomes de carbone substitué avec un ou plusieurs substituants qui peuvent être identiques ou différents, choisis dans le groupe de substituants β, un groupe cycloalkyle, un groupe cycloalkyle substitué avec un ou plusieurs substituants qui peuvent être identiques ou différents, choisis dans le groupe de substituants β, un groupe hétéroaryle, un groupe hétéroaryle substitué avec un ou plusieurs substituants qui peuvent être identiques ou différents, choisis dans le groupe de substituants β, un groupe aryle et un groupe aryle substitué avec un ou plusieurs substituants qui peuvent être identiques ou différents, choisis dans le groupe de substituants β ; et
le groupe de substituants β comprend
un groupe hydroxy, un groupe mercapto, un atome d'halogène, un groupe amino, un groupe alkylamino inférieur ayant 1 à 6 atomes de carbone, un groupe alkyloxy inférieur ayant 1 à 6 atomes de carbone, un groupe alcényle inférieur ayant 2 à 6 atomes de carbone, un groupe cyano, un groupe carboxy, un groupe carbamoyloxy, un groupe carboxamide, un groupe thiocarboxamide et un groupe morpholino.

2. Utilisation suivant la revendication 1, dans laquelle R⁴ représente un groupe nitro.

3. Utilisation suivant la revendication 2, dans laquelle R¹ et R² sont identiques ou différents et représentent un groupe alkyle inférieur, un groupe alkyle inférieur substitué avec un ou plusieurs substituants, choisis dans le groupe de substituants α et le groupe de substituants β, un groupe alcényle inférieur, ou un groupe alcényle inférieur substitué avec un ou plusieurs substituants qui peuvent être identiques ou différents, choisis dans le groupe de substituants α et le groupe de substituants β; et R³ représente un atome d'hydrogène.

4. Utilisation suivant la revendication 3, dans laquelle le groupe servant de substituant α est un groupe alkyloxy inférieur et le groupe servant de substituant β est un groupe hydroxy, un groupe amino, un atome d'halogène, un groupe cycloalkyle, un groupe hétéroaryle ou un groupe aryle.

5. Utilisation suivant la revendication 4, dans laquelle le groupe servant de substituant β est un groupe hydroxy, un groupe amino, un atome d'halogène ou un groupe hétéroaryle.

6. Utilisation suivant la revendication 5, dans laquelle R¹ représente un groupe alkyle inférieur.

7. Utilisation suivant l'une quelconque des revendications 1 à 5, dans laquelle R² représente un groupe alkyle inférieur substitué avec un groupe hydroxy.

8. Utilisation suivant la revendication 1, qui comprend du 2-(2-méthyl-5-nitroimidazole-1-yl)éthanol (dénomination générale : métronidazole), un de ses sels pharmaceutiquement acceptables, un de ses esters ou un autre dérivé de celui-ci, comme ingrédient actif.

9. Utilisation suivant la revendication 3, dans laquelle le groupe servant de substituant α est un groupe alkylsulfonyle inférieur ou un groupe alkylsulfonyle inférieur substitué avec des substituants qui peuvent être identiques ou différents choisis dans le groupe de substituants β, et le groupe servant de substituant β est un groupe hydroxy, un atome d'halogène, un groupe amino, un groupe alkylamino inférieur, un groupe alkyloxy inférieur, un groupe alcényle inférieur, un groupe cyano, un groupe carboxy, un groupe cycloalkyle ou un groupe aryle.

10. Utilisation suivant la revendication 9, dans laquelle R¹ représente un groupe alkyle inférieur ou un groupe alkyle inférieur substitué avec des substituants qui peuvent être identiques ou différents choisis dans le groupe de substituants β.

11. Utilisation suivant la revendication 9, dans laquelle R² représente un groupe alkylsulfonyle inférieur ou un groupe alkylsulfonyle inférieur substitué avec des substituants qui peuvent être identiques ou différents choisis dans le groupe de substituants β.

12. Utilisation suivant la revendication 1, dans laquelle la préparation comprend du 1-(2-éthylsulfonyléthyl)-2-méthyl-5-nitroimidazole (dénomination générale: tinidazole) ou un de ses sels pharmaceutiquement acceptables comme ingrédient actif.

13. Utilisation suivant la revendication 1, dans laquelle la préparation comprend un composé choisi parmi les dérivés de nitroimidazole tels que définis dans l'une quelconque des revendications 1 à 12 et un agent médicamenteux choisi dans le groupe consistant en un agent antimycosique, un agent antibactérien, un sulfamide, un immunosuppresseur, un agent anti-inflammatoire, un antibiotique, un agent antiviral, un antagoniste métabolique, un antihistaminique, un activateur de réparation tissulaire, une vitamine, un antiallergique, un anesthésique local, un agent à action capillaire et un stéroïde, administré simultanément ou séparément avec un intervalle à la peau d'un patient.

14. Utilisation suivant la revendication 13, dans laquelle l'agent antimycosique, l'agent antibactérien, le sulfamide, l'immunosuppresseur, l'agent anti-inflammatoire, l'antibiotique, l'agent antiviral, l'antagoniste métabolique, l'antihistaminique, l'activateur de réparation tissulaire, la vitamine, l'agent antiallergique, l'anesthésique local, l'agent à action capillaire ou le stéroïde est utilisé à une concentration à laquelle l'agent lui-même ne présente aucun effet pharmacologique.

15. Utilisation suivant l'une quelconque des revendications 1 à 14, dans laquelle la préparation comprend un outre du crotamiton.

16. Utilisation suivant l'une quelconque des revendications précédentes, dans laquelle la maladie cutanée est la dermatite atopique.

17. Utilisation suivant l'une quelconque des revendications précédentes, dans laquelle la maladie cutanée est la dermatite atopique du nourrisson.

18. Utilisation suivant l'une quelconque des revendications 1 à 17, dans laquelle la concentration du dérivé de nitroimidazole est de 0,1 à 20 % en poids sur la base de la quantité de la préparation.
